# EUROPEAN PATENT SPECIFICATION

(11) **EP 2 006 305 B1**
(45) Date of publication and mention of the grant of the patent: **10.08.2016**
(21) Application number: 07737666.3
(22) Date of filing: 02.03.2007
(51) Int. Cl.: C07K 16/46, C07K 16/28, C12N 15/09, C12P 21/08

(54) **MODIFIED ANTIBODY WITH ENHANCED BIOACTIVITY**
MODIFIZIERTE ANTIKÖRPER MIT VERBESSERTER BIOLOGISCHER WIRKUNG
ANTICORPS MODIFIE A BIOACTIVITE ACCRUE

(30) Priority: 03.03.2006 JP 2006057475
(43) Date of publication of application: 24.12.2008
(60) Divisional application: 13001539.9
(73) Proprietor: The Chemo-Sero-Therapeutic Research Institute, Kumamoto 860-8568 (JP); Teijin Pharma Limited, Tokyo 100-0013 (JP)
(72) Inventor: MASUHO, Yasuhiko, c/o Tokyo University of Science, Tokyo 162-8601 (JP); NAGASHIMA, Hiroaki, c/o Tokyo University of Science, Tokyo 162-8601 (JP)
(74) Representative: Bösl, Raphael Konrad
(86) International application number: PCT/JP2007/054018
(87) International publication number: WO 2007/100083

(56) References cited:
- WO-A2-2004/029207
- WO-A2-2005/077981
- WO-A2-2006/085967
- JP-A- 04 501 417
- GREENWOOD J. ET AL.: 'Engineering multiple-domain forms of the therapeutic antibody CAMPATH-1H: effects on complement lysis' THERAPEUTIC IMMUNOLOGY vol. 1, 1994, pages 247 - 255, XP009059035
- STEVENSON G.T. ET AL.: 'Conjugation of human Fcgamma in closed-hinge or open-hinge configuration to Fab'gamma and analogous ligands' J. IMMUNOL. vol. 158, no. 5, 1997, pages 2242 - 2250, XP002955118
- NAGASHIMA H. ET AL.: 'Fc Taryotaika ni yoru Kokassei Kotai' PROCEEDINGS OF THE ANNUAL MEETING OF THE PHARMACEUTICAL SOCIETY OF JAPAN vol. 126TH, no. 3, 06 March 2006, page 107, XP003017716
- STEVENSON G.T.: 'Attack on neoplastic cell membranes by therapeutic antibody' MOL. CELL BIOCHEM. vol. 91, 1989, pages 33 - 38, XP001105791
- SHOPES B.: 'A genetically engineered human IgG mutant with enhanced cytolytic activity' J. IMMUNOL. vol. 148, no. 9, 1992, pages 2918 - 2922, XP002141013
- CARON P.C. ET AL.: 'Engineered humanized dimeric forms of IgG are more effective antibodies' J. EXP. MED. vol. 176, 1992, pages 1191 - 1195, XP000914982
- RADAEV S. ET AL.: 'The structure of a human type III Fcgamma receptor in complex with Fc' J. BIOL. CHEM. vol. 276, no. 19, 2001, pages 16469 - 16477, XP002381582

## Description

### Technical Field

The present invention relates to methods for enhancing the effector activity of antibodies, modified antibodies with strong effector activity, and methods for producing the antibodies. More specifically, the present invention relates to methods for enhancing ADCC activity, which is a major effector activity, modified antibodies having a strong ADCC activity, and methods for producing the antibodies.

### Background Art

Antibodies are now being commonly used as therapeutic agents (Non-Patent Document 1). They have become applicable as therapeutic agents solely due to the development of various antibody-related techniques. The method for producing antibodies on a large scale was established based on the cell fusion technique developed by G. Köhler and C. Milstein (Non-Patent Document 2). Alternatively, with the advancement of genetic recombination techniques, large scale antibody production has become possible by inserting antibody genes into expression vectors and introducing them into host cells (Non-Patent Document 3).

Furthermore, antibodies have been improved to become closer to human-derived antibody molecules so that they will have no immunogenicity when administered to humans. Chimeric antibodies consisting of mouse variable regions and human constant regions (Non-Patent Document 4) and humanized antibodies consisting of mouse hypervariable regions, and human framework and constant regions (Non-Patent Document 5) have been developed, for instance. With the development of these techniques, antibodies have been put into practical use as therapeutic agents for cancers, autoimmune diseases, thrombosis, inflammation, infection, and so on. Clinical trials are underway for many more antibodies (Non-Patent Document 6).

While expectations on antibody pharmaceuticals are high, there are cases where, because of low antibody activity, sufficient therapeutic effects on cancers, autoimmune diseases, inflammation, infection, and such cannot be obtained, and cases where increased dose has increased patients' share of cost. Under these circumstances, enhancement of the therapeutic activity is an important objective for antibody therapeutic agents.

The effects of antibody pharmaceuticals include therapeutic effects that are exerted by the binding of their two Fab domains to disease-associated antigen molecules. For example, antibodies against tumor necrosis factor (TNF) inhibit the activity of TNF by binding to TNF, suppress inflammation, and thus exert a therapeutic effect on rheumatoid arthritis (Non-Patent Document 7). Since they produce a therapeutic effect by binding to antigen molecules and inhibiting the activity of the antigens, the higher the affinity against the antigen, the more the antibodies are expected to produce a stronger effect with a small dose. The method of selecting clones having high affinity to a same antigen from a number of monoclonal antibodies is commonly used to improve the antigen-binding affinity. In a possible alternative method, modified antibodies are prepared by genetic recombination and those exhibiting high affinity are selected therefrom.

On the other hand, when antibody pharmaceuticals aim at treating cancers, it is important that they exert cytotoxic effects against their target cancer cells. Antibodies bound to antigens on the surface of target cells bind, via their Fc domain, to Fc receptors on the surface of effector cells such as NK cells and macrophages, thereby exerting damage on target cells. This is called antibody-dependent cellular cytotoxicity (hereinafter abbreviated as ADCC).

Alternatively, antibodies damage cells by activating complements via the Fc domain. This is called complement-dependent cytotoxicity (hereinafter abbreviated as CDC). In addition to the cytotoxic activity, antibodies that bind to infecting microorganisms also have the activity of binding to Fc receptors on effector cells and mediating phagocytosis or impairment of the infecting microorganisms by the effector cells. Such antibody activities exerted via Fc domains are called effector activities.

There are a few different molecular species of Fc receptor to which human IgG binds. FcγRIA is present on the cell surface of macrophages, monocytes, and such, and exhibits high affinity for human IgG. FcγRIIA is present on macrophages, neutrophils, and such, and shows weak affinity for IgG. FcγRIIB is present on B lymphocytes, mast cells, macrophages, and such, exhibits weak affinity for IgG, and transduces suppressive signal. FcγRIIIA is present on natural killer (NK) cells, macrophages, and so on, has weak affinity for IgG, and plays an important role in exerting ADCC activity. FcγRIIIB is present on neutrophils, and has the same extracellular domain as FcγRIIIA but is bound on the cell surface via a GPI anchor. In addition to these, there also exists FcRn which is present in the small intestine and placenta and is involved in IgG metabolism. These Fc receptors are described in a review (Non-Patent Document 8).

There have been various attempts to enhance the effector function of antibodies with the aim of enhancing their cancer therapeutic activity. R. L. Shields *et al.* have generated multiple modified human IgG1 antibodies in which amino acids have been substituted in the CH2 and CH3 domains, which constitute the Fc domain, and have measured their Fc receptor-binding activity and ADCC activity (Non-Patent Document 9). As a result, many modified antibodies exhibited lower binding activities as compared to natural IgG1 antibodies; however, a slightly enhanced ADCC activity was observed for some of the modified antibodies. There is a report on an attempt to enhance CDC activity by substituting amino acids in the CH2 domain to which complements bind; however, although the binding of complement C1q was enhanced, CDC activity was rather attenuated (Non-Patent Document 10).

It is known that a sugar chain is linked to asparagine at position 297 in the Fc domain of IgG1 antibodies and that this difference in sugar chain influences the effector activity of antibodies. R. L. *Shields et al.* have reported that the absence of α1,6-fucose in the sugar chain of IgG1 antibodies has no significant influence on the binding to FcγRI, FcγRIIA, FcγRIIB, and complement Clq, but enhances the FcγRIIIA-binding activity by 50 times (Non-Patent Document 11). There are genetic variants of FcγRIIIA, which are the types in which the amino acid at position 158 is valine (Val) or phenylalanine (Phe). With both of these variants, the binding activity of α1,6-fucose-deficient IgG1 antibodies to FcγRIIIA was enhanced. Furthermore, α1,6-fucose-deficient antibodies were also reported to exhibit enhanced ADCC activity (Non-Patent Document 11). T. Shinkawa *et al.* have also reported similar results (Non-Patent Document 12).

S. G. Telford asserts that antibodies with multiple Fc regions have an improved Fc activity (Patent Document 1). Telford prepared modified antibodies that comprise hetero-divalent Fab consisting of anti-µ chain Fab and anti-CD19 Fab and in which two Fc regions are covalently linked in parallel via a synthetic linker, and measured their ADCC activity. However, when considering that target cells express both CD 19 and µ chain on their cell surface, the enhancement of ADCC activity observed by Telford can be thought to be due to not only the effect from the presence of multiple Fc regions, but also to the effect from efficient binding of the modified antibodies to the target cells through the hetero-divalent Fab. Because Telford has carried out no assessment to rule out the effect of hetero-divalent Fab as a cause of the enhanced ADCC activity, it is not clear whether the increase in the Fc regions is a cause for the enhanced Fc activity. Thus, when modified antibodies having multiple Fc regions but having a structure that differs from that of the modified antibodies of Telford are generated, whether these modified antibodies have an improved Fc activity or not is totally unpredictable.

Meanwhile, J. Greenwood generated modified antibodies with Fc portions linked in tandem and compared their CDC activities (Non-Patent Document 13). Contrary to expectations, all of the modified antibodies had a decreased CDC activity. Greenwood has not assessed the ADCC activity of the various types of modified antibodies.

In Patent Document 2, antibodies are disclosed presenting two Fc domains in tandem repeat on each heavy chain. The method for the production of said antibodies includes the insertion of a fragment encoding extra hinge, CH2 and CH3 domains into a vector encoding the heavy chain of said antibody. However, no effector activity such as antibody-dependent cellular cytotoxicity (ADCC) is disclosed to be exhibited by said antibodies.

As described above, there have been continued attempts to enhance the effector activities of antibodies; however, none of those attempts has provided satisfactory results.
[Patent Document 1] Japanese Patent No. 2907474, Japanese Patent *Kohyo* Publication No. (JP-A) H04-504147 (unexamined Japanese national phase publication corresponding to a non-Japanese international publication), WO90/04413.
[Patent Document 2] WO 2005/077981
[Non-Patent Document 1] Brekke OH. et al., Nature Review Drug Discovery, 2, 52(2003).
[Non-Patent Document 2] Köhler G. et al., Nature, 256, 495 (1975).
[Non-Patent Document 3] Carter P. et al., Nucleic Acid Research, 13, 4431 (1985).
[Non-Patent Document 4] Boulianne GL et al., Nature, 312, 643 (1984).
[Non-Patent Document 5] Jones PT. et al., Nature, 321, 522(1986).
[Non-Patent Document 6] Reichert JM. et al., Nature Biotechnology, 23, 1073 (2005).
[Non-Patent Document 7] Lipsky PE. et al., New England Journal of Medicine, 343, 1594 (2000).
[Non-Patent Document 8] Takai T. Nature Review Immunology, 2, 580 (2002).
[Non-Patent Document 9] Shields RL. et al., Journal of Biological Chemistry, 276, 6591 (2001).
[Non-Patent Document 10] Idusogie EE. et al., Journal of Immunology, 166, 2571 (2001).
[Non-Patent Document 11] Shields RL. et al., Journal of Biological Chemistry, 277, 26733 (2002).
[Non-Patent Document 12] Shinkawa T. et al., Journal of Biological Chemistry, 278, 3466, (2003).
[Non-Patent Document 13] Greenwood J. et al., Therapeutic Immunology, 1,247(1994).
[Non-Patent Document 14] Oettgen HC. et al., Hybridoma, 2, 17(1983).
[Non-Patent Document 15] Huhn D. et al., Blood, 98, 1326(2001).
[Non-Patent Document 16] Press OW. et al., Blood, 69, 584,(1987).

### Disclosure of the Invention

### [Problems to be Solved by the Invention]

The present invention was achieved in view of the circumstances described above. An objective of the present invention is to provide methods for enhancing an effector activity by altering the structure of antibody molecules, wherein the effector activity is ADCC activity. Another objective of the present invention is to provide methods for producing modified antibodies with enhanced effector activity, and such modified antibodies.

### [Means for Solving the Problems]

The present inventors conducted dedicated studies to achieve the objectives described above. Despite the above findings, the present inventors generated modified antibodies with tandemly linked Fc portions and assessed the effector activity of the modified antibodies. Surprisingly, the modified antibodies with tandemly linked Fc portions were confirmed to have significantly enhanced ADCC activity as compared with natural antibodies. According to the previous findings, the possibility that the enhanced ADCC activity of modified antibodies with parallelly-linked Fc is an effect of the hetero-divalent Fab could not be ruled out. Furthermore, considering that tandemly linked modified antibodies had a decreased CDC activity, the effect of the modified antibodies of the present invention was unexpected. Furthermore, modified antibodies having three Fc regions exhibited further enhanced ADCC activity than modified antibodies with two Fc regions. The enhanced ADCC activity of the modified antibodies of the present invention is inferred to be correlated with the number of Fc regions linked in tandem. The present inventors demonstrated that the effector activity of antibodies could be enhanced by tandemly linking Fc domains to antibodies, and thus completed the present invention.

Thus, the present invention relates to methods for enhancing the effector activity of antibodies by linking Fc domains in tandem. More specifically, the present invention provides the following:
[1] a method for enhancing an effector activity of an antibody, wherein one or more structures comprising an Fc domain of human IgG1 are linked in tandem to the C terminus of an antibody heavy chain by a genetic engineering technique, wherein the effector activity is antibody-dependent cellular cytotoxicity activity (ADCC activity), provided that when one structure comprising an Fc domain of human IgG1 is linked in tandem to the C terminus of said antibody heavy chain, said structure comprising an Fc domain of human IgG1 comprises a flexible glycine/serine spacer of 15 amino acid residues, in which the basic unit consists of four glycines and one serine, five amino acids in total, at the N-terminal side of the Fc domain of human IgG1;
[2] a method for producing a modified antibody with enhanced effector activity, wherein one or more structures comprising an Fc domain of human IgG1 are linked in tandem to the C terminus of an antibody heavy chain by a genetic engineering technique, wherein the effector activity is antibody-dependent cellular cytotoxicity activity (ADCC activity), provided that when one structure comprising an Fc domain of human IgG1 is linked in tandem to the C terminus of said antibody heavy chain, said structure comprising an Fc domain of human IgG1 comprises a flexible glycine/serine spacer of 15 amino acid residues, in which the basic unit consists of four glycines and one serine, five amino acids in total, at the N-terminal side of the Fc domain of human IgG1;
[3] a method for producing a modified antibody with enhanced effector activity, wherein the effector activity is antibody-dependent cellular cytotoxicity activity (ADCC activity), which comprises the steps of: (a) expressing a polynucleotide encoding a light chain and a polynucleotide encoding an altered heavy chain in which one or more structures comprising an Fc domain of human IgG1 are linked in tandem to the C terminus of an antibody heavy chain, provided that when one structure comprising an Fc domain of human IgG1 is linked in tandem to the C terminus of said antibody heavy chain, said structure comprising an Fc domain of human IgG1 comprises a flexible glycine/serine spacer of 15 amino acid residues, in which the basic unit consists of four glycines and one serine, five amino acids in total, at the N-terminal side of the Fc domain of human IgG1; and (b) collecting expression products of the polynucleotide;
[4] the method of any one of [1] to [3], wherein the structures comprise a spacer polypeptide at the N-terminal side of the Fc domain of human IgG1;
[5] the method of any one of [1] to [4], wherein the number of structures is two;
[6] the method of [4] or [5], wherein the spacer polypeptide according to [4] is selected from the group consisting of SEQ ID NOs: 48, 49, and 50;
[7] a modified antibody, wherein one or more structures comprising an Fc domain of human IgG1 are linked in tandem to the C terminus of an antibody heavy chain, provided that when one structure comprising an Fc domain of human IgG1 is linked in tandem to the C terminus of said antibody heavy chain, said structure comprising an Fc domain of human IgG1 comprises a flexible glycine/serine spacer of 15 amino acid residues, in which the basic unit consists of four glycines and one serine, five amino acids in total, at the N-terminal side of the Fc domain of human IgG1;
[8] the modified antibody of [7], wherein the structures comprise a spacer polypeptide at the N-terminal side of the Fc domain of human IgG1;
[9] the modified antibody of [7] or [8], wherein the number of structures is two;
[10] The modified antibody of any of [8] or [9], wherein the spacer polypeptide according to [8] is selected from the group consisting of SEQ ID NOs: 48, 49, and 50;
[11] the modified antibody of any one of [7] to [10] for use in medicine;
[12] the modified antibody of any one of [7] to [10] for use in a method for treating cancer;
[13] the method of any one of [1] to [6] or the modified antibody of any one of [7] to [10], wherein the said spacer of 15 amino acid residues is GGGGSGGGGSGGGGS;
[14] the method of any one of [1] to [6], wherein the antibody is an antibody against the B cell-specific differentiation antigen CD20;
[15] the modified antibody of any one of [7] to [10], which is an antibody against the B cell-specific differentiation antigen CD20.

### Brief Description of the Drawings

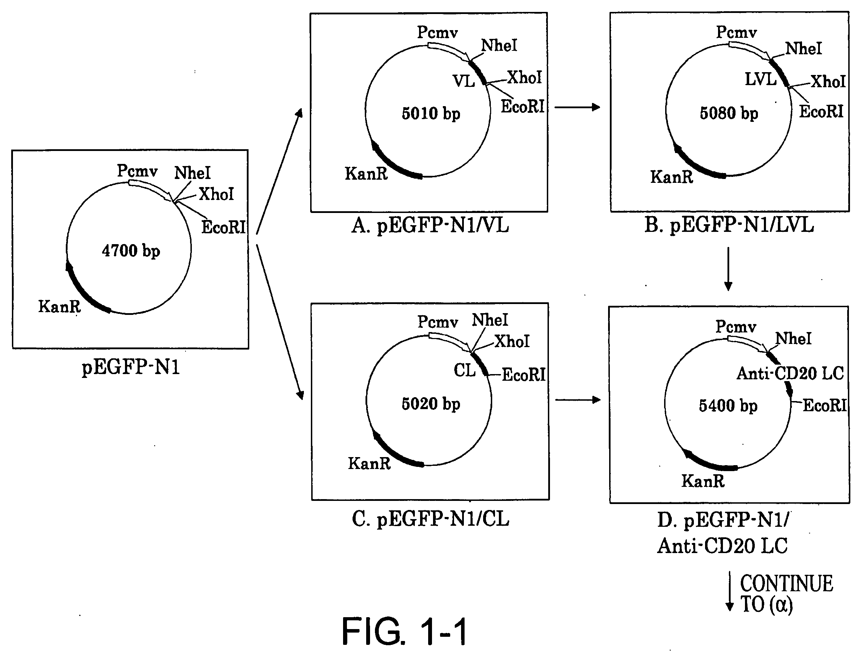

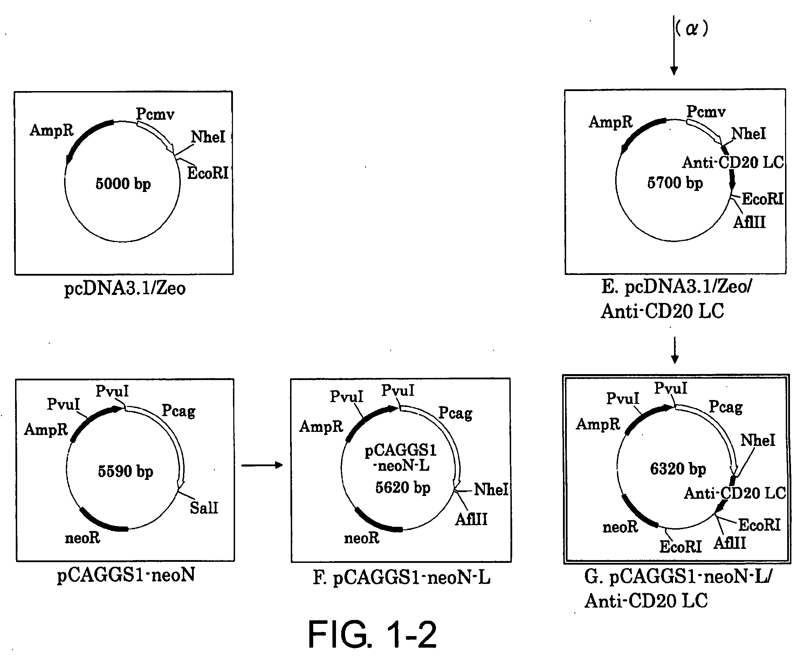

Fig. 1-1 shows the process for constructing the expression vector pCAGGS1-neoN-L/Anti-CD20 L Chain described in Example 1.
Fig. 1-2 is a continuation of Fig. 1-1.
Fig. 2-1 shows the process for constructing the expression vector pCAGGS1-dhfrN-L/Anti-CD20 H Chain described in Example 2.
Fig. 2-2 is a continuation of Fig. 2-1.
Fig. 3 shows the gene structure of the final H chain described in Example 2 and the corresponding primers (SEQ ID NOs: 34 to 45). In (1) to (12) of this figure, single underlines indicate restriction enzyme sites and double underlines indicate spacer sequences.
Fig. 4 is a schematic diagram of the antibodies generated in Example 3.
Fig. 5 shows gel filtration chromatograms after affinity purification with Protein A, described in Example 4. These are chromatograms obtained by gel filtration of M, RTX (Rituximab), D0, D1, D2, D3, T0, T1, T2, and T3 products.
Fig. 6 shows a result of PAGE analysis of antibodies after gel filtration described in Example 5. The result was obtained by carrying out SDS-PAGE under reducing conditions and Western blotting with horseradish peroxidase-labeled goat anti-human IgG (H+L) antibody or goat anti-human κ chain antibody.
Fig. 7 shows results of HPLC analysis of antibodies after gel filtration described in Example 5. HPLCs (gel filtrations) of purified M, RTX, D0, D1, D2, D3, T0, T1, T2, and T3 are shown.
Fig. 8 shows results of CD20-binding assay of antibodies by flow cytometry described in Example 6. (1): M, negative control trastuzumab, and positive control RTX. (2): D0, D1, D2, and D3. (3): T0, T1, T2, and T3.
Fig. 9-1 shows a result of receptor-binding assay by ELISA using recombinant FcγRIA described in Example 7.
Fig. 9-2 shows a result of receptor-binding assay by ELISA using recombinant FcγRIIA described in Example 7.
Fig. 9-3 shows a result of receptor-binding assay by ELISA using recombinant FcγRIIB described in Example 7.
Fig. 9-4 shows a result of receptor-binding assay by ELISA using recombinant FcγRIIIA (Val¹⁵⁸ type) described in Example 7.
Fig. 9-5 shows a result of receptor-binding assay by ELISA using recombinant FcγRIIIA (Phe¹⁵⁸ type) described in Example 7.
Fig. 10 shows a result of ADCC activity assay described in Example 8.
Fig. 11 shows a result of CDC activity assay described in Example 9.

### Best Mode for Carrying Out the Invention

As a novel method for enhancing the effector activity of antibodies, the present invention provides a "method for enhancing the effector activity of antibodies, which comprises linking in tandem one or more structures comprising an Fc domain of human IgG1 to the C terminus of an antibody heavy chain". The method of the present invention enables one to obtain modified antibodies with enhanced effector activity wherein the effector activity is ADCC activity as compared to the original antibodies (hereinafter also referred to as "modified antibodies of the present invention"), while the affinity of the original antibodies against antigens is maintained.

The origin of the antibodies of the present invention is not particularly limited. The antibodies may be derived, for example, from any of: primates such as human, monkey, and chimpanzee; rodents such as mouse, rat, and guinea pig; mammals such as rabbit, horse, sheep, donkey, cattle, goat, dog, and cat; or chicken. However, they are preferably derived from human. The antibodies of the present invention may be natural antibodies or antibodies into which some artificial mutations have been introduced. Furthermore, they may be so-called chimeric antibodies or humanized antibodies. The antibodies of the present invention may be immunoglobulins belonging to any class or any subclass. However, they preferably are from the IgG class, and more preferably from the IgG1 subclass.

The "structure comprising an Fc domain" of the present invention (hereinafter also referred to as the "structure of the present invention") may be the Fc domain itself of an antibody, or an appropriate oligopeptide may be linked as a spacer at the N terminus of the Fc domain.

In general, the Fc domain of an antibody is a fragment that is obtained after digesting an immunoglobulin molecule with papain. An Fc domain is constituted, from the N terminus of the heavy chain constant region, by the hinge region, the CH2 domain, and the CH3 domain. The two heavy chains are linked together via S-S bonds in the hinge region. The antibodies can bend in the hinge region. The two heavy chains of IgG1 are linked together via non-covalent bonds in the CH3 domains and disulfide bonds in the hinge regions. Fc domains have sugar chains; however, the sugar chains may contain mutations as long as the Fc domains have the ability to enhance the effector activity when linked to the C terminus of an antibody heavy chain. For example, the antibodies may lack α1,6-fucose in the sugar chains.

The origin of the Fc domains in the structure of the present invention may be the same as or different from that of the antibody to which the structure of the present invention is to be linked. From the viewpoint of immunogenicity, however, the origin is preferably the same as that of the antibody to which the structure of the present invention is to be linked, when the antibody is used as an antibody pharmaceutical. For example, when an antibody to which the structure of the present invention is linked is a human-derived antibody or a humanized antibody, the Fc domain in the structure of the present invention is preferably a human Fc domain. Many antibody heavy chain (H chain) sequences have been registered in public databases as genes for H chains of IgG1 including V regions. Examples include GenBank Accession No. BC019337 (a DNA sequence of human constant region). The nucleotide sequence of IgG1 heavy chain (leader sequence-CD20-derived V region (amino acid sequence of Accession No. AAL27650)-CH1-hinge-CH2-CH3) used in the Examples is shown in SEQ ID NO: 3 and the amino acid sequence is shown in SEQ ID NO: 4. The nucleotide sequence encoding human Fc domain corresponds to position 721 to 1413 in SEQ ID NO: 3. Fc domain cDNAs can be prepared by methods known to those skilled in the art. Fc domain cDNAs can be prepared, for example, by known nucleic acid amplification methods using primers designed based on the sequence from position 721 to 1413 in SEQ ID NO: 3 and, as template, mRNAs prepared from antibody-expressing cells. Alternatively, they may be prepared by using as a probe a portion of the sequence of SEQ ID NO: 3 and selecting sequences that hybridize to the probe from a cDNA library prepared from antibody-expressing cells.

Furthermore, the Fc domains in the structure of the present invention may comprise spontaneous or artificial mutations as long as they have the Fc receptor-binding activity. For example, polypeptides encoded by sequences that hybridize under stringent conditions to the complementary strand of the nucleotide sequence from position 721 to 1413 in SEQ ID NO: 3, and polypeptides comprising an amino acid sequence with a substitution, deletion, addition, and/or insertion of one or more amino acids in the sequence from position 241 to 471 in the amino acid sequence of SEQ ID NO: 4 are also included in the Fc domain of the structures of the present invention, as long as they have Fc receptor-binding activity. Such Fc domain variants can also be prepared by methods known to those skilled in the art.

Those skilled in the art can appropriately select the above stringent hybridization conditions. For example, pre-hybridization is carried out in a hybridization solution containing 25% formamide, or 50% formamide under more stringent conditions, and 4x SSC, 50 mM Hepes (pH7.0), 10x Denhardt's solution, and 20 µg/ml denatured salmon sperm DNA at 42°C overnight. Labeled probes are then added and hybridization is carried out by incubation at 42°C overnight. Post-hybridization washes are carried out at different levels of stringency, including the moderately stringent "1x SSC, 0.1% SDS, 37°C", highly stringent "0.5x SSC, 0.1% SDS, 42°C", and more highly stringent "0.2x SSC, 0.1% SDS, 65°C" conditions. As the stringency of the post-hybridization washes increases, polynucleotides with greater homology to the probe sequence are expected to be isolated. The above-described combinations of SSC, SDS, and temperature conditions are mere examples. Those skilled in the art can achieve the same stringencies as those described above by appropriately combining the above factors or others (such as probe concentration, probe length, or hybridization period) that affect hybridization stringency.

Polypeptides encoded by polynucleotides isolated using such hybridization techniques will usually comprise amino acid sequences with high homology to the Fc domains described above. "High homology" refers to sequence homology of at least 40% or more, preferably 60% or more, further preferably 80% or more, further preferably 90% or more, further preferably at least 95% or more, and further preferably at least 97% or more (for example, 98% to 99%). Amino acid sequence identity can be determined, for example, using the BLAST algorithm of Karlin and Altschul (Proc. Natl. Acad. Sci. USA (1990) 87, 2264-2268; Proc. Natl. Acad. Sci. USA (1993) 90, 5873-5877). A program called BLASTX has been developed based on this algorithm (Altschul et al., J. Mol. Biol. (1990) 215, 403-410). When using BLASTX to analyze amino acid sequences, the parameters are, for example, a score of 50 and a word length of 3. When using the BLAST and Gapped BLAST programs, the default parameters for each program are used. Specific methodology for these analysis methods is well known (http://www.ncbi.nlm.nih.gov).

Furthermore, techniques for artificially preparing Fc domain variants by artificially introducing mutations into Fc domains are also known to those skilled in the art. Such Fc domain variants can be artificially prepared, for example, by introducing site-specific or random mutations into the nucleotide sequence of SEQ ID NO: 3 by genetic modification methods, such as PCR-based mutagenesis or cassette mutagenesis. Alternatively, sequences with mutations introduced into the nucleotide sequence of SEQ ID NO: 3 can be synthesized using commercially available nucleic acid synthesizers.

The structures comprised by the modified antibody of the present invention may not have any spacer oligopeptide. However, when one structure comprising an Fc domain of human IgG1 is linked in tandem to the C terminus of said antibody heavy chain, said structure comprising an Fc domain of human IgG1 comprises a flexible glycine/serine spacer of 15 amino acid residues, in which the basic unit consists of four glycines and one serine, five amino acids in total, at the N-terminal side of the Fc domain of human IgG1. In addition, the structures preferably contain spacer oligopeptides. Combinations of glycine and serine are often used as the spacer (Journal of Immunology, 162, 6589 (1999)). As described in the Examples, a spacer having a combination of four glycines and one serine (SEQ ID NO: 48), or a spacer in which the above sequence is linked twice (SEQ ID NO: 49) or three times (SEQ ID NO: 50) can be used as the spacer of the present invention. However, the spacer is not limited to these sequences. The spacer may have any structure as long as it allows bending of the hinge region where the spacer is linked. Preferably, a spacer is a peptide sequence that is not readily cleaved by proteases or peptidases. Regarding such sequences, a desired peptide sequence can be obtained, for example, by entering various conditions such as sequence length into LINKER (Xue F, Gu Z, and Feng JA., LINKER: a web server for generating peptide sequences with extended conformation, Nucleic Acids Res. 2004 Jul 1; 32 (Web Server issue):W562-5), a program that assists designing of linker sequences. LINKER can be accessed at http://astro.temple.edu/∼feng/Servers/BioinformaticServers.htm.

When multiple nucleotide sequences are linked together by genetic engineering techniques, one to several residues in the amino acid sequence at the junction are often substituted, deleted, added, and/or inserted, for example, because of the sequences of the restriction enzyme sites. Such mutations are known to those skilled in the art. Such mutations may also occur when the structures of the present invention are constructed or when they are linked to antibodies. Such mutations may occur, for example, at the junctions between the V and C regions, and the junctions between the C terminus of Fc and the N terminus of the structures of the present invention (the N terminus of Fc or spacer). Even with such mutations, they are included in the structures or modified antibodies of the present invention as long as they have an Fc receptor-binding activity.

The structures comprised by the modified antibody of the present invention can enhance the effector activity of an antibody when linked to the C terminus of the antibody heavy chain, wherein the effector activity is ADCC activity. An arbitrary number of structures of the present invention, for example, one, two, three, four, or five structures, may be linked; however, one or two structures are preferably linked. Therefore, modified antibodies onto which the structures of the present invention have been linked can comprise two or more arbitrary Fc domains, and the number of Fc domains in a modified antibody is two or three. In the Examples, modified antibodies into which two structures of the present invention have been linked were confirmed to show a stronger ADCC activity than modified antibodies into which one structure of the present invention has been linked.

There is no limitation on the type of antigen that is recognized by an antibody to which the structures of the present invention are to be linked. The antigen may be any antigen. Specifically, the variable region of a modified antibody of the present invention may recognize any antigen. The H chain and L chain variable regions of the modified antibodies used in the Examples described below are the variable regions of 1 F5, which is a mouse monoclonal antibody against CD20, a differentiation antigen of human B lymphocytes (Non-Patent Document 14). CD20 is a protein of 297 amino acids, and its molecular weight is 33 to 37 kDa. CD20 is highly expressed in B lymphocytes. Rituximab, a chimeric antibody against CD20, is widely used as an effective therapeutic agent for Non-Hodgkin's lymphoma (Non-Patent Document 15). Known anti-CD20 antibodies include mouse monoclonal antibodies B1 and 2H7, in addition to Rituximab and 1F5 (Non-Patent Document 16). However, the variable regions of the modified antibodies of the present invention are not limited to the variable regions of 1 F5. The Fc domains are physically distant from the Fab domains; therefore, it is thought that the Fab domain type has almost no influence on the Fc domain activity. Thus, the variable regions of any antibody other than 1F5 may be used as the variable regions of the modified antibodies of the present invention, and the variable regions of antibodies directed to any antigen other than CD20 may be used as the variable regions of the modified antibodies of the present invention.

Regardless of the antigen-binding activity, the methods of the present invention can increase the therapeutic effect of an antibody by enhancing the effector activity exhibited by the antibody Fc domain. The enhancement of the binding activity to Fc receptors is required to increase the effector activity, wherein the effector activity is ADCC activity, of an antibody. In general, the intensity of binding between two molecules is considered to be as follows. The binding intensity between one antibody molecule and one Fc receptor molecule is represented by: affinity x binding valency = avidity. Natural IgG antibodies have one Fc; thus, the binding valency is 1 even when there are many Fc receptors on the surface of effector cells. However, when antibodies and antigens form complexes, the immune complexes bind to the effector cells in a multivalent manner. The binding valency varies depending on the structure of immune complexes. Cancer cells have many antigens on the cell surface; thus, antibodies bind to these antigens and bind to Fc receptors on the effector cells in a multivalent manner. However, the density of antigens on cancer cells is often low; thus, antibodies bound to the antigens bind with lower valency to Fc receptors. For this reason, ADCC activity cannot be sufficiently exerted and the therapeutic effect is also insufficient (Golay, J. et al., Blood, 95, 3900, (2000)). However, by tandemly linking multiple Fc domains to an antibody molecule, binding to Fc receptors in a multivalent manner is possible. This enhances the binding activity between an antibody and Fc receptors, i.e. the avidity. In addition, the effector activity is enhanced.

The present invention also provides methods for producing modified antibodies with enhanced effector activity. The methods of the present invention not only enhance the effector activity of naturally obtained antibodies and existing chimeric antibodies, but also enable the production of novel altered chimeric antibodies from novel combination of antibody variable and constant regions of different origins. Furthermore, the modified antibodies may also comprise a novel constant region from the combination of CH1 domain and two or more Fc domain variants described above. The nucleotide sequence of human CH1 domain is shown under positions 430 to 720 in the heavy chain nucleotide sequence of SEQ ID NO: 3.

The methods of the present invention can be conducted using an appropriate combination of methods known to those skilled in the art. Thereby, the linking of the structures comprising an Fc domain of human IgG1 to the C terminus of an antibody heavy chain is performed by a genetic engineering technique. An example of expression of the modified antibodies of the present invention is described below, in which DNAs for the heavy chain (H chain) and light chain (L chain) variable and constant regions are prepared and linked using genetic engineering techniques.

The variable region sequences can be prepared, for example, by the following procedure. First, a cDNA library is generated from hybridomas expressing the antibody of interest or cells introduced with the antibody gene, and DNA for the variable region of interest is cloned. An antibody leader sequence L is linked upstream of the H chain variable region VH and L chain variable region VL to construct the DNA structures [LVH] and [LVL].

For the antibody constant region, first, a cDNA library is generated from human myeloma cells or human lymphatic tissues such as tonsil. cDNA fragments for the H chain constant region [CH1-Fc] and for the L chain constant region [CL] are obtained by amplification by known nucleic acid amplification methods such as polymerase chain reaction (PCR) using primers designed based on partial sequences of the 5' and 3' ends of H chain and L chain constant regions. The fragments are then inserted into vectors and cloned.

For the L chain, the DNA structure [LVL-CL] is constructed in which LVL and CL are linked. As an example, the DNA sequence of the DNA structure [LVL-CL] (leader sequence, V region of 1F5, and C region of human Lκ chain) prepared in the Examples is shown in SEQ ID NO: 1, while the amino acid sequence encoded by the DNA structure is shown in SEQ ID NO: 2. The H chain linked with a structure of the present invention (altered H chain) and the H chain without a structure of the present invention linked are constructed by the procedure described below. (i) The DNA structure of H chain having a single Fc (H chain without a structure of the present invention linked) can be constructed by linking together the DNA structure [LVH] and DNA structure [CH1-Fc domain-stop codon]. The DNA sequence of the DNA structure for the H chain with a single Fc prepared in the Examples is shown in SEQ ID NO: 3, while the amino acid sequence encoded by the DNA structure is shown in SEQ ID NO: 4. (ii) The DNA structure of H chain having two Fc linked in tandem (H chain linked with one structure of the present invention) can be constructed by linking together the DNA structure [LVH], DNA structure [CH1-Fc (without stop codon)], and DNA structure [spacer-Fc-stop codon]. The DNA sequences of the DNA structures for the H chain having two Fc linked in tandem prepared in the Examples are shown in SEQ ID NOs: 5 (with no spacer), 7 (with a single spacer: GGGGS (represented as G4S; SEQ ID NO: 48)), 9 (with two G4S as spacer), and 11 (with three G4S as spacer). The amino acid sequences encoded by these DNA structures are shown in SEQ ID NOs: 6 (with no spacer), 8 (with one G4S as spacer), 10 (with two G4S as spacer), and 12 (with three G4S as spacer). (iii) The DNA structure of H chain having three Fcs linked in tandem (H chain linked with two structures of the present invention) can be constructed by linking together the DNA structure [LVH], DNA structure [CH1-Fc (without stop codon)], DNA structure [spacer-Fc- (without stop codon)], and DNA structure [spacer-Fc-stop codon]. The DNA sequences of the DNA structures for the H chain having three Fcs linked in tandem prepared in the Examples are shown in SEQ ID NOs: 13 (with no spacer), 15 (with one G4S as spacer), 17 (with two G4S as spacer), and 19 (with three G4S as spacer). The amino acid sequences encoded by these DNA structures are shown in SEQ ID NOs: 14 (with no spacer), 16 (with one G4S as spacer), 18 (with two G4S as spacer), and 20 (with three G4S as spacer). (iv) The DNA constructs of H chain having four or more Fc linked in tandem can be prepared similarly as in the case with three Fcs, by increasing the number of the DNA structure [spacer-Fc-(without stop codon)].

The L chain DNA structure and altered H chain DNA structure prepared as described above are cloned, and then, together with regulatory regions such as promoter and enhancer, inserted into expression vectors. Alternatively, they may be inserted into expression vectors that already have regulatory regions. Expression vectors that can be used include vectors having the CAG promoter (Gene, 108, 193 (1991)) and pcDNA vector (Immunology and Cell Biology, 75, 515 (1997)). Any expression vectors may be used as long as they are compatible with host cells to be used.

Host cells can be appropriately selected from those that can express glycoproteins. Such host cells can be selected, for example, from animal cells, insect cells, yeast, and the like. Specific examples include CH-DG44 cells (Cytotechnology, 9, 237 (1992)), COS-1 cells, COS-7 cells, mouse myeloma NS0 cells, and rat myeloma YB2/0 cells which can produce antibody molecules having sugar chains lacking fucose, but the cells are not limited thereto.

The recombinant host cells are cultured and modified antibodies are purified from the culture supernatants. Various types of culture media can be used for culture; however, serum-free media are convenient for purifying antibodies. Modified antibodies of interest are purified from the culture supernatants by removing fragments and aggregates of the modified antibodies, and proteins other than the modified antibodies with known purification methods, such as ion exchange chromatography, hydrophobic chromatography, gel filtration chromatography, affinity chromatography with immobilized Protein A having selective binding activity to antibodies or the like, and high performance liquid chromatography (HPLC).

Whether the modified antibodies obtained as described above have enhanced effector activity can be assessed by methods known to those skilled in the art. The binding activity to various Fc receptors can be determined, for example, by enzyme antibody techniques using the extracellular domains of recombinant Fc receptors. A specific example is described below. First, the extracellular domains of FcγRIA, FcγRIIA, FcγRIIB, and FcγRIIIA, are produced as receptors for human IgG. The sequences of these receptors are known, and are available from GenBank under the following Accession Nos: human FcγRIA: NM_000566, human FcγRIIA: NM_021642, human FcγRIIB: NM_001002273, human FcγRIIIA: NM_000569. These receptors are immobilized onto 96-well plates for enzyme antibody techniques. Modified antibodies with varied concentrations are reacted, and labeled anti-human IgG antibodies or such are reacted as a secondary antibody. The amounts of modified antibodies bound to the receptors are measured based on the signal from the label. There are known genetic variants of FcγRIIIA (Journal of Clinical Investigation, 100, 1059 (1997)), and the receptors in which the amino acid at position 158 is valine or phenylalanine are used in the Examples herein.

The ADCC activity of modified antibodies can be measured using effector and target cells. For example, monocytes separated from peripheral blood of healthy individuals can be used as the effector cells. Cells expressing the CD20 antigen, such as Ramos cells and Raji cells, can be used as the target cells. After the target cells are reacted with serially diluted modified antibodies, the effector cells are added. The ratio of the effector to target cell numbers can be in a range of 10 : 1 to 100 : 1, and the ratio is preferably 25 : 1. When the target cells are damaged by the ADCC activity of the modified antibodies, lactate dehydrogenase (LDH) in the cells is released into the culture supernatant. Therefore, the ADCC activity can be determined by collecting the released LDH and measuring its enzymatic activity. As the modified antibody of the present invention enhances ADCC activity, this assay is used when assessing whether the modified antibody has enhanced effector activity.

As for the CDC activity, the cytotoxic activity can be assessed, for example, by reacting the target cells with serially diluted modified antibodies and then adding fresh baby rabbit serum as a source of complements, as described in the Examples. Since serum containing LDH is used, the cytotoxic activity is assessed by measuring the viable cell number using Alamar Blue or such methods.

Modified antibodies obtained by the methods of the present invention not only enhance the *in vitro* effector activity described above but also exert the cellular immunity-enhancing effect *in vivo* based on enhanced effector activity. Thus, the antibodies are thought to contribute to the treatment of diseases that can be expected to be improved by cellular immunity. The modified antibodies of the present invention can be administered in an appropriate dosage form via an appropriate administration route, depending on the type of disease, patient's age, symptoms, and such. Furthermore, the modified antibodies of the present invention can be formulated by known formulation methods and supplied as pharmaceuticals along with instructions indicating the efficacy and effects, cautions for use, and so on. When formulating, appropriate additives, such as pharmaceutically acceptable excipients, stabilizers, preservatives, buffers, suspending agents, emulsifiers, and solubilizing agents can be appropriately added depending on the purpose, such as securing their properties and quality. For example, the antibodies can be combined with Polysorbate 80, sodium chloride, sodium citrate, anhydrous citric acid, or such when formulating as injections, prepared with physiological saline or glucose solution injection at the time of use, and administered by intravenous drip infusion or such method. The dose can be adjusted depending on the patient's age and weight, and such factors. A single dose in such intravenous drip infusion is, for example, 10 to 10000 mg/m², preferably 50 to 5000 mg/m², and more preferably 100 to 1000 mg/m², but is not limited thereto.

All prior art references cited herein are incorporated by reference into this description.

### Examples

Herein below, the present invention is specifically described in the context of Examples; however, it is not to be construed as being limited thereto.

### [Example 1] Construction of expression vector pCAGGS1-neoN-L/anti-CD20 LC (Light Chain)

### 1-1. Preparation of pEGFP-N1/VL vector (Fig. 1-1-A)

The mouse anti-CD20 IgG2a VL region gene was cloned by the following procedure. The mouse hybridoma 1F5 was cultured using RPMI1640 containing 10% inactivated fetal bovine serum, 100 U/ml penicillin, and 100 µg/ml streptomycin (Sigma Aldrich) at 37°C under 5% CO₂, and then total RNA was extracted from the cells using ISOGEN (NIPPON GENE CO.). 10 pmol of oligo dT primer (5'- CGAGCTCGAGCGGCCGCTTTTTTTTTTTTTTTTTT-3' (SEQ ID NO: 21)) was added to 10 µg of the total RNA, and the total volume was adjusted to 12 µl by adding diethylpyrocarbonate (DEPC)-treated water. After two minutes of incubation at 72°C to destroy its higher order structure, the RNA was quickly transferred onto ice and incubated for three minutes. The RNA was added with 2 µl of the appended 10x Reaction Buffer (Wako Pure Chemical Industries, Ltd.), 1 µl of 100 mM DTT (Wako Pure Chemical Industries, Ltd.), 1 µl of 20 mM dNTP (Wako Pure Chemical Industries, Ltd.), and 1 µl of 20 U/µl RNase Inhibitor (Wako Pure Chemical Industries, Ltd.). The total volume was adjusted to 19 µl with DEPC-treated water. After heating up to 42°C, 1 µl of 200 U/µl ReverscriptII (Wako Pure Chemical Industries, Ltd.) was added and the resulting mixture was incubated at 42°C for 50 minutes without further treatment. After reaction, 80 µl of TE (1 mM EDTA, 10 mM Tris-HCl (pH 8.0)) was added. The resulting 100-µl mixture was used as a cDNA solution.

7.8 µl of sterile MilliQ water, 4 µl of the appended 5x Buffer, 2µl of 2.5 mM dNTP, 2 µl of 10 µM forward primer (5'- TCGTCTAGGCTAGCATTGTTCTCTCCCAGTCTCCA-3' (SEQ ID NO: 22) having an *Nhe*I site (underlined)), 2 µl of 10 µM reverse primer (5'-GCTTGAGACTCGAGCAGCTTGGTCCCAGCAC CGAA-3' (SEQ ID NO: 23) having an *Xho*I site (underlined)), 2 µl of 1F5-derived cDNA as a template, and 0.2 µl of 5 U/µl Expand High Fidelity^{PLUS} PCR system (Roche) were combined together on ice. PCR was carried out under the following conditions: heat treatment at 95°C for ten minutes, followed by 30 cycles of 95°C for 30 seconds, 60°C for 30 seconds, and 72°C for 60 seconds. The reaction solution was subjected to electrophoresis using 1% agarose STANDARD 01 (Solana) gel and a band of about 0.31 kbp was collected using RECOCHIP (TaKaRa Bio Inc.). The DNA fragment was purified by phenol/chloroform extraction and isopropyl alcohol precipitation. The DNA fragment was treated with *Nhe*I (TOYOBO) and *Xho*I (TaKaRa Bio Inc.) at the final concentrations of 0.8 U/µl and 0.5 U/µl, respectively, and ligated with 50 ng of pEGFP-N1 (BD Biosciences) treated in the same way. The ligation was carried out using 1.5 U of T4 DNA ligase (Promega) at room temperature for 30 minutes. 100 µl of competent cells of *E*. *coli* DH5α, which had been prepared by the potassium chloride method, was added to the reaction mixture. After reacting for 30 minutes on ice, the cells were heat-shocked at 42°C for 45 seconds. This was then rested for two minutes on ice, after which 1 ml of SOC medium (2% Bacto tryptone (BD Biosciences), 0.5% Bacto yeast extract (BD Biosciences), and 1% sodium chloride (Wako Pure Chemical Industries, Ltd.)) was added. The resulting mixture was transferred into a test tube and the bacteria were cultured with shaking at 37°C for two hours. After shaking, 100 µl of the bacterial suspension was plated onto an LB medium plate containing 100 µg/ml of kanamycin (Wako Pure Chemical Industries, Ltd.). The plate was incubated at 37°C overnight. From the formed colonies, those into which the VL region gene has been inserted were selected, and the vector was named pEGFP-N1/VL.

### 1-2. Preparation of pEGFP-N1/LVL vector (Fig. 1-1-B)

A leader sequence was added to the VL gene by the following procedure. 4 µl of the appended 5x Buffer, 2 µl of 2.5 mM dNTP, 2 µl of 10 µM forward primer (5'-GAGTTT GCTAGCGCCGCCATGGATTTTCAAGTGCAGATTTTCAGCTTCCTGCTAATCAGTGCTT CAGTCATAATGTCCAGAGGACAAATTGTTCTCTCCCAGTCTCCAGCA-3' (SEQ ID NO: 24) having an *Nhe*I site (underlined); the leader sequence corresponds to positions 13 to 57 in SEQ ID NO: 24), 2 µl of 10 µM reverse primer (5'-GCTTGAGACTCGAGCAGCTTGGTCCCAGCACCGAA-3' (SEQ ID NO: 25) having an *Xho*I site (underlined)), 100 ng of pEGFP-N1/VL as a template, which had been prepared as described in (1), and 0.2 µl of 5 U/µl Expand High Fidelity^{PLUS} PCR system were combined together on ice. The total volume was adjusted to 20 µl by adding sterile MilliQ water. PCR was carried out under the following conditions: heat treatment at 95°C for ten minutes, followed by 30 cycles of 95°C for 30 seconds, 60°C for 30 seconds, and 72°C for 60 seconds. The reaction solution was subjected to electrophoresis using 1% agarose STANDARD 01 gel. A band of about 0.38 kbp was recollected using RECOCHIP. The DNA fragment was purified by phenol/chloroform extraction and isopropyl alcohol precipitation.

The DNA fragment was treated with *Nhe*I and *Xho*I at final concentrations of 0.8 and 0.5 U/µl, respectively, and ligated with 50 ng of pEGFP-N1 treated in the same way. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, after which 1 ml of SOC medium was added. The resulting mixture was transferred into a test tube and the bacteria were cultured with shaking at 37°C for two hours. After shaking, 100 µl of the bacterial suspension was plated onto an LB medium plate containing 100 µg/ml of kanamycin. The plate was incubated at 37°C overnight. From the formed colonies, those into which a DNA having the VL region gene with an added leader sequence has been inserted were selected, and the vector was named pEGFP-N1/LVL.

### 1-3. Preparation of pEGFP-N1/CL vector (Fig. 1-1-C)

The human κ chain C region gene was cloned by the following procedure. The human myeloma RPMI8226 was cultured using RPMI1640 containing 10% inactivated fetal bovine serum, 100 U/ml penicillin, and 100 µg/ml streptomycin at 37°C under 5% CO₂, and then total RNA was extracted from the cells using ISOGEN. 10 pmol of oligo dT primer was added to 10 µg of the total RNA, and the total volume was adjusted to 12 µl by adding DEPC-treated water. After two minutes of incubation at 72°C, the RNA was quickly transferred onto ice and incubated for three minutes. The RNA was added with 2 µl of the appended 10x Reaction Buffer, 1 µl of 100 mM DTT, 1 µl of 20 mM dNTP, and 1 µl of 20 U/µl RNase Inhibitor, and the total volume was adjusted to 19 µl with DEPC-treated water. After heating up to 42°C, 1 µl of 200 U/µl ReverscriptII was added and the resulting mixture was incubated at 42°C for 50 minutes without further treatment. After reaction, 80 µl of TE was added. The resulting 100-µl mixture was used as a cDNA solution. 4 µl of the appended 5x Buffer, 2 µl of 2.5 mM dNTP, 2 µl of 10 µM forward primer (5'-ACCTCTAACTCGAGACTGTGGCTGCACC ATCTGT-3' (SEQ ID NO: 26) having an *Xho*I site (underlined)), 2 µl of 10 µM reverse primer (5'- ACTTGAATTCCTAACACTCT CCCCTGTTGA -3' (SEQ ID NO: 27) having an *Eco*RI site (underlined)), 2 µl of RPMI8226-derived cDNA as a template, and 0.2 µl of 5 U/µl Expand High Fidelity^{PLUS} PCR system were combined together on ice. The total volume was adjusted to 20 µl by adding sterile MilliQ water. PCR was carried out under the following conditions: heat treatment at 95°C for ten minutes, followed by 30 cycles of 95°C for 30 seconds, 55°C for 30 seconds, and 72°C for 30 seconds. The reaction solution was subjected to electrophoresis using 1% agarose STANDARD 01 gel and a band of about 0.32 kbp was collected using RECOCHIP. The DNA fragment was purified by phenol/chloroform extraction and isopropyl alcohol precipitation. This was then treated with *Xho*I and *Eco*RI (TOYOBO), both at a final concentration of 0.5 U/µl, and ligated with 50 ng of pEGFP-N1 treated in the same way. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, and 1 ml of SOC medium was added. The resulting mixture was transferred into a test tube and the bacteria were cultured with shaking at 37°C for two hours. After shaking, 100 µl of the bacterial suspension was plated onto an LB medium plate containing 100 µg/ml of kanamycin. The plate was incubated at 37°C overnight. From the formed colonies, those into which the CL region gene has been inserted were selected, and the vector was named pEGFP-N1/CL.

### 1-4. Preparation of pEGFP-N1/Anti-CD20 LC vector (Fig. 1-1-D)

The mouse/human chimeric anti-CD20 L chain gene (the DNA sequence is shown in SEQ ID NO: 1, and the amino acid sequence is shown in SEQ ID NO: 2) was constructed by the following procedure. 0.7 µg of pEGFP-N1/CL was treated with *Xho*I and *Eco*RI, both at a final concentration of 0.5 U/µl. After the whole reaction mixture was subjected to electrophoresis using 1% agarose STANDARD 01 gel, the insert DNA fragment of about 0.32 kbp was collected using RECOCHIP with considerable care not to contaminate it with vector fragments. Separately, pEGFP-N1/LVL vector was treated with *Xho*I and *Eco*RI under the same conditions. Both DNA fragments were purified by phenol/chloroform extraction and isopropyl alcohol precipitation. 50 ng of pEGFP-N1/LVL treated with the restriction enzymes was mixed and ligated with the excised human CL region gene. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, and combined with 1 ml of SOC medium. The resulting mixture was transferred into a test tube and the bacteria were cultured with shaking at 37°C for two hours. After shaking, 100 µl of the bacterial suspension was plated onto an LB medium plate containing 100 µg/ml of kanamycin. The plate was incubated at 37°C overnight. From the formed colonies, those into which a DNA for the VL region gene added with the leader sequence and the human CL region gene has been inserted were selected, and the vector was named pEGFP-N1/Anti-CD20 LC.

### 1-5. Preparation of pcDNA3.1/Zeo/Anti-CD20 LC vector (Fig. 1-2-E)

The mouse/human chimeric anti-CD20 L chain gene was transferred from pEGFP-N1 vector to pcDNA3.1/Zeo by the following procedure. 0.5 µg of pEGFP-N1/Anti-CD20 L Chain was treated with *Nhe*I and *Eco*RI at final concentrations of 0.8 U/µl and 0.5 U/µl, respectively. After the whole reaction mixture was subjected to electrophoresis using 1% agarose STANDARD 01 gel, the insert DNA fragment of about 0.70 kbp was collected using RECOCHIP with considerable care not to contaminate it with vector fragments. Separately, pcDNA3.1/Zeo vector (Invitrogen) was treated wit *Nhe*I and *Eco*RI under the same conditions. Both DNA fragments were purified by phenol/chloroform extraction and isopropyl alcohol precipitation. 50 ng of pcDNA3.1/Zeo treated with the restriction enzymes was mixed and ligated with the excised Anti-CD20 LC gene. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, and the whole mixture was plated onto an LB medium plate containing 100 µg/ml of ampicillin (Sigma Aldrich). The plate was incubated at 37°C overnight. From the formed colonies, those into which a DNA for the anti-CD20 LC gene has been inserted were selected, and the vector was named pcDNA3.1/Zeo/Anti-CD20 LC.

### 1-6. Preparation of pCAGGS1-neoN-L (Fig. 1-2-F)

A spacer was inserted into the expression vector pCAGGS1-neoN containing the CAG promoter and neomycin resistance gene by the following procedure. pCAGGS1-neoN was treated with *Sal*I at a final concentration of 0.5 U/ml. The resulting DNA fragment was purified by phenol/chloroform extraction and isopropyl alcohol precipitation. Separately, two DNA strands (sense DNA: GTCGACGCTAGCAAGGATCCTTGAATTCCTTAAGG (SEQ ID NO: 28); antisense DNA: GTCGACCTTAAGGAATTCAAGGATCCTTGCTAGCG (SEQ ID NO: 29)) were synthesized. These DNAs were mixed together at a final concentration of 1 µM, and the total volume was adjusted to 10 µl with MilliQ water. After five minutes of heating at 75°C, the mixture was rested at room temperature to gradually cool it. 1 µl of this solution was mixed and ligated with 50 ng of *Sal*I-treated pCAGGS1-neoN. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, and the whole mixture was plated onto an LB medium plate containing 100 µg/ml of ampicillin. The plate was incubated at 37°C overnight. A pCAGGS1-neoN vector having an inserted spacer was selected from the formed colonies. The resulting vector was named pCAGGS1-neoN-L. As a result of the spacer insertion, two *Sal*I sites were newly generated in pCAGGS1-neoN, and the sequence of restriction enzyme sites became 5'-SalI-NheI-BamHI-EcoRI-AflII-SalI-3'.

### 1-7. Preparation of pCAGGS1-neoN-L/Anti-CD20 LC vector (Fig. 1-2-G)

The mouse/human chimeric anti-CD20 L chain gene was transferred from pcDNA3.1/Zeo vector to pCAGGS1-neoN-L vector by the following procedure. 0.5 µg of pcDNA3.1/Zeo/Anti-CD20 LC was treated with *Nhe*I and *Afl*II (New England Biolabs) at final concentrations of 0.8 U/µl and 1.0 U/µl, respectively. After the whole reaction mixture was subjected to electrophoresis using 1% agarose STANDARD 01 gel, the insert DNA fragment of about 0.70 kbp was collected using RECOCHIP with considerable care not to contaminate it with vector fragments. Separately, pCAGGS1-neoN-L was treated with *Nhe*I and *Afl*II under the same conditions. Both DNA fragments were purified by phenol/chloroform extraction and isopropyl alcohol precipitation. 50 ng of pCAGGS1-neoN-L treated with the restriction enzymes was mixed and ligated with the excised Anti-CD20 L Chain gene. The ligation was carried out using 1.5 U of T4 DNA ligase at room temperature for 30 minutes. 100 µl of competent cells *of E. coli* DH5α was added to the reaction mixture. After reacting for 30 minutes on ice, the cells were heat-shocked at 42°C for 45 seconds. This was then rested for two minutes on ice, and the whole mixture was plated onto an LB medium plate containing 100 µg/ml of ampicillin. The plate was incubated at 37°C overnight. From the formed colonies, those into which a DNA for the anti-CD20 LC gene has been inserted were selected, and the vector was named pCAGGS1-neoN-L/Anti-CD20 LC.

### [Example 2] Construction of expression vector pCAGGS1-dhfrN-L/anti-CD20 HC (Heavy Chain)

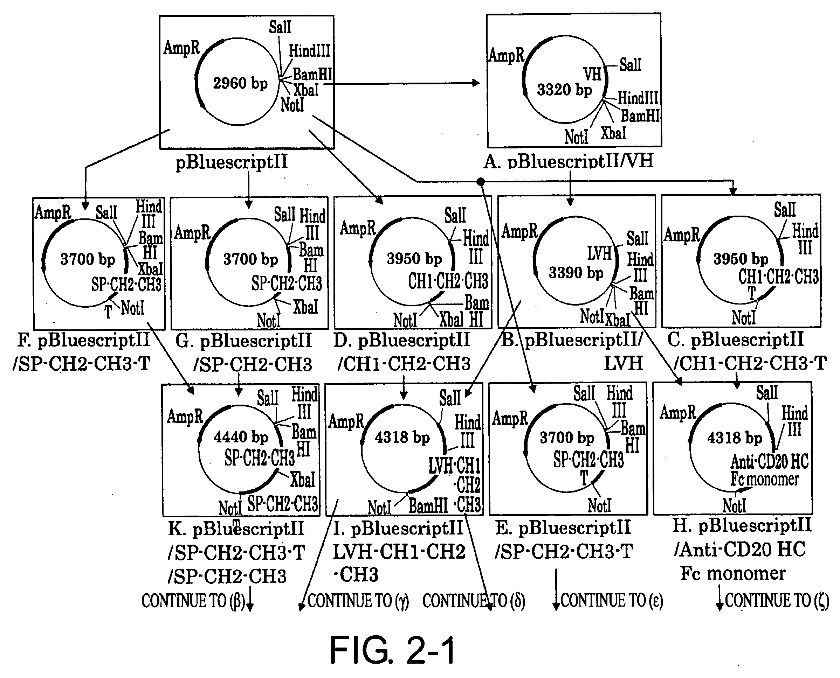

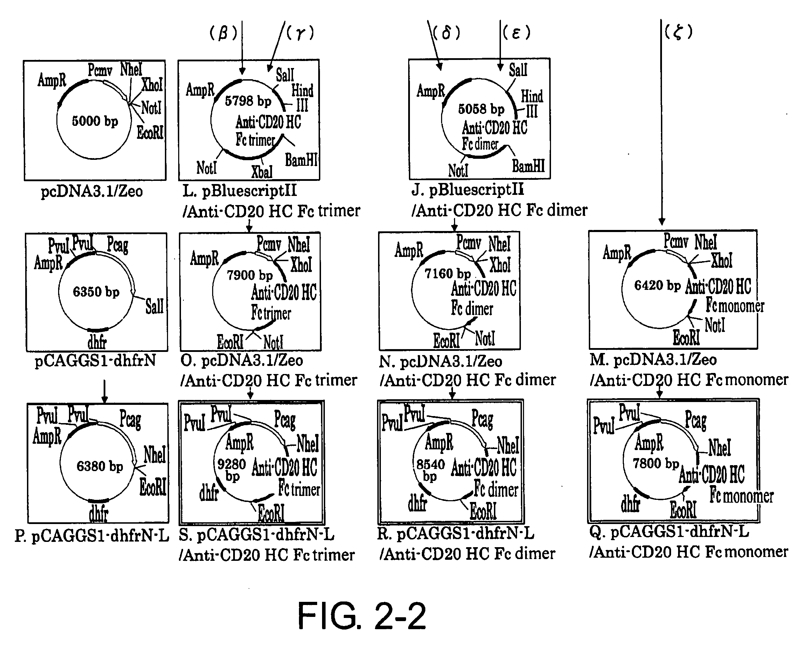

### 2-1. Preparation of pBluescriptII/VH vector (Fig. 2-1-A)

The mouse anti-CD20 IgG2a VH region gene was cloned by the following procedure. 7.8 µl of sterile Milli-Q, 4 µl of the appended 5x Buffer, 2 µl of 2.5 mM dNTP, 2 µl of 10 µM forward primer (5'- CACGCGTCGACGCCGCCATGGCCCAGGTGCAACTG -3' (SEQ ID NO: 30) having a *Sal*I site (underlined)), 2 µl of 10 µM reverse primer (5'-GCGGCCAAGCTTAGAGGAGACTGTGAGAGTGGTGC -3' (SEQ ID NO: 31) having a *Hin*dIII site (underlined)), 2 µl of 1F5-derived cDNA as a template, and 0.2 µl of 5 U/µl Expand High Fidelity^{PLUS} PCR system were combined together on ice. PCR was carried out under the following conditions: heat treatment at 95°C for ten minutes, followed by 30 cycles of 95°C for 30 seconds, 60°C for 30 seconds, and 72°C for 60 seconds. The reaction mixture was subjected to electrophoresis using 1% agarose STANDARD 01 gel and a band of about 0.36 kbp was collected using RECOCHIP. This DNA fragment was purified by phenol/chloroform extraction and isopropyl alcohol precipitation. The DNA fragment was treated with *Sal*I (TOYOBO) and *Hin*dIII (New England Biolabs) at final concentrations of 0.5 U/µl and 1.0 U/µl, respectively. The fragment was ligated with 50 ng of pBluescriptII treated in the same way. The ligation was carried out using 1.5 U of T4 DNA ligase at room temperature for 30 minutes. 100 µl of competent cells *of E. coli* DH5α was added to the reaction mixture. After reacting for 30 minutes on ice, the cells were heat-shocked at 42°C for 45 seconds. This was then rested for two minutes on ice, the whole mixture was plated onto LB medium plate containing 100 µg/ml of ampicillin. The plate was incubated at 37°C overnight. From the formed colonies, those into which a DNA for the VH region gene has been inserted were selected, and the vector was named pBluescriptII/VH.

### 2-2. Preparation of pBluescriptII/LVH vector (Fig. 2-1-B)

A leader sequence was added to the VH gene by the following procedure. 4 µl of the appended 5x Buffer, 2 µl of 2.5 mM dNTP, 2 µl of 10 µM forward primer (5'-CACGCGTCGAC GCCGCCATGGGATGGAGCTGTATCATCTTCTTTTT GGTAGCAACAGCTACAGGTGTCCACTCCCAGGTGCAACTGCGGCAGCCTGGG-3' (SEQ ID NO: 32) having a *Sal*I site (underlined)), 2 µl of 10 µM reverse primer (5'-GCGGCCAAGCTTAGAGGAGACTGTGAGAGTGGTGC-3' (SEQ ID NO: 33) having a *Hin*dIII site (underlined)), 100 ng of pBluescriptII/VH as a template, and 0.2 µl of 5 U/µl Expand High Fidelity^{PLUS} PCR system were combined together on ice. The total volume was adjusted to 20 µl by adding sterile MilliQ. PCR was carried out under the following conditions: heat treatment at 95°C for ten minutes, followed by 12 cycles of 95°C for 30 seconds, 60°C for 30 seconds, and 72°C for 60 seconds. The reaction mixture was subjected to electrophoresis using 1% agarose STANDARD 01 gel and a band of about 0.43 kbp was recollected using RECOCHIP. This DNA fragment was purified by phenol/chloroform extraction and isopropyl alcohol precipitation. The DNA fragment was treated with *Sal*I and *Hin*dIII at final concentrations of 0.5 U/µl and 1.0 U/µl, respectively, and ligated with 50 ng of pBluescriptII treated in the same way. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. After resting for two minutes on ice, and the whole mixture was plated onto an LB medium plate containing 100 µg/ml of ampicillin. The plate was incubated at 37°C overnight. From the formed colonies, those into which a DNA having the VH region gene with an added leader sequence has been inserted were selected, and the vector was named pBluescriptII/LVH.

### 2-3. Preparation of pBluescriptII/CH1-CH2-CH3-T vector (Fig. 2-1-C)

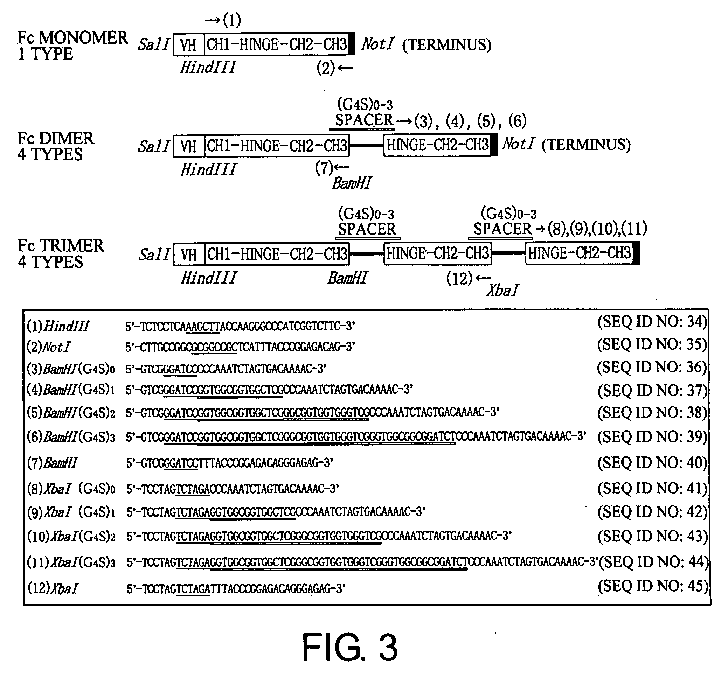

The gene for human IgG1 C region, namely, CH1 domain to CH3 domain up to the stop codon (-T), was cloned by the following procedure. Total RNA was extracted from tonsillar cells from a healthy human using ISOGEN. 10 pmol of oligo dT primer was added to 5 µg of the total RNA. The total volume was adjusted to 12 µl by adding DEPC-treated water. After two minutes of incubation at 72°C, the RNA was quickly transferred onto ice and incubated for three minutes. 2 µl of the appended 10x Reaction Buffer, 1 µl of 100 mM DTT, 1 µl of 20 mM dNTP, and 1 µl of 20 U/µl RNase inhibitor were added, and the total volume was adjusted to 19 µl with DEPC-treated water. After heating up to 42°C, 1 µl of 200 U/µl ReverscriptII was added and the resulting mixture was incubated at 42°C for 50 minutes without further treatment. After reaction, 30 µl of TE was added. The resulting 50-µl mixture was used as a cDNA solution. 4 µl of the appended 5x Buffer, 2 µl of 2.5 mM dNTP, 2 µl of 10 µM forward primer (Fig. 3-(1)), 2 µl of 10 µM reverse primer (Fig. 3-(2)), 2 µl of human tonsillar cell-derived cDNA as a template, and 0.2 µl of 5 U/µl Expand High Fidelity^{PLUS} PCR system were combined together on ice. After the total volume was adjusted to 20 µl by adding sterile MilliQ, PCR was carried out under the following conditions: heat treatment at 95°C for ten minutes, followed by 30 cycles of 95°C for 30 seconds, 55°C for 30 seconds, and 72°C for 60 seconds. The reaction mixture was subjected to electrophoresis using 1% agarose STANDARD 01 gel and a band of about 0.99 kbp was collected using RECOCHIP. This DNA fragment was purified by phenol/chloroform extraction and isopropyl alcohol precipitation. The DNA fragment was treated with *Hin*dIII and *Not*I (New England Biolabs) at final concentrations of 1.0 U/µl and 0.5 U/µl, respectively, and ligated with 50 ng of pBluescriptII treated in the same way. The ligation was carried out using 1.5 U of T4 DNA ligase at room temperature for 30 minutes. 100 µl of competent cells *of E. coli* DH5α was added to the reaction mixture. After reacting for 30 minutes on ice, the cells were heat-shocked at 42°C for 45 seconds. This was then rested for two minutes on ice, and the whole mixture was plated onto LB an medium plate containing 100 µg/ml of ampicillin. The plate was incubated at 37°C overnight. From the formed colonies, those into which the human IgG1 C region gene has been inserted were selected, and the vector was named pBluescriptII/CH1-CH2-CH3-T.

### 2-4. Preparation of pBluescriptII/CH1-CH2-CH3 vector (Fig. 2-1-D), pBluescriptII/SP-CH2-CH3-T vector (Fig. 2-1-E and 2-1-F), and pBluescriptII/SP-CH2-CH3 vector (Fig. 2-1-G)

The gene covering CH1 domain to CH3 domain without the stop codon, the gene covering CH2 domain to CH3 domain containing the hinge and stop codon, and the gene covering CH2 domain to CH3 domain containing the hinge but not stop codon, all of which were derived from the C region of human IgG1, were cloned by the following procedure. 4 µl of the appended 5x Buffer, 2 µl of 2.5 mM dNTP, 2 µl each of 10 µM forward and reverse primers, 0.1 µg of pBluescriptII/CH1-CH2-CH3-T as a template, and 0.2 µl of 5 U/µl Expand High Fidelity^{PLUS} PCR system were combined together on ice. The total volume was adjusted to 20 µl by adding sterile MilliQ. The thirteen pairs of primers used were: primers shown in Fig. 3-(1) and (7) to amplify CH1-CH2-CH3; those shown in Fig. 3-(3) to (6) and (2), or (8) to (11) and (2), to amplify SP-CH2-CH3-T; and those shown in Fig. (3) to (6) and (12) to amplify SP-CH2-CH3. Spacers used in this study were flexible glycine/serine spacers of 0, 5, 10, or 15 amino acid residues (a.a.), in which the basic unit consists of four glycines and one serine, five amino acids in total. However, the type of spacer is not particularly limited. Any conventional peptide spacers (SPs) may be used. Such conventional SPs include, for example, A(EAAAK)nA (SEQ ID NO: 63; the sequence in the parenthesis is a repeating sequence and n represents the repetition number; Arai R et al., Protein Engineering 14, 529-532 (2001)). PCR was carried out under the following conditions: heat treatment at 95°C for ten minutes, followed by 12 cycles of 95°C for 30 seconds, 60°C for 30 seconds, and 72°C for 60 seconds. After the reaction mixture was subjected to electrophoresis using 1% agarose STANDARD 01 gel, the DNA fragments of about 0.99 and 0.74 kbp, covering CH1 domain to CH3 domain and CH2 domain to CH3 domain containing the peptide spacer sequence, respectively, were collected from the bands using RECOCHIP. These DNA fragments were purified by phenol/chloroform extraction and isopropyl alcohol precipitation. The DNA fragments were treated with corresponding restriction enzymes (final concentrations: 1.0 U/µl *Hin*dIII, 0.75 U/µl *Bam*HI (TaKaRa Bio Inc.), 0.75 U/µl *Xba*I (TaKaRa Bio Inc.), and 0.5 U/µl *Not*I), and ligated with 50 ng of pBluescriptII treated in the same way. The ligation was carried out using 1.5 U of T4 DNA ligase at room temperature for 30 minutes. 100 µl of competent cells of E. *coli* DH5α was added to the reaction mixture. After reacting for 30 minutes on ice, the cells were heat-shocked at 42°C for 45 seconds. This was then rested for two minutes on ice, and the whole mixture was plated onto an LB medium plate containing 100 µg/ml of ampicillin. The plate was incubated at 37°C overnight. From the formed colonies, those into which a C region gene of interest has been inserted were selected, and the vectors were named pBluescriptII/CH1-CH2-CH3, pBluescriptII/SP-CH2-CH3-T, and pBluescriptII/SP-CH2-CH3.

### 2-5. Preparation of pBluescriptII/Anti-CD20 HC Fc Monomer vector (Fig. 2-1-H)

The mouse/human chimeric anti-CD20 Fc H chain monomer gene (the DNA sequence is shown in SEQ ID NO: 3, and the amino acid sequence is shown in SEQ ID NO: 4) was constructed by the following procedure. 0.5 µg ofpBluescriptII/LVH (Fig. 2-1-B) was treated with *Sal*I and *Hin*dIII at final concentrations of 0.5 and 1.0 U/µl, respectively. After the whole reaction mixture was subjected to electrophoresis using 1% agarose STANDARD 01 gel, the insert DNA fragment of about 0.43 kbp was collected using RECOCHIP with considerable care not to contaminate it with vector fragments. Separately, pBluescriptII/CH1-CH2-CH3-T vector (Fig. 2-1-C) was treated with *Sal*I and *Hin*dIII under the same conditions. Both DNA fragments were purified by phenol/chloroform extraction and isopropyl alcohol precipitation. 50 ng of pBluescriptII/CH1-CH2-CH3-T treated with the restriction enzymes was mixed and ligated with the excised LVH gene. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, the whole mixture was plated onto an LB medium plate containing 100 µg/ml of ampicillin. The plate was incubated at 37°C overnight. From the formed colonies, those into which a DNA for the VH region gene with an added leader sequence and the human IgG1 H chain CH1-CH2-CH3-T region gene has been inserted were selected, and the vector was named pBluescriptII/Anti-CD20 HC Fc Monomer.

### 2-6. Preparation of pBluescriptII/LVH-CH1-CH2-CH3 vector (Fig. 2-1-I)

0.5 µg of pBluescriptII/LVH (Fig. 2-1-B) was treated with *Sal*I and *Hin*dIII at final concentrations of 0.5 U/µl and 1.0 U/µl, respectively. After the whole reaction mixture was subjected to electrophoresis using 1% agarose STANDARD 01 gel, the insert DNA fragment of about 0.43 kbp was collected using RECOCHIP with considerable care not to contaminate it with vector fragments. Separately, pBluescriptII/CH1-CH2-CH3 (Fig. 2-1-D) vector was treated with *Sal*I and *Hin*dIII under the same conditions. Both DNA fragments were purified by phenol/chloroform extraction and isopropyl alcohol precipitation. 50 ng of pBluescriptII/CH1-CH2-CH3 treated with the restriction enzymes was mixed and ligated with the excised LVH gene. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, the whole mixture was plated onto LB medium plate containing 100 µg/ml of ampicillin. The plate was incubated at 37°C overnight. From the formed colonies, those into which a DNA for the VH region gene with an added leader sequence and the human IgG1 H chain CH1-CH2-CH3 region gene has been inserted were selected, and the vector was named pBluescriptII/LVH-CH1-CH2-CH3.

### 2-7. Preparation of pBluescriptII/Anti-CD20 HC Fc Dimer vector (Fig. 2-2-J)

0.5 µg of pBluescriptII/LVH-CH1-CH2-CH3 (Fig. 2-1-I) was treated with *Sal*I and *Bam*HI at final concentrations of 0.5 U/µl and 0.75 U/µl, respectively. After the whole reaction mixture was subjected to electrophoresis using 1% agarose STANDARD 01 gel, the insert DNA fragment of about 1.42 kbp was collected using RECOCHIP with considerable care not to contaminate it with vector fragments. Separately, four types of pBluescriptII/SP-CH2-CH3-T vectors which are different in the length of glycine/serine spacer (Fig. 2-1-E) were treated with *Sal*I and *Bam*HI under the same conditions. These DNA fragments were purified by phenol/chloroform extraction and isopropyl alcohol precipitation. 50 ng of pBluescriptII/SP-CH2-CH3-T treated with the restriction enzymes was combined and ligated with the excised LVH-CH1-CH2-CH3 gene. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, and the whole mixture was plated onto an LB medium plate containing 100 µg/ml of ampicillin. The plate was incubated at 37°C overnight. A vector carrying an insert DNA having all of the VL region gene added with the leader sequence, and genes for human IgG1 H chain CH1-CH2-CH3 region and CH2-CH3-T region containing the peptide spacer was selected from the formed colonies. The resulting vector was named pBluescriptII/Anti-CD20 HC Fc Dimer. DNA sequences of the human IgG1 H chain CH1-CH2-CH3 region linked with the CH2-CH3-T region are shown in SEQ ID NOs: 5 (0 spacer), 7 (one spacer), 9 (two spacers), and 11 (three spacers). Furthermore, the corresponding amino acid sequences are shown in SEQ ID NOs: 6, 8, 10, and 12.

### 2-8. Preparation of pBluescriptII/SP-CH2-CH3-SP-CH2-CH3-T vector (Fig. 2-1-K)

0.5 µg each of four types of pBluescriptII/SP-CH2-CH3-T vectors which are different in the length of glycine/serine spacer (Fig. 2-1-F) were treated with *Xba*I and *Not*I at final concentrations of 0.75 U/µl and 0.5 U/µl, respectively. After the whole reaction mixture was subjected to electrophoresis using 1% agarose STANDARD 01 gel, the insert DNA fragment of about 0.74 kbp was collected using RECOCHIP with considerable care not to contaminate it with vector fragments. Separately, four types of pBluescriptII/SP-CH2-CH3 vectors which are different in the length of glycine/serine spacer (Fig. 2-1-G) were treated with *Xba*I and *Not*I under the same conditions. These DNA fragments were purified by phenol/chloroform extraction and isopropyl alcohol precipitation. 50 ng of pBluescriptII/SP-CH2-CH3 treated with the restriction enzymes was mixed and ligated with the excised SP-CH2-CH3 gene having a peptide spacer of the same length. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, and the whole mixture was plated onto an LB medium plate containing 100 µg/ml of ampicillin. The plate was incubated at 37°C overnight. From the formed colonies, those into which two units of CH2-CH3 gene containing the peptide spacer have been inserted were selected, and the vector was named pBluescriptII/SP-CH2-CH3-SP-CH2-CH3-T.

### 2-9. Preparation of pBluescriptII/Anti-CD20 H Chain Fc Trimer vector (Fig. 2-2-L)

0.5 µg of pBluescriptII/LVH-CH1-CH2-CH3 (Fig. 2-1-I) was treated with *Sal*I and *Bam*HI at final concentrations of 0.5 U/µl and 0.75 U/µl, respectively. After the whole reaction mixture was subjected to electrophoresis using 1% agarose STANDARD 01 gel, the insert DNA fragment of about 1.42 kbp was collected using RECOCHIP with considerable care not to contaminate it with vector fragments. Separately, four types of pBluescriptII/SP-CH2-CH3-SP-CH2-CH3-T vectors which are different in the length of glycine/serine spacer (Fig. 2-1-K) were treated with *Sal*I and *Bam*HI under the same conditions. These DNA fragments were purified by phenol/chloroform extraction and isopropyl alcohol precipitation. 50 ng of pBluescriptII/SP-CH2-CH3-SP-CH2-CH3-T treated with the restriction enzymes was mixed and ligated with the excised LVH-CH1-CH2-CH3 gene. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. This was then rested for two minutes on ice, and the whole mixture was plated onto an LB medium plate containing 100 µg/ml of ampicillin. The plate was incubated at 37°C overnight. A vector carrying an insert DNA having the VH region gene added with the leader sequence, human IgG1 H chain CH1-CH2-CH3 region gene, and two units of CH2-CH3 region gene containing the peptide spacer was selected from the formed colonies. The resulting vector was named pBluescriptII/Anti-CD20 HC Fc Trimer. DNA sequences of the human IgG1 H chain CH1-CH2-CH3 region linked with two units of CH2-CH3-T regions are shown in SEQ ID NOs: 13 (0 spacer), 15 (one spacer), 17 (two spacers), and 19 (three spacers). Furthermore, the corresponding amino acid sequences are shown in SEQ ID NOs: 14, 16, 18, and 20.

### 2-10. Preparation of pcDNA3.1/Anti-CD20 HC vector (Fig. 2-2-M, 2-2-N, and 2-2-O)

The mouse/human chimeric anti-CD20 H chain gene was transferred from pBluescriptII vector to pcDNA3.1/Zeo vector by the following procedure. 0.5 µg each of pBluescriptII/Anti-CD20 HC Fc Monomer (Fig. 2-1-H), pBluescriptII/Anti-CD20 HC Fc Dimer (Fig. 2-2-J), and pBluescriptII/Anti-CD20 HC Fc Trimer (Fig. 2-2-L) were treated with *Sal*I and *Not*I, both at a final concentration of 0.5 U/µl. After the whole reaction mixture was subjected to electrophoresis using 1% agarose STANDARD 01 gel, the insert DNA fragments of about 1.42 kbp, 2.16 kbp, and 2.90 kbp, covering Anti-CD20 HC Fc Monomer gene, Anti-CD20 HC Fc Dimer gene, and Anti-CD20 HC Fc Trimer gene, respectively, were collected using RECOCHIP. Separately, pcDNA3.1/Zeo vector was treated with *Xho*I and *Not*I, both at a final concentration of 0.5 U/µl. These DNA fragments were purified by phenol/chloroform extraction and isopropyl alcohol precipitation. 50 ng of pcDNA3.1/Zeo treated with the restriction enzymes was mixed and ligated with the excised Anti-CD20 HC genes. The ligation was carried out using 1.5 U of T4 DNA ligase at room temperature for 30 minutes. 100 µl of competent cells of *E*. *coli* DH5α was added to the reaction mixtures. After reacting for 30 minutes on ice, the cells were heat-shocked at 42°C for 45 seconds. This was then rested for two minutes on ice, and the whole mixtures were plated onto an LB medium plates containing 100 µg/ml of ampicillin. The plates were incubated at 37°C overnight. From the formed colonies, those into which the anti-CD20 HC gene has been inserted were selected, and the vectors were named pcDNA3.1/Zeo/Anti-CD20 HC Fc Monomer, pcDNA3.1/Zeo/Anti-CD20 HC Fc Dimer, and pcDNA3.1/Zeo/Anti-CD20 HC Fc Trimer.

### 2-11. Preparation of pCAGGS1-dhfrN-L vector (Fig. 2-2-P)

A spacer was inserted into pCAGGS1-dhfrN, an expression vector carrying the CAG promoter and dihydrofolate reductase (dhfr) gene, by the following procedure. pCAGGS1-dhfrN was treated with *Sal*I at a final concentration of 0.5 U/ml. This DNA fragment was purified by phenol/chloroform extraction and isopropyl alcohol precipitation. Separately, two DNA strands (sense DNA: GTCGACGCTAGCAAGGATCCTTGAA TTCCTTAAGG (SEQ ID NO: 46); antisense DNA: GTCGACCTTAAGGAATTCAAGGATCCTTGCTAGCG (SEQ ID NO: 47)) were synthesized, and combined together at a final concentration of 1 µM. The total volume was adjusted to 10 µl with MilliQ water. After five minutes of heating at 75°C, the mixture was rested at room temperature to gradually cool it. 1 µl of this solution was mixed and ligated with 50 ng of *Sal*I-treated pCAGGS1-dhfrN. The ligation was carried out using 1.5 U of T4 DNA ligase at room temperature for 30 minutes. 100 µl of competent cells of *E*. *coli* DH5α was added to the reaction mixture. After reacting for 30 minutes on ice, the cells were heat-shocked at 42°C for 45 seconds. After rested for two minutes on ice, and the whole mixture was plated onto an LB medium plate containing 100 µg/ml of ampicillin. The plate was incubated at 37°C overnight. A pCAGGS1-dhfrN vector containing the spacer as an insert was selected from the formed colonies. The resulting vector was named pCAGGS1-dhfrN-L. As a result of the spacer insertion, two *Sal*I sites were newly generated in pCAGGS1-dhfrN, and the sequence of restriction enzyme sites became 5'-SalI-NheI-BamHI-EcoRI-AflII-SalI-3'.

### 2-12. Preparation of pCAGGS1-dhfrN-L/Anti-CD20 HC vector (Fig. 2-2-Q, 2-2-R, and 2-2-S)

The mouse/human chimeric anti-CD20 H chain gene was transferred from pcDNA3.1/Zeo vector to pCAGGS1-dhfrN-L vector by the following procedure. 0.5 µg each of pcDNA3.1/Zeo/Anti-CD20 HC Fc Monomer, pcDNA3.1/Zeo/Anti-CD20 HC Fc Dimer, and pcDNA3.1/Zeo/Anti-CD20 HC Fc Trimer were treated with *Nhe*I and *Eco*RI at final concentrations of 0.8 U/µl and 0.5 U/µl, respectively. After the whole reaction mixtures were subjected to electrophoresis using 1% agarose STANDARD 01 gel, the insert DNA fragments of about 1.42, 2.16, and 2.90 kbp, covering Anti-CD20 HC Fc Monomer gene, Anti-CD20 HC Fc Dimer gene, and Anti-CD20 HC Fc Trimer gene, respectively, were collected using RECOCHIP. Separately, pCAGGS1-dhfrN-L vector was treated with *Nhe*I and *Eco*RI under the same conditions. These DNA fragments were purified by phenol/chloroform extraction and isopropyl alcohol precipitation. 50 ng of pCAGGS1-dhfrN-L treated with the restriction enzymes was mixed and ligated with the excised Anti-CD20 HC genes. The ligation was carried out using 1.5 U of T4 DNA ligase at room temperature for 30 minutes. 100 µl of competent cells *of E. coli* DH5α was added to the reaction mixtures. After reacting for 30 minutes on ice, the cells were heat-shocked at 42°C for 45 seconds. This was then rested for two minutes on ice, and the whole mixtures were plated onto an LB medium plates containing 100 µg/ml of ampicillin. The plates were incubated at 37°C overnight. From the formed colonies, those into which the anti-CD20 HC gene has been inserted were selected, and the vectors were named pCAGGS1-dhfrN-L/Anti-CD20 HC Fc Monomer (Fig. 2-2-Q), pCAGGS1-dhfrN-L/Anti-CD20 HC Fc Dimer (Fig. 2-2-R), and pCAGGS1-dhfrN-L/Anti-CD20 HC Fc Trimer (Fig. 2-2-S).

### [Example 3] Selection of cell clones expressing an modified antibody from G418- and MTX-resistant cells

### 3-1. Production of transformants

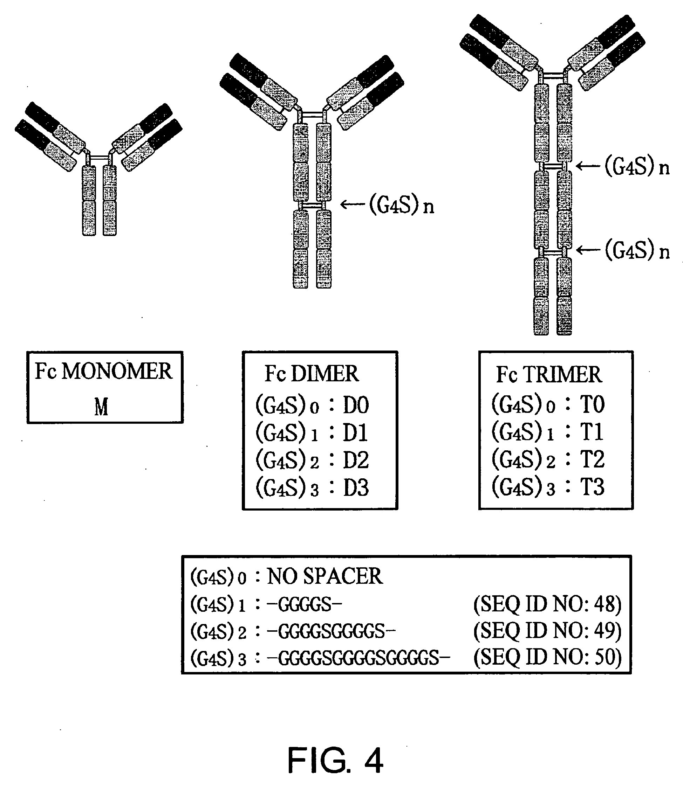

The plasmid pCAGGS1-neoN-L/Anti-CD20 LC prepared as described in Example 1 and the plasmid pCAGGS1-dhfrN-L/Anti-CD20 HC prepared as described in Example 2 were linearized using *Pvu*I (TOYOBO) at a final concentration of 1.0 U/µl. Cells of CHO DG44 line were plated at 3 x 10⁵ cells/well in 6-well multiplate (FALCON353046) using IMDM (Sigma Aldrich) supplemented with 10% fetal bovine serum, 0.1 mM hypoxanthine (Wako Pure Chemical Industries, Ltd.), 0.016 mM thymidine (Wako Pure Chemical Industries, Ltd.), 100 U/ml penicillin, and 100 µg/ml streptomycin. The cells were cultured at 37°C under 5% CO₂ for 24 hours. Nine batches of cells of CHO DG44 line were transfected with 1.35 µg of L chain expression vector and 1.35 µg each of the nine types of H chain expression vectors using Trans Fast Transfection Reagent (Promega). The cells were cultured at 37°C under 5% CO₂ for 48 hours. The nine types of antibodies produced as a result of introduction of these plasmids and altered forms of the antibodies are schematically illustrated in Fig. 4, where M, D, and T are schematic diagrams for Fc monomer, dimer, and trimer, respectively. D and T also include altered forms that were produced so as to have various numbers of spacers. D and T having zero, one (SEQ ID NO: 48), two (SEQ ID NO: 49), or three (SEQ ID NO: 50) unit(s) of the spacer were produced, where the sequence of glycine-glycine-glycine-glycine-serine (GGGGS) was defined as the unit spacer. The respective products were named D0, D1, D2, D3, T0, T1, T2, and T3 (hereinafter the abbreviations are used for the nine types of antibodies and altered forms thereof). After the culture supernatants of the transformed cells were discarded, the cells were washed with PBS and then 1 ml of Trypsin-EDTA Solution (Sigma Aldrich) was added thereto. The cells were incubated at 37°C for three minutes. After confirming cell detachment from the plate and spherical shape, cells were suspended in IMDM selection medium supplemented with 10% fetal bovine serum, 0.8 mg/ml G418, 500 nM methotrexate, 100 U/ml penicillin, and 100 µg/ml streptomycin. Cells of each transformant were plated and cultured in two 96-well flat-bottomed multiplates (FALCON353072) at 3 x 10³ cells/well (medium volume: 100 µl/well).

### 3-2. Anti-CD20 and determination of concentrations

The concentrations of antibody in culture supernatants were determined by enzyme immunoassay (ELISA). A goat anti-human γ chain antibody (Biosource) was diluted to 0.5 µg/ml with PBS. 50 µl of the immobilized antibody was aliquoted onto a 96-well plate (FALCON353912) and incubated at 4°C overnight. The antibody solution was discarded, and the plate was blocked by adding 150 µl of PBS solution containing 0.1 % BSA (Wako Pure Chemical Industries, Ltd.) and incubating at 37°C for two hours. The blocking solution was discarded and the cell culture supernatants ten-times diluted with PBS were aliquoted (100 µl) into corresponding wells. The plate was then incubated at 37°C for two hours. After the diluted culture media were discarded, the wells were washed three times with 100 µl of PBS solution containing 0.05% Tween20 (MP Biomedicals) (PBST). A peroxidase-labeled goat anti-human γ chain antibody (Sigma Aldrich) was diluted to 0.5 µg/ml with PBS containing 0.1% BSA, and then aliquoted (50 µl) into each well. The plate was incubated at 37°C for one hour. After the solution was discarded and the wells were washed three times with PBST, 50 µl of substrate solution (sodium citrate buffer (pH 5.0) containing 0.4% o-phenylene diamine dihydrochloride (Sigma Aldrich) and 0.003% H₂O₂ (Wako Pure Chemical Industries, Ltd.)) was aliquoted and A450 was measured with a microplate reader (BIO-RAD Model 550).

### 3-3. Cell cloning

Four days after the transformants were plated onto 96-well plates, 100 µl of a selection medium was added thereto. One week after addition of the medium, concentrations of antibody in culture supernatants were determined by the method described in Example 3-2. Of 192 wells, twelve wells exhibiting high A450 values were selected for each transformant. These cells were trypsinized, and then combined together using the selection medium. Again, the cells were plated onto one 96-well multiplate at 3 cells/well and two 96-well multiplates at 1 cell/well, in total three 96-well plates. The cells were cultured at 37°C under 5% CO₂ (medium volume: 100 µl/well). Sixteen days after plating, wells with a single colony were selected and concentrations of antibody in the culture supernatants were determined. Twelve wells exhibiting high A450 value were selected and treated with trypsin. Then, the cells were plated onto a 24-well multiplate using the selection medium (medium volume: 1 ml/well). Again, concentrations of antibody in the culture supernatants were determined when the cells were grown to confluency. Six wells exhibiting high A450 value were selected and treated with trypsin. Then, the cells were plated onto a 6-well multiplate (medium volume: 4 ml/well). Again, concentrations of antibody in the culture supernatants were determined when the cells were grown to confluency. Three wells exhibiting high A450 value were selected and treated with trypsin. Then, the cells were separately plated onto 10-cm dishes (FALCON353003) (medium volume: 10 ml). Of these, one clone was conditioned to serum-free medium, and the other two clones were frozen and stored.

### 3-4. Conditioning to serum-free medium

The cells grown to confluency in 10-cm dishes were trypsinized, and then 2 x 10⁶ cells were suspended in 10 ml of a mixed medium consisting of 2.5 ml of CD CHO Medium (GIBCO) and 7.5 ml of IMDM supplemented with 10% fetal bovine serum, 100 U/ml penicillin, and 100 µg/ml streptomycin. The cells were plated onto 10-cm dishes (mixing ratio: 25%:75%). The cells were assumed to be conditioned to the mixed medium when they grew to confluency. Subsequently, the cells were passaged while varying the mixing ratio between CD CHO Medium and IMDM supplemented with 10% fetal bovine serum to 50%:50%, 75%:25%, and 90%:10% in succession.

### [Example 4] Large scale culture of antibody-producing cells and purification of expressed antibodies

### 4-1. Large scale culture of antibody-producing cells

The antibody-expressing cells prepared as described in Example 3 were plated onto a total of seven 15-cm dishes at 10⁶ cells/dish (medium volume: 20 ml) using a mixed medium where the mixing ratio between CD CHO medium and IMDM supplemented with 10% fetal bovine serum was 90%:10%. When the cells were grown to confluency, the medium was discarded and the cells were washed three times with 20 ml of PBS. Then, 30 ml of CD CHO medium was added and the cells were cultured at 37°C at 8% CO₂ for ten days.

### 4-2. Purification of antibodies from culture supernatants using Protein A agarose

The culture supernatants of antibody-expressing cells were collected into four 50-ml conical tubes, and then centrifuged at 3,000 g for 30 minutes. The supernatants were collected into an Erlenmeyer flask with care not to contaminate them with the pellets. A column filled with 1 ml of Protein A agarose (Santa Cruz) was equilibrated with 5 ml of PBS. The whole culture supernatant was then loaded onto the column. The column was washed with 5 ml of PBS to remove non-specifically adsorbed materials, and then eluted with 3 ml of 0.1 M glycine (Wako Pure Chemical Industries, Ltd.)/hydrochloric acid elution solution (pH 2.7). 300-µl fractions were collected. Immediately, 30 µl of neutralizing solution (pH 9.0) consisting of Tris (Wako Pure Chemical Industries, Ltd.) and hydrochloric acid was added to the collected fractions and the combined solutions were mixed by inversion. After sodium dodecyl sulfate-polyacrylamide gel electrophoresis (SDS-PAGE) and CBB staining (BIO-RAD; BIO-Safe Coomassie), quantitation was performed using Image J (National Institutes of Health, USA), an analytical program for electrophoresis. The result showed that the yields of the nine types of antibodies were 2 to 4 µg/ml medium.

### 4-3. Secondary purification step using gel filtration chromatography

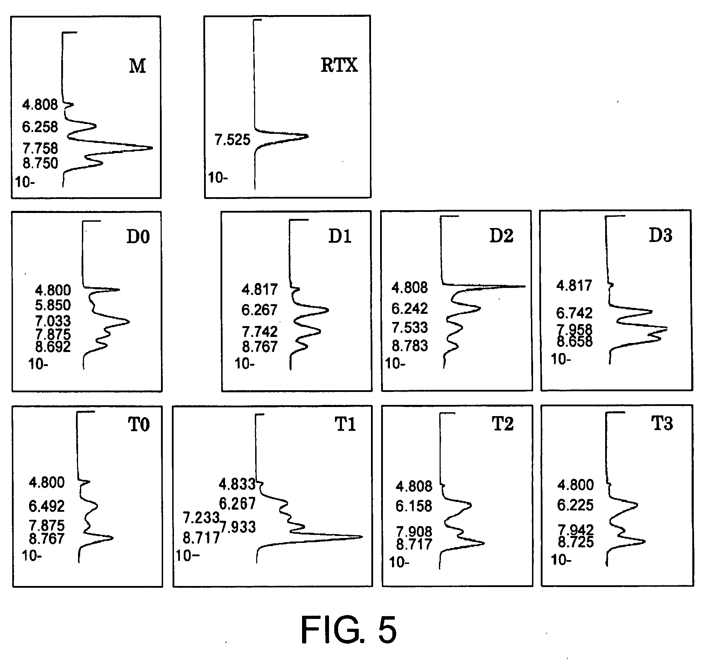

The antibodies underwent secondary purification using an HPLC system (JASCO CO.) with a column of Protein Pak 300SW (Waters). The flow rate of the elution solution (0.1 M phosphate buffer (pH 7.0) containing 0.15 M sodium chloride) was 1 ml/min. A Rituxan (RTX; molecular weight, 145 kDa; Chugai Pharmaceutical Co.) qualitative test showed a retention time of 7.5 minutes. Based on this result, 100 µl each of the nine types of antibodies (100 µg/ml) was injected in quadruplicates, and peak fractions were collected: M (154 kDa) at a retention time of 7.8 min, D0 (208 kDa) at 7.0 min, D1 (154 kDa) at 7.7 min, D2 (154 kDa) at 7.5 min, D3 (208 kDa) at 6.7 min, T0 (262 kDa) at 6.5 min, T1 (262 kDa) at 6.3 min, T2 (262 kDa) at 6.2 min, and T3 (262 kDa) at 6.2 min. The volume of each collected antibody was about 4 ml. The chromatograms are shown in Fig. 5.

### 4-4. Concentration using ultrafiltration

1 ml of 5% Tween20 was poured into filter units of Amicon Ultra-4 (Millipore) whose molecular cut-off is 50k Da. The units were allowed to stand at room temperature for one hour. The aqueous solution of Tween20 was discarded and the filter units were washed three times with 1 ml of MilliQ water. 4 ml each of the solutions collected in the secondary purification step using gel filtration were loaded onto the filter units. The units were centrifuged at 3,000 g and 25°C for 25 minutes. The solutions that were concentrated to about 100 µl were collected. The concentrations were determined by quantitative HPLC using RTX as standard substance.

### [Example 5] Structural analysis of antibodies

### 5-1. SDS-PAGE analysis

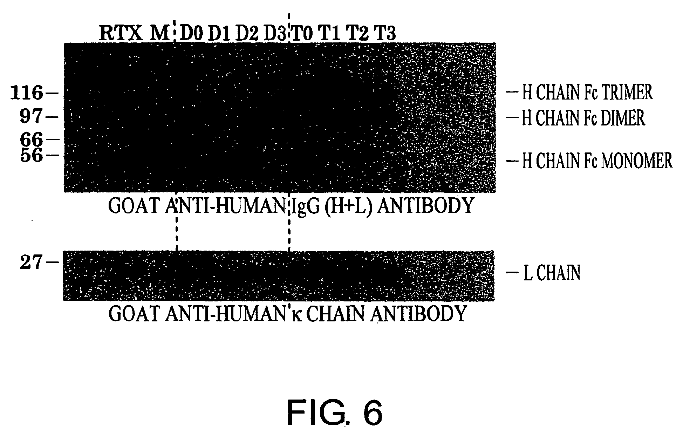

10% polyacrylamide gel was prepared with the following composition. The separating gel was prepared by mixing 1.9 ml of MilliQ water, 1.7 ml of 30% acrylamide (29% acrylamide (Wako Pure Chemical Industries, Ltd.), 1% N,N'-methylene bis-acrylamide (Wako Pure Chemical Industries, Ltd.)), 1.3 ml of 0.5 M Tris-HCl buffer (pH 6.8), 50 µl of 10% SDS (Wako Pure Chemical Industries, Ltd.), 50 µl of APS (Wako Pure Chemical Industries, Ltd.), and 3 µl of TEMED (Wako Pure Chemical Industries, Ltd.). This was immediately poured into a gel plate taking care not to introduce air bubbles. 0.5 ml of MilliQ water was laid on top, and this was allowed to stand at room temperature for 30 minutes. After polymerization, the laid Milli-Q water was discarded. The concentrating gel was prepared by mixing 1.4 ml of MilliQ water, 0.25 ml of 30% acrylamide, 0.33 ml of 1.5 M Tris-HCl buffer (pH 8.8), 20 µl of 10% SDS, 20 µl of APS, and 2 µl of TEMED, and this was poured into the gel plate. A comb was placed and allowed to stand at room temperature for 30 minutes to let the gel polymerize. 300 ng of each antibody was adjusted to 10 µl with the elution solution used for the HPLC, and then adjusted to 20 µl by adding Laemmli Sample buffer (BIO-RAD) containing 10% 2-mercaptoethanol (Wako Pure Chemical Industries, Ltd.) thereto. After vortexing, the samples were heat-treated at 95°C for five minutes, and applied into the wells of the 10% acrylamide gel. SDS-PAGE was carried out at a constant current of 0.02 A according to the method of Laemmli. PVDF membrane (Pall Corporation) was soaked thoroughly in methanol, and then Transfer buffer (0.78% Tris, 3.6% glycine) was added thereto so that the methanol content was 20%. The gel after electrophoresis was placed on top and shaken at room temperature for 15 minutes. Two sheets of filter paper soaked with the Transfer buffer were placed onto the Trans-Blot SD SEMI-DRY TRANSFER CELL (BIO-RAD), and the PVDF membrane and gel were laid thereon in this order. Another two sheets of filter paper soaked with the Transfer buffer were placed, and Western blotting was carried out at a constant current of 0.2 A for 30 minutes. After blotting, the PVDF membrane was immersed in a PBST solution containing 5% skimmed milk (Snow Brand) and blocked at 4°C overnight. The PVDF membrane was sandwiched in between vinyl sheets and immersed in 1 ml of blocking solution containing 0.13 µg/ml HRP-labeled goat anti-human IgG(H+L) (Chemicon) and 0.33 µg/ml HRP-labeled goat anti-human Kappa chain (Sigma Aldrich) with shaking at room temperature for two hours. The PVDF membrane was removed from the vinyl sheets, and shaken in PBST for five minutes. This washing treatment of the PVDF membrane was repeated three times. The PVDF membrane was sandwiched in between vinyl sheets and 1 ml of "ECL Western blotting detection reagents and analysis system" (Amersham Biosciences) was added thereto. An X-ray film (Kodak) was exposed for one minute in a dark room. The film was immersed in RENDOL solution (Fujifilm) until bands could be confirmed, and then rinsed with tap water and fixed with RENFIX solution (Fujifilm). The result is shown in Fig. 6.

### 5-2. HPLC analysis

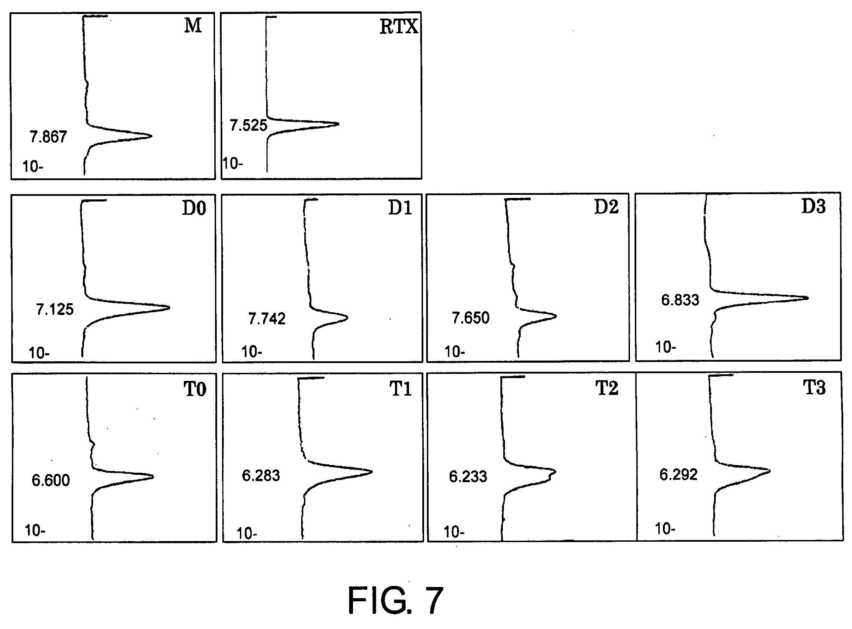

Antibody molecules were analyzed by HPLC using Protein Pak 300SW. An elution solution (0.1 M phosphate buffer (pH 7.0) containing 0.15 M sodium chloride) was used at a rate of 1 ml/min. Chromatograms were obtained after injecting about 600 ng of each antibody (Fig. 7). The results of SDS-PAGE and HPLC analyses demonstrated that purified products of D0 and D3 had two of the hinge portion and Fc domain linked in tandem, while the major components of D1 and D2 were molecules with only one of these. Meanwhile, T0 contained three of the hinge portion and Fc domain in tandem, while T1, T2, and T3 were mixtures of molecules having three of these in tandem and molecules only having two of these.

### [Example 6] CD20-binding assay of antibodies using flow cytometry

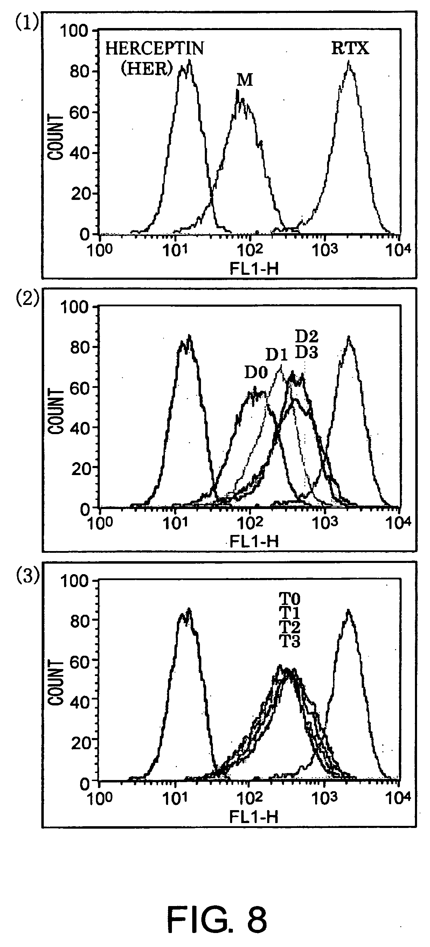

CD20-positive human Burkitt's lymphoma Ramos cells were cultured using RPMI1640 containing 10% heat-inactivated fetal bovine serum, 1 mM sodium pyruvate (Wako), 100 U/ml penicillin, and 100 µg/ml streptomycin at 37°C under 5% CO₂. The Ramos cell culture solution was centrifuged at 600 g for five minutes. After the medium was removed, the cells were suspended in an appropriate volume of medium. After another centrifugation at 600 g for five minutes, the medium was removed. 5 ml of FACS buffer (PBS containing 0.1% BSA and 0.02% NaN₃) was added to suspend the cells, and this was left on ice and blocked for 30 minutes. The supernatant was removed after centrifugation at 600 g for five minutes, cells were suspended at 5 x 10⁶ cells/ml in FACS buffer, and 100 µl were aliquoted per 1.5-ml tube. The prepared anti-CD20 antibody and RTX were added at a final concentration of 50 nM. Moreover, Herceptin (HER) (trastuzumab; 148 kDa; Chugai Pharmaceutical Co.) was added at a final concentration of 30 nM, this was allowed to stand on ice for 30 minutes to let the antibodies react with cells, and then centrifuged at 600 g for five minutes. The supernatants were removed, and cells were washed by adding 500 µl of FACS buffer. This process was repeated twice to completely remove the non-reacted antibodies. Cells were suspended in 100 µl of FACS buffer containing 20 µg/ml FITC-labeled goat anti-human Kappa chain antibody (Biosource International), and allowed to stand on ice in the dark for 30 minutes. Cells were washed with the method described above, suspended in 100 µl of FACS buffer, filtered through a mesh with a hole size of 59 µm, and transferred into FACS tubes. The CD20 binding activity of each antibody was analyzed by fluorescence measurement using FACScan (Becton Dickinson) (Fig. 8).

All of these antibodies, which comprise the variable region of mouse monoclonal anti-CD20 antibody 1F5 and a human constant region, bound to Ramos cells. The amount of bound T0, T1, T2, T3, D1, D2, and D3 was slightly greater than that of D0. The amount of bound M was slightly lower than that of D0.

### [Example 7] Receptor binding assay by ELISA using recombinant FcγR

### 7-1. Preparation of recombinant FcγR

### (i) Construction of pBluescriptII/Gly-His₆-GST vector

A GST gene sequence was inserted into pBluescriptII by the following procedure: 4 µl of the appended 5x Buffer, 1.6 µl of 2.5 mM dNTP, 1 µl of 10 µM forward primer (5'-ATCTATCTAGAGGCCATCACCATCACCATCACATGTCCCCTATACTAGGTTATTG -3' (SEQ ID NO: 51) having an *Xba*I site (underlined) and a Gly-His₆ sequence (position 12 to 32 in SEQ ID NO: 51)) and 1 µl of 10 µM reverse primer (*5*'-ATTAATCAGCGGCCGCTCACGGGGATCCAACAG AT-3' (SEQ ID NO: 52) having a *Not*I site (underlined)) to amplify the GST sequence, 100 ng of pGEX-2TK as a template, and 0.2 µl of 5 U/µl Expand High Fidelity^{PLUS} PCR system were combined together on ice. The total volume was adjusted to 20 µl by adding MilliQ water. PCR was carried out under the following conditions: heating at 95°C for 2 minutes, followed by 10 cycles of the three steps of 95°C for 30 seconds, 60°C for 30 seconds, and 72°C for 60 seconds. The reaction solution was subjected to electrophoresis using a 1% agarose STANDARD 01 gel. A band of about 0.70 kbp was recollected using RECOCHIP. This DNA fragment was purified by phenol/chloroform extraction and isopropyl alcohol precipitation. The DNA fragment was treated with *Xba*I and *Not*I at final concentrations of 0.75 U/µl and 0.5 *U/µl, respectively, and ligated with 50 ng of similarly-treated pBluescriptII at room temperature for 30 minutes using 1.5 U of T4 DNA ligase. 100 µl *of E. coli* DH5α competent cells was added to the reaction solution, this was left on ice for 30 minutes, and heat-shocked at 42°C for 45 seconds. After two minutes of incubation on ice, the whole amount was plated onto an LB culture plate containing 100 µg/ml ampicillin. The plate was incubated at 37°C overnight. From the formed colonies, those into which the Gly-His₆-GST sequence has been inserted were selected, and this vector was named pBluescriptII/Gly-His₆-GST.

### (ii) Construction of pBluescriptII/FcyR/Gly-His₆-GST vector

The gene sequences for the extracellular domains of four types of FcγR, namely FcγRIA, FcγRIIA, FcγRIIB, and FcγRIIIA, were inserted into pBluescriptII/Gly-His₆-GST by the following procedure: 4 µl of the appended 5x Buffer, 1.6 µl of 2.5 mM dNTP, 1 µl of 10 µM forward primer (FcγRIA: 5'-CCCCAAGCTTGCCGCCATGTGGTTCTTGACAACTC-3' (SEQ ID NO: 53) having a *Hin*dIII site (underlined); FcγRIIA: 5'-AACAAAAGCTTGCCGCCATGGAGACCCAAATGTCT-3' (SEQ ID NO: 54) having a *Hin*dIII site (underlined); FcγRIIB: 5'-CCCCAAGCTTGCCGCCATGGGAATCCTGTCATTCT-3' (SEQ ID NO: 55) having a *Hin*dIII site (underlined); and FcγRIIIA: 5'-ATATGAATTCGCCGCCATGTGGCAGCTGCTC-3' (SEQ ID NO: 56) having an *Eco*RI site (underlined)) and 1 µl of 10 µM reverse primer (FcγRIA: 5'-GCGAATCTAGAATGAAACCAGACAGGAG-3' (SEQ ID NO: 57) having an *Xba*I site (underlined); FcγRIIA: 5'-ACGATTCTAGACA TTGGTGAAGAGCTGCC-3'(SEQ ID NO: 58) having an *Xba*I site (underlined); FcγRIIB: 5'-ACGATTCTAGACATCGGTGAAGAGCTGGG-3' (SEQ ID NO: 59) having an *Xba*I site (underlined); and FcγRIIIA: 5'-CGGCATCTAGATTGGTACCCAGGTGGAAAG-3' (SEQ ID NO: 60) having an *Xba*I site (underlined)) to amplify the extracellular domain sequence of FcγR, 100 ng of a vector into which the full-length FcγR gene has been inserted as a template, and 0.2 µl of 5 U/µl Expand High Fidelity^{PLUS} PCR system were combined together on ice. The total volume was adjusted to 20 µl by adding MilliQ water. PCR was carried out under the following conditions: heating at 95°C for 2 minutes, followed by 10 cycles of the three steps of 95°C for 30 seconds, 60°C for 30 seconds, and 72°C for 60 seconds. The reaction solution was subjected to electrophoresis using a 1% agarose STANDARD 01 gel. Bands of about 0.88 kbp, 0.65 kbp, 0.65 kbp, and 0.73 kbp were recollected for FcγRIA, FcγRIIA, FcγRIIB, and FcγRIIIA using RECOCHIP, respectively. These DNA fragments were purified by phenol/chloroform extraction and isopropanol precipitation. The DNA fragments for FcγRIA, FcγRIIA, and FcγRIIB were treated with *Hin*dIII and *Xba*I at final concentrations of 1.0 U/µl and 0.75 U/µl, respectively, while FcγRIIIA was treated with *Eco*RI and *Xba*I at final concentrations of 0.5 U/µl and 0.75 U/µl, respectively, and these were ligated with 50 ng of similarly-treated pBluescriptII/Gly-His₆-GST at room temperature for 30 minutes using 1.5 U of T4 DNA ligase. 100 µl *of E. coli* DH5α competent cells was added to the reaction solution, this was left on ice for 30 minutes, and heat-shocked at 42°C for 45 seconds. After two minutes of incubation on ice, the whole amount was plated onto an LB culture plate containing 100 µg/ml ampicillin. The plate was incubated at 37°C overnight. From the formed colonies, those into which the gene for the extracellular domain of FcγR has been inserted were selected, and the vector was named pBluescriptII/FcγR/Gly-His₆-GST.

### (iii) Construction of pcDNA3.1/Zeo/FcγR/Gly-His₆-GST vector

The FcγR/Gly-His₆-GST sequence was transferred from pBluescriptII/vector to pcDNA3.1/Zeo vector by the following procedure: 0.5 µg of pBluescriptII/FcγR/Gly-His₆-GST was treated with *Apa*I and *Not*I, both at a final concentration of 0.5 U/ml. After electrophoresis using a 1% agarose STANDARD 01 gel, bands of about 1.58 kbp, 1.35 kbp, 1.35 kbp, and 1.43 kbp were collected for FcγRIA, FcγRIIA, FcγRIIB, and FcγRIIIA using RECOCHIP, respectively. Separately, pcDNA3.1/Zeo vector was treated with *Apa*I and *Not*I, both at a final concentration of 0.5 U/µl. Both of these DNA fragments were purified by phenol/chloroform extraction and isopropyl alcohol precipitation. 50 ng of the restriction enzyme-treated pcDNA3.1/Zeo was combined with the excised FcγR/Gly-His₆-GST gene and ligation was carried out using 1.5 U of T4 DNA ligase at room temperature for 30 minutes. 100 µl of *E*. *coli* DH5α competent cells was added to the reaction solution, this was left on ice for 30 minutes, and heat-shocked at 42°C for 45 seconds. After two minutes of incubation on ice, the whole amount was plated onto LB culture plate containing 100 µg/ml ampicillin. The plate was incubated at 37°C overnight. From the formed colonies, those into which the FcγR/Gly-His₆-GST gene has been inserted were selected, and the vector was named pcDNA3.1/Zeo/FcγR/Gly-His₆-GST.

### (iv) Construction of pcDNA3.1/Zeo/FcyRIIIA(F)/Gly-His₆-GST vector

pcDNA3.1/Zeo/FcyRIIIA/Gly-His₆-GST vector constructed in (iii) was a type-V FcγRIIIA. pcDNA3.1/Zeo/FcγRIIIA(F)/Gly-His₆-GST vector was prepared by site-directed mutagenesis using the following procedure: 2 µl of the appended 10x Buffer, 1.6 µl of 2.5 mM dNTP, 0.5 µl of 10 µM sense primer (5'-TCTGCAGGGGGCTTTTTGGGAGTAAAAAT-3' (SEQ ID NO: 61)) and 0.5 µl of 10 µM antisense primer (5'-ATTTTTACTCCCAAAAAGCCCCCTGCAGA-3' (SEQ ID NO: 62)) for mutagenesis, 10 ng of pcDNA3.1/Zeo/FcγRIIIA(157V)/Gly-His₆-GST as a template, and 0.4 µl of 2.5 U/µl Pfu Polymerase were combined together on ice. The total volume was adjusted to 20 µl by adding MilliQ water. PCR was carried out under the following conditions: heating at 95°C for 2 minutes, followed by 14 cycles of the three steps of 95°C for 30 seconds, 55°C for 30 seconds, and 68°C for 8 minutes. After reaction, 0.3 µl of 20 U/µl *Dpn*I was added to the reaction solution, and this was incubated at 37°C for one hour. 100 µl of *E. coli* DH5α competent cells was added to the reaction solution, this was left on ice for 30 minutes, and heat-shocked at 42°C for 45 seconds. After two minutes of incubation on ice, the whole amount was plated onto an LB culture plate containing 100 µg/ml ampicillin. The plate was incubated at 37°C overnight. From the formed colonies, those into which the FcyRIIIA(F)/Gly-His₆-GST sequence has been inserted were selected, and the vector was named pcDNA3.1/Zeo/FcγRIIIA(F)/Gly-His₆-GST.

### (v) Gene transfer into 293T and culture

1 x 10⁷ cells of 293T were plated onto a 150-mm cell culture dish and cultured at 37°C under 5% CO₂ for 24 hours. The cells were transfected with 48 µg of pcDNA3.1/Zeo/FcγR/Gly-His₆-GST using TransFast Transfection Reagent, and cultured at 37°C under 5% CO₂ for 24 hours. The medium was discarded. The trypsinized cells were suspended in 120 ml of DMDM selection medium containing 10% fetal bovine serum, 50 µg/ml zeocin, 100 U/ml penicillin, and 100 µg/ml streptomycin, and plated onto four 150-mm cell culture dishes. The cells were then cultured at 37°C under 5% CO₂ for seven days.

### (vi) Purification of Fcγ receptor

The culture supernatants were collected into 50-ml conical tubes, centrifuged at 3000 g for 20 minutes, and collected into an Erlenmeyer flask taking care not to take in the pellets. A column packed with 1 ml of Ni-NTA agarose was equilibrated by loading 5 ml of Native Binding buffer, then the total culture supernatant was loaded thereon. The column was washed by loading 5 ml of Native Wash buffer to remove non-specifically adsorbed materials. Then, Native Elution buffer was loaded and 400-µl were collected per fraction. After SDS-PAGE, staining with BIO-Safe Coomassie and quantification using Image J, an electrophoretic analysis program, were carried out.

### 7-2. Measurement of Fc receptor-binding

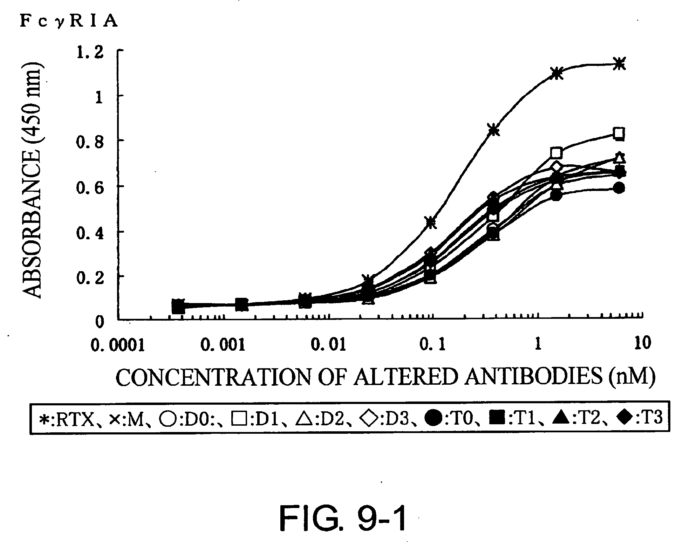

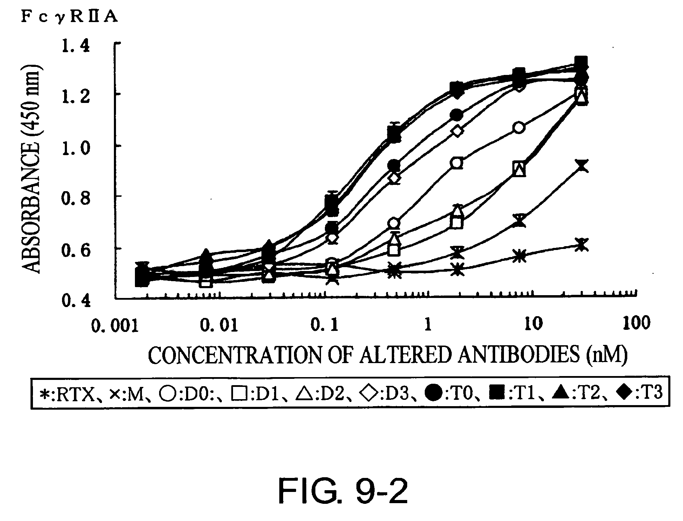

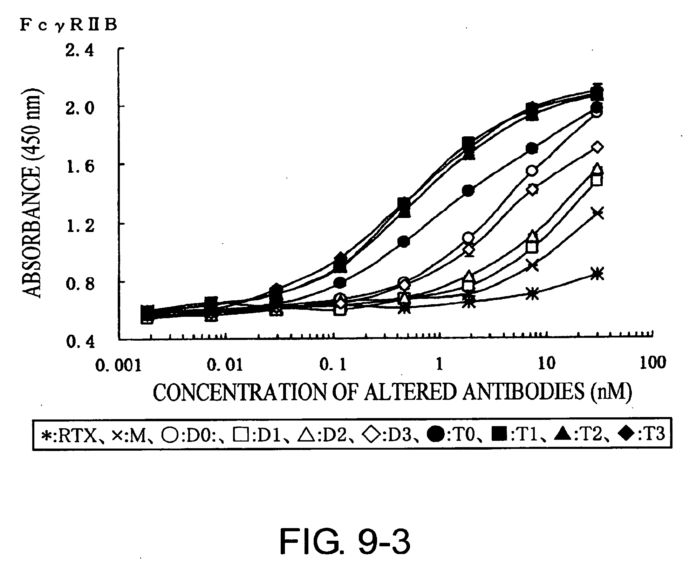

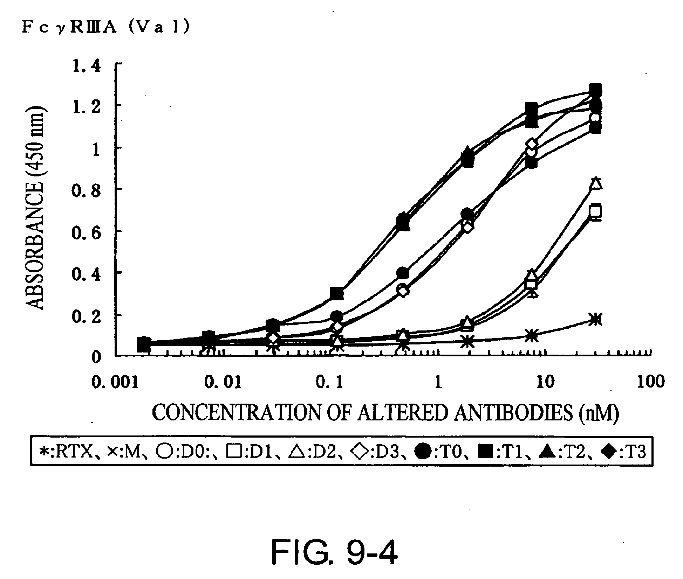

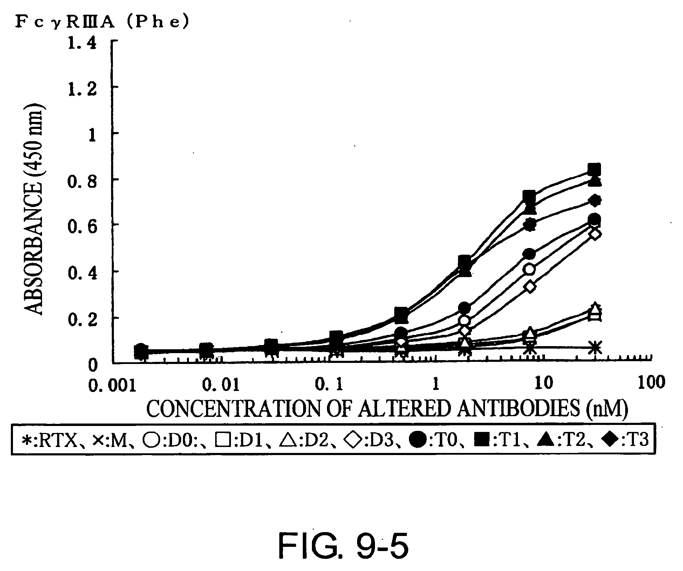

The prepared FcγR (FcγRIA, FcγRIIA, FcγRIIB, FcγRIIIA^{val}, and FcγRIIIA^{Phe}) were adjusted to 4 µg/ml with PBS, aliquoted into 96-well plates at 50 µl per well, and allowed to stand at 4°C overnight. Then the solutions were discarded and 180 µl of ELISA Assay buffer (0.5% BSA, 2 mM EDTA, 0.05% Tween20, 25 mM TBS (pH 7.4)) was added to each well. The plates were allowed to stand and blocked at 37°C for 2 hours. The solutions were discarded, 50 µl of antibody solutions serially diluted with ELISA Assay buffer were added to each well and allowed to react at 37°C for two hours. The antibody solutions were discarded and wells were washed three times with 150 µl of ELISA Assay buffer. 50 µl of ELISA Assay buffer containing 0.33 µg/ml HRP-labeled goat anti-human Kappa chain antibody was aliquoted into each well and allowed to stand at 37°C for one hour. After the antibody solution was removed, wells were washed three times with 150 µl of ELISA Assay buffer. 50 µl of a substrate solution (sodium citrate buffer (pH 5.0) containing 0.4% o-phenylenediamine dihydrochloride and 0.003% H₂O₂) was aliquoted and allowed to stand at room temperature in the dark for ten minutes. Then measurements at A450 were carried out using a microplate reader (Fig. 9).

As shown in Fig. 9-1, all of the antibodies had comparable affinity for FcγRIA. Ab50, the antibody concentration showing 50% of the maximal antibody binding value, was in the range of 0.1 nM to 0.3 nM for all modified antibodies.

As shown in Fig. 9-2, regarding FcγRIIA, the binding intensity of the modified antibodies were in the following order: T1, T2, T3>T0, D3>D0>D1, D2>M. The Trimers were the strongest, the Dimers were next, and M was the weakest. The Ab50 of the Trimers differed from that of M by about 100 times. The Ab50 of D3 was also about 60 times lower than that of M. When the Trimers were compared, the binding activity of T1, T2, and T3, which have spacers, were found to be stronger than that of T0, which has no spacer. With Dimers also, the activity of D3 was stronger than that of D0. The reason why D1 and D2 were weak is probably because there were more Monomers than Dimers contained in the samples.

As shown in Fig. 9-3, regarding FcγRIIB as well, the receptor-binding activities of the modified antibodies were in the following order: Trimers>Dimers>M. With this receptor also, T1, T2, and T3, which have spacers, were stronger than T0, which has no spacer. There was no difference between D3 and D0. The Ab50 of T1, T2, and T3 were about 0.5 nM, while that of D3 was about 5 nM and that of M was about 50 nM.

There are genetic variants of FcγRIIIA, which are the types in which the amino acid at position 158 is valine or phenylalanine. As shown in Fig. 9-4, regarding FcγRIIIA^{Val} as well, the binding intensity shown was as follows: T1, T2, T3>T0, D3, D0>D2, D1, M (Fig. 9-4). The Ab50 of T1, T2, and T3 were about 0.5 nM; the Ab50 of T0, D3, and D0 were about 2 nM; and the Ab50 of D1, D2, and M were about 30 nM. The affinity for the other receptor type, FcγRIIIA^{Phe}, was weaker than that for FcγRIIIA^{val}; however, the order Trimers>Dimers>M was the same (Fig. 9-5).

### [Example 8] ADCC activity assay

### 8-1. Preparation of PBMC effector cells

A suspension of peripheral blood mononuclear cells (PBMC) was prepared as effector cells by the following procedure: 100 ml of blood was collected from healthy adults. 80 ml of 3.5% Dextran 200000 (Wako Pure Chemical Industries, Ltd.) and 0.9% sodium chloride aqueous solution was added to 100 ml of the blood, mixed by inversion, and allowed to stand at room temperature for 20 minutes so that most of the erythrocytes were precipitated. 25 ml of the supernatant was transferred into a 50-ml conical tube, and 25 ml of the RPMI1640 medium was added thereto. Centrifugation was carried out at 400 g for 10 minutes to pellet the cells, then the supernatant was discarded. 20 ml of RPMI1640 medium was added to resuspend the cells. Centrifugation at 400 g for ten minutes was carried out again to pellet the cells, then the supernatant was discarded. The cells were suspended in 30 ml of RPMI1640 medium and were overlaid onto 15 ml of Ficoll-Paque Plus (Amersham) taking care not to disturb the interface. After centrifugation at 400 g for 30 minutes, the white opaque band-like layer formed between the plasma and separation solution was transferred into a different 50-ml conical tube. 20 ml of RPMI1640 medium was added and centrifuged at 400 g for 10 minutes. After confirming the pellet, the supernatant was discarded. 15 ml of ADCC Assay buffer (RPMI 1640 not containing Phenol Red, 1% fetal bovine serum, 2 mM L-glutamine, 10 mM HEPES (pH 7.2), 100 U/ml penicillin, and 100 mg/ml streptomycin) was added thereto, and centrifugation was again carried out at 400 g for ten minutes. The pellet was confirmed and suspended at 5 x 10⁶ cells/ml in ADCC Assay buffer, and this was used as PBMC suspension.

### 8-2. Measurement of ADCC activity

As target cells, Ramos cells were suspended at 2 x 10⁵ cells/ml in ADCC Assay buffer, and 50 µl was aliquoted per well into 96-well round-bottomed multiplates (FALCON 353077) (10⁴ cells/well). Each antibody was serially diluted with ADCC Assay buffer, 50 µl was added to plates, and incubated at 37°C under 5% CO₂ for 30 minutes. 50 µl of the PBMC suspension prepared in (1) was added to each well, and incubated at 37°C under 5% CO₂ for four hours (2.5 x 10⁵ cells/well; effector:target = 25:1). Centrifugation was carried out at 300 g for 10 minutes and 50 µl of the supernatant was transferred onto a 96-well plate. A reaction solution of the Cytotoxic Detection kit (Roche) was prepared and 50 µl was aliquoted onto the 96-well plate. After letting this react for 30 minutes at room temperature, the absorbance at 450 nm was measured. Based on the obtained A450, calculation was carried out using the following formula: "cytotoxicity (%) = 100 x (test sample - target control - effector control) / (2% Tween control - target control)". The target control is a sample to which ADCC Assay buffer was added instead at the step of adding the effector cells. The effector control is a sample to which ADCC Assay buffer was added instead at the step of adding the target cells.

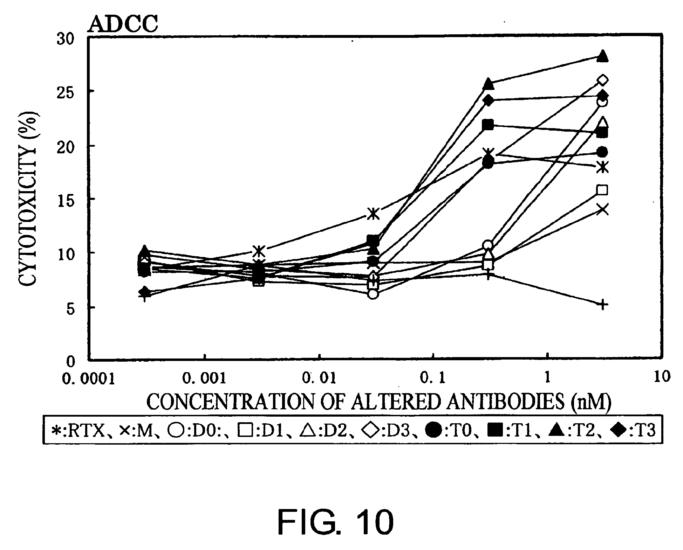

As shown in Fig. 10, T1, T2, and T3 exhibited the strongest ADCC activity, and T0 and D3 came next. D1, D2, and M exhibited only weak cell-killing activity even at the highest antibody concentration. Trastuzumab, which does not bind to the target cells, showed no cytotoxicity.

### [Example 9] CDC activity assay

To use as target cells, CD20-positive human Burkitt's lymphoma Ramos cells were cultured in RPMI1640 containing 10% heat-inactivated fetal bovine serum, 1 mM sodium pyruvate, 100 U/ml penicillin, and 100 µg/ml streptomycin at 37°C under 5% CO₂. Ramos cells were washed with RHB buffer (RPMI 1640 (Sigma Aldrich) not containing Phenol Red, 20 mM HEPES (pH 7.2)(Dojindo Laboratories), 2 mM L-glutamine (Wako Pure Chemical Industries, Ltd.), 0.1% BSA, 100 U/ml penicillin, and 100 mg/ml streptomycin), adjusted to 10⁶ cells/ml, and 50 µl was aliquoted onto a 96-well flat-bottomed multiplate (5 x 10⁴ cells/well). 50 µl of antibodies serially diluted with RHB buffer and 50 µl of fresh baby rabbit serum (Cedarlane Laboratories) 12 times-diluted also with RHB buffer were added and incubated at 37°C under 5% CO₂ for two hours. 50 µl of Alamar Blue (AccuMed International) was added to each well and incubated at 37°C under 5% CO₂ overnight. On the next day, the cover was removed from the plate and, using a fluorescence plate reader (CYTOFLUOR Series 4000; PerSeptive Biosystems), an excitation light of 530 nm was irradiated and fluorescence at 590 nm was measured. From the data obtained as RFU (Relative Fluorescent Unit), calculations were carried out according to the following formula: cytotoxicity (%) = 100 x (RFU background-RFU test sample)/ RFU background. The RFU background corresponds to RFU obtained from a well into which RHB buffer was added instead of antibodies at the step of adding the antibodies.

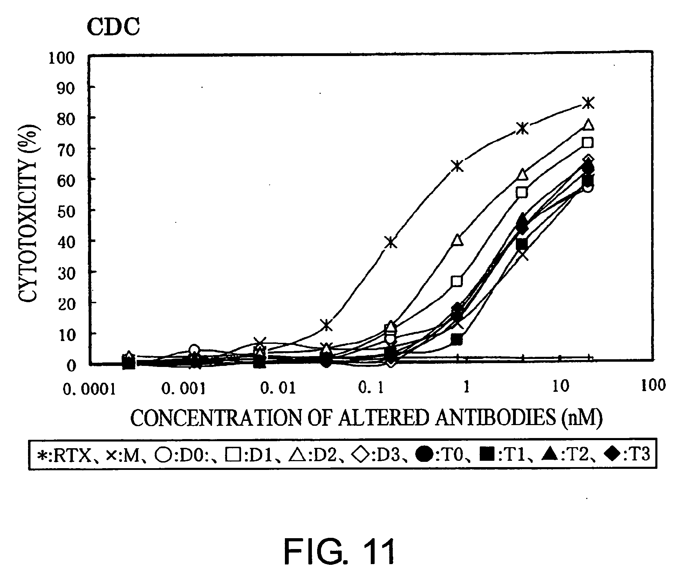

As shown in Fig. 11, no change in the CDC activity was observed by the alterations. Meanwhile, trastuzumab used as a control showed no cytotoxicity.

### Industrial Applicability

The present invention provides novel methods for enhancing the effector activity of antibodies. By using the methods of the present invention, antibody pharmaceuticals that are more effective even at low doses due to enhanced effector activity can be provided, regardless of the antibody-antigen binding activity. It is expected that antibody pharmaceuticals with a remarkable therapeutic effect can be obtained by selecting antibodies with high affinity for antigens that are specific to target cells, such as cancer cells, and modifying the antibodies by the present methods. Furthermore, existing antibody pharmaceuticals already known to be therapeutically effective can be modified to be more effective.

## Claims

1. A method for enhancing an effector activity of an antibody, wherein one or more structures comprising an Fc domain of human IgG1 are linked in tandem to the C terminus of an antibody heavy chain by a genetic engineering technique, wherein the effector activity is antibody-dependent cellular cytotoxicity activity (ADCC activity), provided that when one structure comprising an Fc domain of human IgG1 is linked in tandem to the C terminus of said antibody heavy chain, said structure comprising an Fc domain of human IgG1 comprises a flexible glycine/serine spacer of 15 amino acid residues, in which the basic unit consists of four glycines and one serine, five amino acids in total, at the N-terminal side of the Fc domain of human IgG1.

2. A method for producing a modified antibody with enhanced effector activity, wherein one or more structures comprising an Fc domain of human IgG1 are linked in tandem to the C terminus of an antibody heavy chain by a genetic engineering technique, wherein the effector activity is antibody-dependent cellular cytotoxicity activity (ADCC activity), provided that when one structure comprising an Fc domain of human IgG1 is linked in tandem to the C terminus of said antibody heavy chain, said structure comprising an Fc domain of human IgG1 comprises a flexible glycine/serine spacer of 15 amino acid residues, in which the basic unit consists of four glycines and one serine, five amino acids in total, at the N-terminal side of the Fc domain of human IgG1.

3. A method for producing a modified antibody with enhanced effector activity, wherein the effector activity is antibody-dependent cellular cytotoxicity activity (ADCC activity), which comprises the steps of:
(a) expressing a polynucleotide encoding a light chain and a polynucleotide encoding an altered heavy chain in which one or more structures comprising an Fc domain of human IgG1 are linked in tandem to the C terminus of an antibody heavy chain,
provided that when one structure comprising an Fc domain of human IgG1 is linked in tandem to the C terminus of said antibody heavy chain, said structure comprising an Fc domain of human IgG1 comprises a flexible glycine/serine spacer of 15 amino acid residues, in which the basic unit consists of four glycines and one serine, five amino acids in total, at the N-terminal side of the Fc domain of human IgG1; and
(b) collecting expression products of the polynucleotides.

4. The method of any one of claims 1 to 3, wherein the structures comprise a spacer polypeptide at the N-terminal side of the Fc domain of human IgG1.

5. The method of any one of claims 1 to 4, wherein the number of structures is two.

6. The method of claim 4 or 5, wherein the spacer polypeptide according to claim 4 is selected from the group consisting of SEQ ID NOs: 48, 49, and 50.

7. A modified antibody, wherein one or more structures comprising an Fc domain of human IgG1 are linked in tandem to the C terminus of an antibody heavy chain, provided that when one structure comprising an Fc domain of human IgG1 is linked in tandem to the C terminus of said antibody heavy chain, said structure comprising an Fc domain of human IgG1 comprises a flexible glycine/serine spacer of 15 amino acid residues, in which the basic unit consists of four glycines and one serine, five amino acids in total, at the N-terminal side of the Fc domain of human IgG1.

8. The modified antibody of claim 7, wherein the structures comprise a spacer polypeptide at the N-terminal side of the Fc domain of human IgG1.

9. The modified antibody of claim 7 or 8, wherein the number of structures is two.

10. The modified antibody of any of claims 8 or 9, wherein the spacer polypeptide according to claim 8 is selected from the group consisting of SEQ ID NOs: 48, 49, and 50.

11. The modified antibody of any one of claims 7 to 10 for use in medicine.

12. The modified antibody of any one of claims 7 to 10 for use in a method for treating cancer.

13. The method of any one of claims 1 to 6 or the modified antibody of any one of claims 7 to 10, wherein the said spacer of 15 amino acid residues is GGGGSGGGGSGGGGS.

14. The method of any one of claims 1 to 6, wherein the antibody is an antibody against the B cell-specific differentiation antigen CD20.

15. The modified antibody of any one of claims 7 to 10, which is an antibody against the B cell-specific differentiation antigen CD20.

## Patentansprüche

1. Verfahren zum Verstärken einer Effektoraktivität eines Antikörpers, wobei eine oder mehrere Strukturen, umfassend eine Fc-Domäne von humanem IgG1, mittels einer gentechnischen Technik in Tandem mit dem C-Terminus einer schweren Kette eines Antikörpers verknüpft werden, wobei die Effektoraktivität Aktivität Antikörper-abhängiger zellulärer Zytotoxizität (ADCC-Aktivität) ist,
unter der Maßgabe, dass, wenn eine (1) Struktur, umfassend eine Fc-Domäne von humanem IgG1, in Tandem mit dem C-Terminus der schweren Kette eines Antikörpers verknüpft wird, die Struktur, umfassend eine Fc-Domäne von humanem IgG1, einen flexiblen Glycin/Serin-Spacer von 15 Aminosäureresten, bei welchem die Grundeinheit aus vier Glycinen und einem Serin besteht, fünf Aminosäuren insgesamt, an der N-terminalen Seite der Fc-Domäne von humanem IgG1 umfasst.

2. Verfahren zum Herstellen eines modifizierten Antikörpers mit verstärkter Effektoraktivität, wobei eine oder mehrere Strukturen, umfassend eine Fc-Domäne von humanem IgG1, mittels einer gentechnischen Technik in Tandem mit dem C-Terminus einer schweren Kette eines Antikörpers verknüpft werden, wobei die Effektoraktivität Aktivität Antikörper-abhängiger zellulärer Zytotoxizität (ADCC-Aktivität) ist,
unter der Maßgabe, dass, wenn eine (1) Struktur, umfassend eine Fc-Domäne von humanem IgG1, in Tandem mit dem C-Terminus der schweren Kette eines Antikörpers verknüpft wird, die Struktur, umfassend eine Fc-Domäne von humanem IgG1, einen flexiblen Glycin/Serin-Spacer von 15 Aminosäureresten, bei welchem die Grundeinheit aus vier Glycinen und einem Serin besteht, fünf Aminosäuren insgesamt, an der N-terminalen Seite der FC-Domäne von humanem IgG1 umfasst.

3. Verfahren zum Herstellen eines modifizierten Antikörpers mit verstärkter Effektoraktivität, wobei die Effektoraktivität Aktivität Antikörper-abhängiger zellulärer Zytotoxizität (ADCC-Aktivität) ist, welches die folgenden Schritte umfasst:
(a) Exprimieren eines Polynucleotids, kodierend eine leichte Kette, und eines Polynucleotids, kodierend eine veränderte schwere Kette, bei welcher/m eine oder mehrere Strukturen, umfassend eine Fc-Domäne von humanem IgG1, in Tandem mit dem C-Terminus einer schweren Kette eines Antikörpers verknüpft sind/werden,
unter der Maßgabe, dass, wenn eine (1) Struktur, umfassend eine Fc-Domäne von humanem IgG1, in Tandem mit dem C-Terminus der schweren Kette eines Antikörpers verknüpft ist/wird, die Struktur, umfassend eine Fc-Domäne von humanem IgG1, einen flexiblen Glycin/Serin-Spacer von 15 Aminosäureresten, bei welchem die Grundeinheit aus vier Glycinen und einem Serin besteht, fünf Aminosäuren insgesamt, an der N-terminalen Seite der Fc-Domäne von humanem IgG1 umfasst; und
(b) Sammeln von Expressionsprodukten der Polynucleotide.

4. Verfahren nach einem beliebigen der Ansprüche 1 bis 3, wobei die Strukturen ein Spacer-Polypeptid an der N-terminalen Seite der Fc-Domäne von humanem IgG1 umfassen.

5. Verfahren nach einem beliebigen der Ansprüche 1 bis 4, wobei die Anzahl von Strukturen zwei ist.

6. Verfahren nach Anspruch 4 oder 5, wobei das Spacer-Polypeptid gemäß Anspruch 4 ausgewählt ist aus der Gruppe, bestehend aus SEQ ID Nos: 48, 49 und 50.

7. Modifizierter Antikörper, wobei eine oder mehrere Strukturen, umfassend eine Fc-Domäne von humanem IgG1, in Tandem mit dem C-Terminus einer schweren Kette eines Antikörpers verknüpft sind,
unter der Maßgabe, dass, wenn eine (1) Struktur, umfassend eine Fc-Domäne von humanem IgG1, in Tandem mit dem C-Terminus der schweren Kette eines Antikörpers verknüpft ist, die Struktur, umfassend eine Fc-Domäne von humanem IgG1, einen flexiblen Glycin/Serin-Spacer von 15 Aminosäureresten, bei welchem die Grundeinheit aus vier Glycinen und einem Serin besteht, fünf Aminosäuren insgesamt, an der N-terminalen Seite der Fc-Domäne von humanem IgG1 umfasst.

8. Modifizierter Antikörper nach Anspruch 7, wobei die Strukturen ein Spacer-Polypeptid an der N-terminalen Seite der Fc-Domäne von humanem IgG1 umfassen.

9. Modifizierter Antikörper nach Anspruch 7 oder 8, wobei die Anzahl von Strukturen zwei ist.

10. Modifizierter Antikörper nach einem beliebigen der Ansprüche 8 oder 9, wobei das Spacer-Polypeptid gemäß Anspruch 8 ausgewählt ist aus der Gruppe, bestehend aus SEQ ID Nos: 48, 49 und 50.

11. Modifizierter Antikörper nach einem beliebigen der Ansprüche 7 bis 10 zur Verwendung in der Medizin.

12. Modifizierter Antikörper nach einem beliebigen der Ansprüche 7 bis 10 zur Verwendung in einem Verfahren zum Behandeln von Krebs.

13. Verfahren nach einem beliebigen der Ansprüche 1 bis 6, oder modifizierter Antikörper nach einem beliebigen der Ansprüche 7 bis 10, wobei der Spacer von 15 Aminosäureresten GGGGSGGGGSGGGGS ist.

14. Verfahren nach einem beliebigen der Ansprüche 1 bis 6, wobei der Antikörper ein Antikörper gegen das B-Zell-spezifische Differenzierungsantigen CD20 ist.

15. Modifizierter Antikörper nach einem beliebigen der Ansprüche 7 bis 10, welches einen Antikörper gegen das B-Zell-spezifische Differenzierungsantigen CD20 ist.

## Revendications

1. Procédé pour améliorer une activité effectrice d'un anticorps, dans lequel une ou plusieurs structures comprenant un domaine Fc d'une IgG1 humaine sont liées en tandem à l'extrémité C-terminale d'une chaîne lourde d'anticorps par une technique de génie génétique, dans lequel l'activité effectrice est une activité de cytotoxicité cellulaire dépendante des anticorps (activité ADCC), à condition que lorsqu'une structure comprenant un domaine Fc d'une IgG1 humaine est liée en tandem à l'extrémité C-terminale de ladite chaîne lourde d'anticorps, ladite structure comprenant un domaine Fc d'une IgG1 humaine comprend un espaceur glycine/sérine flexible de 15 résidus d'acides aminés, dans lequel l'unité de base consiste en quatre glycines et une sérine, cinq acides aminés au total, du côté N-terminal du domaine Fc d'une IgG1 humaine.

2. Procédé de production d'un anticorps modifié possédant une activité effectrice améliorée, dans lequel une ou plusieurs structures comprenant un domaine Fc d'une IgG1 humaine sont liées en tandem à l'extrémité C-terminale d'une chaîne lourde d'anticorps par une technique de génie génétique, dans lequel l'activité effectrice est une activité de cytotoxicité cellulaire dépendante des anticorps (activité ADCC), à condition que lorsqu'une structure comprenant un domaine Fc d'une IgG1 humaine est liée en tandem à l'extrémité C-terminale de ladite chaîne lourde d'anticorps, ladite structure comprenant un domaine Fc d'une IgG1 humaine comprend un espaceur glycine/sérine flexible de 15 résidus d'acides aminés, dans lequel l'unité de base consiste en quatre glycines et une sérine, cinq acides aminés au total, du côté N-terminal du domaine Fc d'une IgG1 humaine.

3. Procédé de production d'un anticorps modifié possédant une activité effectrice améliorée, dans lequel l'activité effectrice est une activité de cytotoxicité cellulaire dépendante des anticorps (activité ADCC), qui comprend les étapes consistant à :
(a) exprimer un polynucléotide codant pour une chaîne légère et un polynucléotide codant pour une chaîne lourde modifiée dans laquelle une ou plusieurs structures comprenant un domaine Fc d'une IgG1 humaine sont liées en tandem à l'extrémité C-terminale d'une chaîne lourde d'anticorps, à condition que lorsqu'une structure comprenant un domaine Fc d'une IgG1 humaine est liée en tandem à l'extrémité C-terminale de ladite chaîne lourde d'anticorps, ladite structure comprenant un domaine Fc d'une IgG1 humaine comprend un espaceur glycine/sérine flexible de 15 résidus d'acides aminés, dans lequel l'unité de base consiste en quatre glycines et une sérine, cinq acides aminés au total, du côté N-terminal du domaine Fc d'une IgG1 humaine ; et
(b) collecter les produits d'expression des polynucléotides.

4. Procédé selon l'une quelconque des revendications 1 à 3, dans lequel les structures comprennent un polypeptide espaceur du côté N-terminal du domaine Fc d'une IgG1 humaine.

5. Procédé selon l'une quelconque des revendications 1 à 4, dans lequel le nombre de structures est de deux.

6. Procédé selon la revendication 4 ou 5, dans lequel le polypeptide espaceur selon la revendication 4 est sélectionné dans le groupe consistant en SEQ ID NO: 48, 49, et 50.

7. Anticorps modifié, dans lequel une ou plusieurs structures comprenant un domaine Fc d'une IgG1 humaine sont liées en tandem à l'extrémité C-terminale d'une chaîne lourde d'anticorps, à condition que lorsqu'une structure comprenant un domaine Fc d'une IgG1 humaine est liée en tandem à l'extrémité C-terminale de ladite chaîne lourde d'anticorps, ladite structure comprenant un domaine Fc d'une IgG1 humaine comprend un espaceur glycine/sérine flexible de 15 résidus d'acides aminés, dans lequel l'unité de base consiste en quatre glycines et une sérine, cinq acides aminés au total, du côté N-terminal du domaine Fc d'une IgG1 humaine.

8. Anticorps modifié selon la revendication 7, dans lequel les structures comprennent un polypeptide espaceur du côté N-terminal du domaine Fc d'une IgG1 humaine.

9. Anticorps modifié selon la revendication 7 ou 8, dans lequel le nombre de structures est de deux.

10. Anticorps modifié selon l'une quelconque des revendications 8 ou 9, dans lequel le polypeptide espaceur selon la revendication 8 est sélectionné dans le groupe consistant en SEQ ID NO: 48, 49, et 50.

11. Anticorps modifié selon l'une quelconque des revendications 7 à 10 destiné à être utilisé en médecine.

12. Anticorps modifié selon l'une quelconque des revendications 7 à 10 destiné à être utilisé dans un procédé de traitement du cancer.

13. Procédé selon l'une quelconque des revendications 1 à 6 ou anticorps modifié selon l'une quelconque des revendications 7 à 10, dans lequel ledit espaceur de 15 résidus d'acides aminés est GGGGSGGGGSGGGGS.

14. Procédé selon l'une quelconque des revendications 1 à 6, dans lequel l'anticorps est un anticorps dirigé contre l'antigène de différenciation CD20 spécifique aux cellules B.

15. Anticorps modifié selon l'une quelconque des revendications 7 à 10, qui est un anticorps dirigé contre l'antigène de différenciation CD20 spécifique aux cellules B.


### SEQUENCE LISTING

<110> TOKYO UNIVERSITY OF SCIENCE
<120> A modified antibody improved biological activity
<130> T4-A0601P
<150> JP 2006-057475
   <151> 2006-03-03
<160> 63
<170> PatentIn version 3.3
<210> 1
   <211> 702
   <212> DNA
   <213> Artificial
<220>
   <223> An artificially synthesized nucleotide sequence
<400> 1
   atggattttc aagtgcagat tttcagcttc ctgctaatca gtgcttcagt cataatgtcc     60
   agaggacaaa ttgttctctc ccagtctcca gcaatccttt ctgcatctcc aggggagaag    120
   gtcacaatga cttgcagggc cagctcaagt ttaagtttca tgcactggta ccagcagaag    180
   ccaggatcct cccccaaacc ctggatttat gccacatcca acctggcttc tggagtccct    240
   gctcgcttca gtggcagtgg gtctgggacc tcttactctc tcacaatcag cagagtggag    300
   gctgaagatg ctgccactta tttctgccat cagtggagta gtaacccgct cacgttcggt    360
   gctgggacca agctgctcga gactgtggct gcaccatctg tcttcatctt cccgccatct    420
   gatgagcagt tgaaatctgg aactgcctct gttgtgtgcc tgctgaataa cttctatccc    480
   agagaggcta aagtacagtg gaaggtggat aacgccctcc aatcgggtaa ctcccaggag    540
   agtgtcacag agcaggacag caaggacagc acctacagcc tcagcagcac cctgacgctg    600
   agcaaagcag actacgagaa acacaaagtc tacgcctgcg aagtcaccca tcagggcctg    660
   agctcgcccg tcacaaagag cttcaacagg ggagagtgtt ag                       702
<210> 2
   <211> 233
   <212> PRT
   <213> Artificial
<220>
   <223> An artificially synthesized peptide sequence
<400> 2
   Met Asp Phe Gln Val Gln Ile Phe Ser Phe Leu Leu Ile Ser Ala Ser
   1               5                   10                  15
   Val Ile Met Ser Arg Gly Gln Ile Val Leu Ser Gln Ser Pro Ala Ile
   20 25 30
   Leu Ser Ala Ser Pro Gly Glu Lys Val Thr Met Thr Cys Arg Ala Ser
   35 40 45
   Ser Ser Leu Ser Phe Met His Trp Tyr Gln Gln Lys Pro Gly Ser Ser
   50 55 60
   Pro Lys Pro Trp Ile Tyr Ala Thr Ser Asn Leu Ala Ser Gly Val Pro
   65 70 75 80
   Ala Arg Phe Ser Gly Ser Gly Ser Gly Thr Ser Tyr Ser Leu Thr Ile
   85 90 95
   Ser Arg Val Glu Ala Glu Asp Ala Ala Thr Tyr Phe Cys His Gln Trp
   100 105 110
   Ser Ser Asn Pro Leu Thr Phe Gly Ala Gly Thr Lys Leu Leu Glu Thr
   115 120 125
   Val Ala Ala Pro Ser Val Phe Ile Phe Pro Pro Ser Asp Glu Gln Leu
   130 135 140
   Lys Ser Gly Thr Ala Ser Val Val Cys Leu Leu Asn Asn Phe Tyr Pro
   145 150 155 160
   Arg Glu Ala Lys Val Gln Trp Lys Val Asp Asn Ala Leu Gln Ser Gly
   165 170 175
   Asn Ser Gln Glu Ser Val Thr Glu Gln Asp Ser Lys Asp Ser Thr Tyr
   180 185 190
   Ser Leu Ser Ser Thr Leu Thr Leu Ser Lys Ala Asp Tyr Glu Lys His
   195 200 205
   Lys Val Tyr Ala Cys Glu Val Thr His Gln Gly Leu Ser Ser Pro Val
   210 215 220
   Thr Lys Ser Phe Asn Arg Gly Glu Cys
   225 230
<210> 3
   <211> 1416
   <212> DNA
   <213> Artificial
<220>
   <223> An artificially synthesized nucleotide sequence
<400> 3
   atgggatgga gctgtatcat cttctttttg gtagcaacag ctacaggtgt ccactcccag      60
   gtgcaactgc ggcagcctgg ggctgagctg gtgaagcctg gggcctcagt gaagatgtcc     120
   tgcaaggctt ctggctacac atttaccagt tacaatatgc actgggtaaa gcagacacct     180
   ggacagggcc tggaatggat tggagctatt tatccaggaa atggtgatac ttcctacaat     240
   cagaagttca aaggcaaggc cacattgact gcagacaaat cctccagcac agcctacatg     300
   cagctcagca gtctgacatc tgaggactct gcggtctatt actgtgcaag atcgcactac     360
   ggtagtaact acgtagacta ctttgactac tggggccaag gcaccactct cacagtctcc     420
   tctaagctta ccaagggccc atcggtcttc cccctggcac cctcctccaa gagcacctct     480
   gggggcacag cggccctggg ctgcctggtc aaggactact tccccgaacc ggtgacggtg     540
   tcgtggaact caggcgccct gaccagcggc gtgcacacct tcccggctgt cctacagtcc     600
   tcaggactct actccctcag cagcgtggtg accgtgccct ccagcagctt gggcacccag     660
   acctacatct gcaacgtgaa tcacaagccc agcaacacca aggtggacaa gaaggttgag     720
   cccaaatctt gtgacaaaac tcacacatgc ccaccgtgcc cagcacctga actcctgggg     780
   ggaccgtcag tcttcctctt ccccccaaaa cccaaggaca ccctcatgat ctcccggacc     840
   cctgaggtca catgcgtggt ggtggacgtg agccacgaag accctgaggt caagttcaac     900
   tggtacgtgg acggcgtgga ggtgcataat gccaagacaa agccgcggga ggagcagtac     960
   aacagcacgt accgtgtggt cagcgtcctc accgtcctgc accaggactg gctgaatggc    1020
   aaggagtaca agtgcaaggt ctccaacaaa gccctcccag cccccatcga gaaaaccatc    1080
   tccaaagcca aagggcagcc ccgagaacca caggtgtaca ccctgccccc atcccgggat    1140
   gagctgacca agaaccaggt cagcctgacc tgcctggtca aaggcttcta tcccagcgac    1200
   atcgccgtgg agtgggagag caatgggcag ccggagaaca actacaagac cacgcctccc    1260
   gtgctggact ccgacggctc cttcttcctc tacagcaagc tcaccgtgga caagagcagg    1320
   tggcagcagg ggaacgtctt ctcatgctcc gtgatgcatg aggctctgca caaccactac    1380
   acgcagaaga gcctctccct gtctccgggt aaatga                               1416
<210> 4
   <211> 471
   <212> PRT
   <213> Artificial
<220>
   <223> An artificially synthesized peptide sequence
<400> 4
   Met Gly Trp Ser Cys Ile Ile Phe Phe Leu Val Ala Thr Ala Thr Gly
   1               5                   10                  15
   Val His Ser Gln Val Gln Leu Arg Gln Pro Gly Ala Glu Leu Val Lys
   20 25 30
   Pro Gly Ala Ser Val Lys Met Ser Cys Lys Ala Ser Gly Tyr Thr Phe
   35 40 45
   Thr Ser Tyr Asn Met His Trp Val Lys Gln Thr Pro Gly Gln Gly Leu
   50 55 60
   Glu Trp Ile Gly Ala Ile Tyr Pro Gly Asn Gly Asp Thr Ser Tyr Asn
   65 70 75 80
   Gln Lys Phe Lys Gly Lys Ala Thr Leu Thr Ala Asp Lys Ser Ser Ser
   85 90 95
   Thr Ala Tyr Met Gln Leu Ser Ser Leu Thr Ser Glu Asp Ser Ala Val
   100 105 110
   Tyr Tyr Cys Ala Arg Ser His Tyr Gly Ser Asn Tyr Val Asp Tyr Phe
   115 120 125
   Asp Tyr Trp Gly Gln Gly Thr Thr Leu Thr Val Ser Ser Lys Leu Thr
   130 135 140
   Lys Gly Pro Ser Val Phe Pro Leu Ala Pro Ser Ser Lys Ser Thr Ser
   145 150 155 160
   Gly Gly Thr Ala Ala Leu Gly Cys Leu Val Lys Asp Tyr Phe Pro Glu
   165 170 175
   Pro Val Thr Val Ser Trp Asn Ser Gly Ala Leu Thr Ser Gly Val His
   180 185 190
   Thr Phe Pro Ala Val Leu Gln Ser Ser Gly Leu Tyr Ser Leu Ser Ser
   195 200 205
   Val Val Thr Val Pro Ser Ser Ser Leu Gly Thr Gln Thr Tyr Ile Cys
   210 215 220
   Asn Val Asn His Lys Pro Ser Asn Thr Lys Val Asp Lys Lys Val Glu
   225 230 235 240
   Pro Lys Ser Cys Asp Lys Thr His Thr Cys Pro Pro Cys Pro Ala Pro
   245 250 255
   Glu Leu Leu Gly Gly Pro Ser Val Phe Leu Phe Pro Pro Lys Pro Lys
   260 265 270
   Asp Thr Leu Met Ile Ser Arg Thr Pro Glu Val Thr Cys Val Val Val
   275 280 285
   Asp Val Ser His Glu Asp Pro Glu Val Lys Phe Asn Trp Tyr Val Asp
   290 295 300
   Gly Val Glu Val His Asn Ala Lys Thr Lys Pro Arg Glu Glu Gln Tyr
   305 310 315 320
   Asn Ser Thr Tyr Arg Val Val Ser Val Leu Thr Val Leu His Gln Asp
   325 330 335
   Trp Leu Asn Gly Lys Glu Tyr Lys Cys Lys Val Ser Asn Lys Ala Leu
   340 345 350
   Pro Ala Pro Ile Glu Lys Thr Ile Ser Lys Ala Lys Gly Gln Pro Arg
   355 360 365
   Glu Pro Gln Val Tyr Thr Leu Pro Pro Ser Arg Asp Glu Leu Thr Lys
   370 375 380
   Asn Gln Val Ser Leu Thr Cys Leu Val Lys Gly Phe Tyr Pro Ser Asp
   385 390 395 400
   Ile Ala Val Glu Trp Glu Ser Asn Gly Gln Pro Glu Asn Asn Tyr Lys
   405 410 415
   Thr Thr Pro Pro Val Leu Asp Ser Asp Gly Ser Phe Phe Leu Tyr Ser
   420 425 430
   Lys Leu Thr Val Asp Lys Ser Arg Trp Gln Gln Gly Asn Val Phe Ser
   435 440 445
   Cys Ser Val Met His Glu Ala Leu His Asn His Tyr Thr Gln Lys Ser
   450 455 460
   Leu Ser Leu Ser Pro Gly Lys
   465 470
<210> 5
   <211> 2115
   <212> DNA
   <213> Artificial
<220>
   <223> An artificially synthesized nucleotide sequence
<400> 5
   atgggatgga gctgtatcat cttctttttg gtagcaacag ctacaggtgt ccactcccag      60
   gtgcaactgc ggcagcctgg ggctgagctg gtgaagcctg gggcctcagt gaagatgtcc     120
   tgcaaggctt ctggctacac atttaccagt tacaatatgc actgggtaaa gcagacacct     180
   ggacagggcc tggaatggat tggagctatt tatccaggaa atggtgatac ttcctacaat     240
   cagaagttca aaggcaaggc cacattgact gcagacaaat cctccagcac agcctacatg     300
   cagctcagca gtctgacatc tgaggactct gcggtctatt actgtgcaag atcgcactac     360
   ggtagtaact acgtagacta ctttgactac tggggccaag gcaccactct cacagtctcc     420
   tctaagctta ccaagggccc atcggtcttc cccctggcac cctcctccaa gagcacctct     480
   gggggcacag cggccctggg ctgcctggtc aaggactact tccccgaacc ggtgacggtg     540
   tcgtggaact caggcgccct gaccagcggc gtgcacacct tcccggctgt cctacagtcc     600
   tcaggactct actccctcag cagcgtggtg accgtgccct ccagcagctt gggcacccag     660
   acctacatct gcaacgtgaa tcacaagccc agcaacacca aggtggacaa gaaggttgag     720
   cccaaatctt gtgacaaaac tcacacatgc ccaccgtgcc cagcacctga actcctgggg     780
   ggaccgtcag tcttcctctt ccccccaaaa cccaaggaca ccctcatgat ctcccggacc     840
   cctgaggtca catgcgtggt ggtggacgtg agccacgaag accctgaggt caagttcaac     900
   tggtacgtgg acggcgtgga ggtgcataat gccaagacaa agccgcggga ggagcagtac     960
   aacagcacgt accgtgtggt cagcgtcctc accgtcctgc accaggactg gctgaatggc    1020
   aaggagtaca agtgcaaggt ctccaacaaa gccctcccag cccccatcga gaaaaccatc    1080
   tccaaagcca aagggcagcc ccgagaacca caggtgtaca ccctgccccc atcccgggat    1140
   gagctgacca agaaccaggt cagcctgacc tgcctggtca aaggcttcta tcccagcgac    1200
   atcgccgtgg agtgggagag caatgggcag ccggagaaca actacaagac cacgcctccc    1260
   gtgctggact ccgacggctc cttcttcctc tacagcaagc tcaccgtgga caagagcagg    1320
   tggcagcagg ggaacgtctt ctcatgctcc gtgatgcatg aggctctgca caaccactac    1380
   acgcagaaga gcctctccct gtctccgggt aaaggatccc ccaaatctag tgacaaaact    1440
   cacacatgcc caccgtgccc agcacctgaa ctcctggggg gaccgtcagt cttcctcttc    1500
   cccccaaaac ccaaggacac cctcatgatc tcccggaccc ctgaggtcac atgcgtggtg    1560
   gtggacgtga gccacgaaga ccctgaggtc aagttcaact ggtacgtgga cggcgtggag    1620
   gtgcataatg ccaagacaaa gccgcgggag gagcagtaca acagcacgta ccgtgtggtc    1680
   agcgtcctca ccgtcctgca ccaggactgg ctgaatggca aggagtacaa gtgcaaggtc    1740
   tccaacaaag ccctcccagc ccccatcgag aaaaccatct ccaaagccaa agggcagccc    1800
   cgagaaccac aggtgtacac cctgccccca tcccgggatg agctgaccaa gaaccaggtc    1860
   agcctgacct gcctggtcaa aggcttctat cccagcgaca tcgccgtgga gtgggagagc 1920
   aatgggcagc cggagaacaa ctacaagacc acgcctcccg tgctggactc cgacggctcc 1980
   ttcttcctct acagcaagct caccgtggac aagagcaggt ggcagcaggg gaacgtcttc 2040
   tcatgctccg tgatgcatga ggctctgcac aaccactaca cgcagaagag cctctccctg 2100
   tctccgggta aatga 2115
<210> 6
   <211> 704
   <212> PRT
   <213> Artificial
<220>
   <223> An artificially synthesized peptide sequence
<400> 6
<210> 7
   <211> 2130
   <212> DNA
   <213> Artificial
<220>
   <223> An artificially synthesized nucleotide sequence
<400> 7
   atgggatgga gctgtatcat cttctttttg gtagcaacag ctacaggtgt ccactcccag 60
   gtgcaactgc ggcagcctgg ggctgagctg gtgaagcctg gggcctcagt gaagatgtcc 120
   tgcaaggctt ctggctacac atttaccagt tacaatatgc actgggtaaa gcagacacct 180
   ggacagggcc tggaatggat tggagctatt tatccaggaa atggtgatac ttcctacaat 240
   cagaagttca aaggcaaggc cacattgact gcagacaaat cctccagcac agcctacatg 300
   cagctcagca gtctgacatc tgaggactct gcggtctatt actgtgcaag atcgcactac 360
   ggtagtaact acgtagacta ctttgactac tggggccaag gcaccactct cacagtctcc 420
   tctaagctta ccaagggccc atcggtcttc cccctggcac cctcctccaa gagcacctct 480
   gggggcacag cggccctggg ctgcctggtc aaggactact tccccgaacc ggtgacggtg 540
   tcgtggaact caggcgccct gaccagcggc gtgcacacct tcccggctgt cctacagtcc 600
   tcaggactct actccctcag cagcgtggtg accgtgccct ccagcagctt gggcacccag 660
   acctacatct gcaacgtgaa tcacaagccc agcaacacca aggtggacaa gaaggttgag 720
   cccaaatctt gtgacaaaac tcacacatgc ccaccgtgcc cagcacctga actcctgggg 780
   ggaccgtcag tcttcctctt ccccccaaaa cccaaggaca ccctcatgat ctcccggacc 840
   cctgaggtca catgcgtggt ggtggacgtg agccacgaag accctgaggt caagttcaac 900
   tggtacgtgg acggcgtgga ggtgcataat gccaagacaa agccgcggga ggagcagtac 960
   aacagcacgt accgtgtggt cagcgtcctc accgtcctgc accaggactg gctgaatggc 1020
   aaggagtaca agtgcaaggt ctccaacaaa gccctcccag cccccatcga gaaaaccatc 1080
   tccaaagcca aagggcagcc ccgagaacca caggtgtaca ccctgccccc atcccgggat 1140
   gagctgacca agaaccaggt cagcctgacc tgcctggtca aaggcttcta tcccagcgac 1200
   atcgccgtgg agtgggagag caatgggcag ccggagaaca actacaagac cacgcctccc 1260
   gtgctggact ccgacggctc cttcttcctc tacagcaagc tcaccgtgga caagagcagg 1320
   tggcagcagg ggaacgtctt ctcatgctcc gtgatgcatg aggctctgca caaccactac 1380
   acgcagaaga gcctctccct gtctccgggt aaaggatccg gtggcggtgg ctcgcccaaa 1440
   tctagtgaca aaactcacac atgcccaccg tgcccagcac ctgaactcct ggggggaccg 1500
   tcagtcttcc tcttcccccc aaaacccaag gacaccctca tgatctcccg gacccctgag 1560
   gtcacatgcg tggtggtgga cgtgagccac gaagaccctg aggtcaagtt caactggtac 1620
   gtggacggcg tggaggtgca taatgccaag acaaagccgc gggaggagca gtacaacagc 1680
   acgtaccgtg tggtcagcgt cctcaccgtc ctgcaccagg actggctgaa tggcaaggag 1740
   tacaagtgca aggtctccaa caaagccctc ccagccccca tcgagaaaac catctccaaa 1800
   gccaaagggc agccccgaga accacaggtg tacaccctgc ccccatcccg ggatgagctg 1860
   accaagaacc aggtcagcct gacctgcctg gtcaaaggct tctatcccag cgacatcgcc 1920
   gtggagtggg agagcaatgg gcagccggag aacaactaca agaccacgcc tcccgtgctg 1980
   gactccgacg gctccttctt cctctacagc aagctcaccg tggacaagag caggtggcag 2040
   caggggaacg tcttctcatg ctccgtgatg catgaggctc tgcacaacca ctacacgcag 2100
   aagagcctct ccctgtctcc gggtaaatga 2130
<210> 8
   <211> 709
   <212> PRT
   <213> Artificial
<220>
   <223> An artificially synthesized peptide sequence
<400> 8
   Met Gly Trp Ser Cys Ile Ile Phe Phe Leu Val Ala Thr Ala Thr Gly
   1               5                   10                  15
   Val His Ser Gln Val Gln Leu Arg Gln Pro Gly Ala Glu Leu Val Lys
               20                  25                  30
   Pro Gly Ala Ser Val Lys Met Ser Cys Lys Ala Ser Gly Tyr Thr Phe
           35                  40                  45
   Thr Ser Tyr Asn Met His Trp Val Lys Gln Thr Pro Gly Gln Gly Leu
       50                  55                  60
   Glu Trp Ile Gly Ala Ile Tyr Pro Gly Asn Gly Asp Thr Ser Tyr Asn
   65                  70                  75                  80
   Gln Lys Phe Lys Gly Lys Ala Thr Leu Thr Ala Asp Lys Ser Ser Ser
                   85                  90                  95
   Thr Ala Tyr Met Gln Leu Ser Ser Leu Thr Ser Glu Asp Ser Ala Val
               100                 105                 110
   Tyr Tyr Cys Ala Arg Ser His Tyr Gly Ser Asn Tyr Val Asp Tyr Phe
           115                 120                 125
   Asp Tyr Trp Gly Gln Gly Thr Thr Leu Thr Val Ser Ser Lys Leu Thr
       130                 135                 140
   Lys Gly Pro Ser Val Phe Pro Leu Ala Pro Ser Ser Lys Ser Thr Ser
   145                 150                 155                 160
   Gly Gly Thr Ala Ala Leu Gly Cys Leu Val Lys Asp Tyr Phe Pro Glu
                   165                 170                 175
   Pro Val Thr Val Ser Trp Asn Ser Gly Ala Leu Thr Ser Gly Val His
               180                 185                 190
   Thr Phe Pro Ala Val Leu Gln Ser Ser Gly Leu Tyr Ser Leu Ser Ser
           195                 200                 205
   Val Val Thr Val Pro Ser Ser Ser Leu Gly Thr Gln Thr Tyr Ile Cys
       210                 215                 220
   Asn Val Asn His Lys Pro Ser Asn Thr Lys Val Asp Lys Lys Val Glu
   225                 230                 235                 240
   Pro Lys Ser Cys Asp Lys Thr His Thr Cys Pro Pro Cys Pro Ala Pro
                   245                 250                 255
   Glu Leu Leu Gly Gly Pro Ser Val Phe Leu Phe Pro Pro Lys Pro Lys
               260                 265                 270
   Asp Thr Leu Met Ile Ser Arg Thr Pro Glu Val Thr Cys Val Val Val
           275                 280                 285
   Asp Val Ser His Glu Asp Pro Glu Val Lys Phe Asn Trp Tyr Val Asp
       290                 295                 300
   Gly Val Glu Val His Asn Ala Lys Thr Lys Pro Arg Glu Glu Gln Tyr
   305                 310                 315                 320
   Asn Ser Thr Tyr Arg Val Val Ser Val Leu Thr Val Leu His Gln Asp
                   325                 330                 335
   Trp Leu Asn Gly Lys Glu Tyr Lys Cys Lys Val Ser Asn Lys Ala Leu
               340                 345                 350
   Pro Ala Pro Ile Glu Lys Thr Ile Ser Lys Ala Lys Gly Gln Pro Arg
   355 360 365
   Glu Pro Gln Val Tyr Thr Leu Pro Pro Ser Arg Asp Glu Leu Thr Lys
   370 375 380
   Asn Gln Val Ser Leu Thr Cys Leu Val Lys Gly Phe Tyr Pro Ser Asp
   385 390 395 400
   Ile Ala Val Glu Trp Glu Ser Asn Gly Gln Pro Glu Asn Asn Tyr Lys
   405 410 415
   Thr Thr Pro Pro Val Leu Asp Ser Asp Gly Ser Phe Phe Leu Tyr Ser
   420 425 430
   Lys Leu Thr Val Asp Lys Ser Arg Trp Gln Gln Gly Asn Val Phe Ser
   435 440 445
   Cys Ser Val Met His Glu Ala Leu His Asn His Tyr Thr Gln Lys Ser
   450 455 460
   Leu Ser Leu Ser Pro Gly Lys Gly Ser Gly Gly Gly Gly Ser Pro Lys
   465 470 475 480
   Ser Ser Asp Lys Thr His Thr Cys Pro Pro Cys Pro Ala Pro Glu Leu
   485 490 495
   Leu Gly Gly Pro Ser Val Phe Leu Phe Pro Pro Lys Pro Lys Asp Thr
   500 505 510
   Leu Met Ile Ser Arg Thr Pro Glu Val Thr Cys Val Val Val Asp Val
   515 520 525
   Ser His Glu Asp Pro Glu Val Lys Phe Asn Trp Tyr Val Asp Gly Val
   530 535 540
   Glu Val His Asn Ala Lys Thr Lys Pro Arg Glu Glu Gln Tyr Asn Ser
   545 550 555 560
   Thr Tyr Arg Val Val Ser Val Leu Thr Val Leu His Gln Asp Trp Leu
   565 570 575
   Asn Gly Lys Glu Tyr Lys Cys Lys Val Ser Asn Lys Ala Leu Pro Ala
   580 585 590
   Pro Ile Glu Lys Thr Ile Ser Lys Ala Lys Gly Gln Pro Arg Glu Pro
   595 600 605
   Gln Val Tyr Thr Leu Pro Pro Ser Arg Asp Glu Leu Thr Lys Asn Gln
   610 615 620
   Val Ser Leu Thr Cys Leu Val Lys Gly Phe Tyr Pro Ser Asp Ile Ala
   625 630 635 640
   Val Glu Trp Glu Ser Asn Gly Gln Pro Glu Asn Asn Tyr Lys Thr Thr
   645 650 655
   Pro Pro Val Leu Asp Ser Asp Gly Ser Phe Phe Leu Tyr Ser Lys Leu
   660 665 670
   Thr Val Asp Lys Ser Arg Trp Gln Gln Gly Asn Val Phe Ser Cys Ser
   675 680 685
   Val Met His Glu Ala Leu His Asn His Tyr Thr Gln Lys Ser Leu Ser
   690 695 700
   Leu Ser Pro Gly Lys
   705
<210> 9
   <211> 2145
   <212> DNA
   <213> Artificial
<220>
   <223> An artificially synthesized nucleotide sequence
<400> 9
   atgggatgga gctgtatcat cttctttttg gtagcaacag ctacaggtgt ccactcccag     60
   gtgcaactgc ggcagcctgg ggctgagctg gtgaagcctg gggcctcagt gaagatgtcc    120
   tgcaaggctt ctggctacac atttaccagt tacaatatgc actgggtaaa gcagacacct    180
   ggacagggcc tggaatggat tggagctatt tatccaggaa atggtgatac ttcctacaat    240
   cagaagttca aaggcaaggc cacattgact gcagacaaat cctccagcac agcctacatg    300
   cagctcagca gtctgacatc tgaggactct gcggtctatt actgtgcaag atcgcactac    360
   ggtagtaact acgtagacta ctttgactac tggggccaag gcaccactct cacagtctcc    420
   tctaagctta ccaagggccc atcggtcttc cccctggcac cctcctccaa gagcacctct    480
   gggggcacag cggccctggg ctgcctggtc aaggactact tccccgaacc ggtgacggtg    540
   tcgtggaact caggcgccct gaccagcggc gtgcacacct tcccggctgt cctacagtcc    600
   tcaggactct actccctcag cagcgtggtg accgtgccct ccagcagctt gggcacccag    660
   acctacatct gcaacgtgaa tcacaagccc agcaacacca aggtggacaa gaaggttgag    720
   cccaaatctt gtgacaaaac tcacacatgc ccaccgtgcc cagcacctga actcctgggg    780
   ggaccgtcag tcttcctctt ccccccaaaa cccaaggaca ccctcatgat ctcccggacc    840
   cctgaggtca catgcgtggt ggtggacgtg agccacgaag accctgaggt caagttcaac    900
   tggtacgtgg acggcgtgga ggtgcataat gccaagacaa agccgcggga ggagcagtac    960
   aacagcacgt accgtgtggt cagcgtcctc accgtcctgc accaggactg gctgaatggc   1020
   aaggagtaca agtgcaaggt ctccaacaaa gccctcccag cccccatcga gaaaaccatc   1080
   tccaaagcca aagggcagcc ccgagaacca caggtgtaca ccctgccccc atcccgggat   1140
   gagctgacca agaaccaggt cagcctgacc tgcctggtca aaggcttcta tcccagcgac   1200
   atcgccgtgg agtgggagag caatgggcag ccggagaaca actacaagac cacgcctccc   1260
   gtgctggact ccgacggctc cttcttcctc tacagcaagc tcaccgtgga caagagcagg   1320
   tggcagcagg ggaacgtctt ctcatgctcc gtgatgcatg aggctctgca caaccactac   1380
   acgcagaaga gcctctccct gtctccgggt aaaggatccg gtggcggtgg ctcgggcggt   1440
   ggtgggtcgc ccaaatctag tgacaaaact cacacatgcc caccgtgccc agcacctgaa   1500
   ctcctggggg gaccgtcagt cttcctcttc cccccaaaac ccaaggacac cctcatgatc   1560
   tcccggaccc ctgaggtcac atgcgtggtg gtggacgtga gccacgaaga ccctgaggtc   1620
   aagttcaact ggtacgtgga cggcgtggag gtgcataatg ccaagacaaa gccgcgggag   1680
   gagcagtaca acagcacgta ccgtgtggtc agcgtcctca ccgtcctgca ccaggactgg   1740
   ctgaatggca aggagtacaa gtgcaaggtc tccaacaaag ccctcccagc ccccatcgag   1800
   aaaaccatct ccaaagccaa agggcagccc cgagaaccac aggtgtacac cctgccccca   1860
   tcccgggatg agctgaccaa gaaccaggtc agcctgacct gcctggtcaa aggcttctat   1920
   cccagcgaca tcgccgtgga gtgggagagc aatgggcagc cggagaacaa ctacaagacc   1980
   acgcctcccg tgctggactc cgacggctcc ttcttcctct acagcaagct caccgtggac   2040
   aagagcaggt ggcagcaggg gaacgtcttc tcatgctccg tgatgcatga ggctctgcac   2100
   aaccactaca cgcagaagag cctctccctg tctccgggta aatga                   2145
<210> 10
   <211> 714
   <212> PRT
   <213> Artificial
<220>
   <223> An artificially synthesized peptide sequence
<400> 10
   Met Gly Trp Ser Cys Ile Ile Phe Phe Leu Val Ala Thr Ala Thr Gly
   1               5                   10                  15
   Val His Ser Gln Val Gln Leu Arg Gln Pro Gly Ala Glu Leu Val Lys
               20                  25                  30
   Pro Gly Ala Ser Val Lys Met Ser Cys Lys Ala Ser Gly Tyr Thr Phe
   35 40 45
   Thr Ser Tyr Asn Met His Trp Val Lys Gln Thr Pro Gly Gln Gly Leu
   50 55 60
   Glu Trp Ile Gly Ala Ile Tyr Pro Gly Asn Gly Asp Thr Ser Tyr Asn
   65 70 75 80
   Gln Lys Phe Lys Gly Lys Ala Thr Leu Thr Ala Asp Lys Ser Ser Ser
   85 90 95
   Thr Ala Tyr Met Gln Leu Ser Ser Leu Thr Ser Glu Asp Ser Ala Val
   100 105 110
   Tyr Tyr Cys Ala Arg Ser His Tyr Gly Ser Asn Tyr Val Asp Tyr Phe
   115 120 125
   Asp Tyr Trp Gly Gln Gly Thr Thr Leu Thr Val Ser Ser Lys Leu Thr
   130 135 140
   Lys Gly Pro Ser Val Phe Pro Leu Ala Pro Ser Ser Lys Ser Thr Ser
   145 150 155 160
   Gly Gly Thr Ala Ala Leu Gly Cys Leu Val Lys Asp Tyr Phe Pro Glu
   165 170 175
   Pro Val Thr Val Ser Trp Asn Ser Gly Ala Leu Thr Ser Gly Val His
   180 185 190
   Thr Phe Pro Ala Val Leu Gln Ser Ser Gly Leu Tyr Ser Leu Ser Ser
   195 200 205
   Val Val Thr Val Pro Ser Ser Ser Leu Gly Thr Gln Thr Tyr Ile Cys
   210 215 220
   Asn Val Asn His Lys Pro Ser Asn Thr Lys Val Asp Lys Lys Val Glu
   225 230 235 240
   Pro Lys Ser Cys Asp Lys Thr His Thr Cys Pro Pro Cys Pro Ala Pro
   245 250 255
   Glu Leu Leu Gly Gly Pro Ser Val Phe Leu Phe Pro Pro Lys Pro Lys
   260 265 270
   Asp Thr Leu Met Ile Ser Arg Thr Pro Glu Val Thr Cys Val Val Val
   275 280 285
   Asp Val Ser His Glu Asp Pro Glu Val Lys Phe Asn Trp Tyr Val Asp
   290 295 300
   Gly Val Glu Val His Asn Ala Lys Thr Lys Pro Arg Glu Glu Gln Tyr
   305 310 315 320
   Asn Ser Thr Tyr Arg Val Val Ser Val Leu Thr Val Leu His Gln Asp
   325 330 335
   Trp Leu Asn Gly Lys Glu Tyr Lys Cys Lys Val Ser Asn Lys Ala Leu
   340 345 350
   Pro Ala Pro Ile Glu Lys Thr Ile Ser Lys Ala Lys Gly Gln Pro Arg
   355 360 365
   Glu Pro Gln Val Tyr Thr Leu Pro Pro Ser Arg Asp Glu Leu Thr Lys
   370 375 380
   Asn Gln Val Ser Leu Thr Cys Leu Val Lys Gly Phe Tyr Pro Ser Asp
   385 390 395 400
   Ile Ala Val Glu Trp Glu Ser Asn Gly Gln Pro Glu Asn Asn Tyr Lys
   405 410 415
   Thr Thr Pro Pro Val Leu Asp Ser Asp Gly Ser Phe Phe Leu Tyr Ser
   420 425 430
   Lys Leu Thr Val Asp Lys Ser Arg Trp Gln Gln Gly Asn Val Phe Ser
   435 440 445
   Cys Ser Val Met His Glu Ala Leu His Asn His Tyr Thr Gln Lys Ser
   450 455 460
   Leu Ser Leu Ser Pro Gly Lys Gly Ser Gly Gly Gly Gly Ser Gly Gly
   465 470 475 480
   Gly Gly Ser Pro Lys Ser Ser Asp Lys Thr His Thr Cys Pro Pro Cys
   485 490 495
   Pro Ala Pro Glu Leu Leu Gly Gly Pro Ser Val Phe Leu Phe Pro Pro
   500 505 510
   Lys Pro Lys Asp Thr Leu Met Ile Ser Arg Thr Pro Glu Val Thr Cys
   515 520 525
   Val Val Val Asp Val Ser His Glu Asp Pro Glu Val Lys Phe Asn Trp
       530                 535                 540
   Tyr Val Asp Gly Val Glu Val His Asn Ala Lys Thr Lys Pro Arg Glu
   545                 550                 555                 560
   Glu Gln Tyr Asn Ser Thr Tyr Arg Val Val Ser Val Leu Thr Val Leu
                   565                 570                 575
   His Gln Asp Trp Leu Asn Gly Lys Glu Tyr Lys Cys Lys Val Ser Asn
               580                 585                 590
   Lys Ala Leu Pro Ala Pro Ile Glu Lys Thr Ile Ser Lys Ala Lys Gly
           595                 600                 605
   Gln Pro Arg Glu Pro Gln Val Tyr Thr Leu Pro Pro Ser Arg Asp Glu
       610                 615                 620
   Leu Thr Lys Asn Gln Val Ser Leu Thr Cys Leu Val Lys Gly Phe Tyr
   625                 630                 635                 640
   Pro Ser Asp Ile Ala Val Glu Trp Glu Ser Asn Gly Gln Pro Glu Asn
                   645                 650                 655
   Asn Tyr Lys Thr Thr Pro Pro Val Leu Asp Ser Asp Gly Ser Phe Phe
               660                 665                 670
   Leu Tyr Ser Lys Leu Thr Val Asp Lys Ser Arg Trp Gln Gln Gly Asn
           675                 680                 685
   Val Phe Ser Cys Ser Val Met His Glu Ala Leu His Asn His Tyr Thr
       690                 695                 700
   Gln Lys Ser Leu Ser Leu Ser Pro Gly Lys
   705                 710
<210> 11
   <211> 2160
   <212> DNA
   <213> Artificial
<220>
   <223> An artificially synthesized nucleotide sequence
<400> 11
   atgggatgga gctgtatcat cttctttttg gtagcaacag ctacaggtgt ccactcccag 60
   gtgcaactgc ggcagcctgg ggctgagctg gtgaagcctg gggcctcagt gaagatgtcc 120
   tgcaaggctt ctggctacac atttaccagt tacaatatgc actgggtaaa gcagacacct 180
   ggacagggcc tggaatggat tggagctatt tatccaggaa atggtgatac ttcctacaat 240
   cagaagttca aaggcaaggc cacattgact gcagacaaat cctccagcac agcctacatg 300
   cagctcagca gtctgacatc tgaggactct gcggtctatt actgtgcaag atcgcactac 360
   ggtagtaact acgtagacta ctttgactac tggggccaag gcaccactct cacagtctcc 420
   tctaagctta ccaagggccc atcggtcttc cccctggcac cctcctccaa gagcacctct 480
   gggggcacag cggccctggg ctgcctggtc aaggactact tccccgaacc ggtgacggtg 540
   tcgtggaact caggcgccct gaccagcggc gtgcacacct tcccggctgt cctacagtcc 600
   tcaggactct actccctcag cagcgtggtg accgtgccct ccagcagctt gggcacccag 660
   acctacatct gcaacgtgaa tcacaagccc agcaacacca aggtggacaa gaaggttgag 720
   cccaaatctt gtgacaaaac tcacacatgc ccaccgtgcc cagcacctga actcctgggg 780
   ggaccgtcag tcttcctctt ccccccaaaa cccaaggaca ccctcatgat ctcccggacc 840
   cctgaggtca catgcgtggt ggtggacgtg agccacgaag accctgaggt caagttcaac 900
   tggtacgtgg acggcgtgga ggtgcataat gccaagacaa agccgcggga ggagcagtac 960
   aacagcacgt accgtgtggt cagcgtcctc accgtcctgc accaggactg gctgaatggc 1020
   aaggagtaca agtgcaaggt ctccaacaaa gccctcccag cccccatcga gaaaaccatc 1080
   tccaaagcca aagggcagcc ccgagaacca caggtgtaca ccctgccccc atcccgggat 1140
   gagctgacca agaaccaggt cagcctgacc tgcctggtca aaggcttcta tcccagcgac 1200
   atcgccgtgg agtgggagag caatgggcag ccggagaaca actacaagac cacgcctccc 1260
   gtgctggact ccgacggctc cttcttcctc tacagcaagc tcaccgtgga caagagcagg 1320
   tggcagcagg ggaacgtctt ctcatgctcc gtgatgcatg aggctctgca caaccactac 1380
   acgcagaaga gcctctccct gtctccgggt aaaggatccg gtggcggtgg ctcgggcggt 1440
   ggtgggtcgg gtggcggcgg atctcccaaa tctagtgaca aaactcacac atgcccaccg 1500
   tgcccagcac ctgaactcct ggggggaccg tcagtcttcc tcttcccccc aaaacccaag 1560
   gacaccctca tgatctcccg gacccctgag gtcacatgcg tggtggtgga cgtgagccac 1620
   gaagaccctg aggtcaagtt caactggtac gtggacggcg tggaggtgca taatgccaag 1680
   acaaagccgc gggaggagca gtacaacagc acgtaccgtg tggtcagcgt cctcaccgtc 1740
   ctgcaccagg actggctgaa tggcaaggag tacaagtgca aggtctccaa caaagccctc 1800
   ccagccccca tcgagaaaac catctccaaa gccaaagggc agccccgaga accacaggtg 1860
   tacaccctgc ccccatcccg ggatgagctg accaagaacc aggtcagcct gacctgcctg 1920
   gtcaaaggct tctatcccag cgacatcgcc gtggagtggg agagcaatgg gcagccggag 1980
   aacaactaca agaccacgcc tcccgtgctg gactccgacg gctccttctt cctctacagc 2040
   aagctcaccg tggacaagag caggtggcag caggggaacg tcttctcatg ctccgtgatg 2100
   catgaggctc tgcacaacca ctacacgcag aagagcctct ccctgtctcc gggtaaatga 2160
<210> 12
   <211> 719
   <212> PRT
   <213> Artificial
<220>
   <223> An artificially synthesized peptide sequence
<400> 12
   Met Gly Trp Ser Cys Ile Ile Phe Phe Leu Val Ala Thr Ala Thr Gly
   1               5                   10                  15
   Val His Ser Gln Val Gln Leu Arg Gln Pro Gly Ala Glu Leu Val Lys
               20                  25                  30
   Pro Gly Ala Ser Val Lys Met Ser Cys Lys Ala Ser Gly Tyr Thr Phe
           35                  40                  45
   Thr Ser Tyr Asn Met His Trp Val Lys Gln Thr Pro Gly Gln Gly Leu
       50                  55                  60
   Glu Trp Ile Gly Ala Ile Tyr Pro Gly Asn Gly Asp Thr Ser Tyr Asn
   65                  70                  75                  80
   Gln Lys Phe Lys Gly Lys Ala Thr Leu Thr Ala Asp Lys Ser Ser Ser
                   85                  90                  95
   Thr Ala Tyr Met Gln Leu Ser Ser Leu Thr Ser Glu Asp Ser Ala Val
               100                 105                 110
   Tyr Tyr Cys Ala Arg Ser His Tyr Gly Ser Asn Tyr Val Asp Tyr Phe
           115                 120                 125
   Asp Tyr Trp Gly Gln Gly Thr Thr Leu Thr Val Ser Ser Lys Leu Thr
       130                 135                 140
   Lys Gly Pro Ser Val Phe Pro Leu Ala Pro Ser Ser Lys Ser Thr Ser
   145                 150                 155                 160
   Gly Gly Thr Ala Ala Leu Gly Cys Leu Val Lys Asp Tyr Phe Pro Glu
                   165                 170                 175
   Pro Val Thr Val Ser Trp Asn Ser Gly Ala Leu Thr Ser Gly Val His
               180                 185                 190
   Thr Phe Pro Ala Val Leu Gln Ser Ser Gly Leu Tyr Ser Leu Ser Ser
   195 200 205
   Val Val Thr Val Pro Ser Ser Ser Leu Gly Thr Gln Thr Tyr Ile Cys
   210 215 220
   Asn Val Asn His Lys Pro Ser Asn Thr Lys Val Asp Lys Lys Val Glu
   225 230 235 240
   Pro Lys Ser Cys Asp Lys Thr His Thr Cys Pro Pro Cys Pro Ala Pro
   245 250 255
   Glu Leu Leu Gly Gly Pro Ser Val Phe Leu Phe Pro Pro Lys Pro Lys
   260 265 270
   Asp Thr Leu Met Ile Ser Arg Thr Pro Glu Val Thr Cys Val Val Val
   275 280 285
   Asp Val Ser His Glu Asp Pro Glu Val Lys Phe Asn Trp Tyr Val Asp
   290 295 300
   Gly Val Glu Val His Asn Ala Lys Thr Lys Pro Arg Glu Glu Gln Tyr
   305 310 315 320
   Asn Ser Thr Tyr Arg Val Val Ser Val Leu Thr Val Leu His Gln Asp
   325 330 335
   Trp Leu Asn Gly Lys Glu Tyr Lys Cys Lys Val Ser Asn Lys Ala Leu
   340 345 350
   Pro Ala Pro Ile Glu Lys Thr Ile Ser Lys Ala Lys Gly Gln Pro Arg
   355 360 365
   Glu Pro Gln Val Tyr Thr Leu Pro Pro Ser Arg Asp Glu Leu Thr Lys
   370 375 380
   Asn Gln Val Ser Leu Thr Cys Leu Val Lys Gly Phe Tyr Pro Ser Asp
   385 390 395 400
   Ile Ala Val Glu Trp Glu Ser Asn Gly Gln Pro Glu Asn Asn Tyr Lys
   405 410 415
   Thr Thr Pro Pro Val Leu Asp Ser Asp Gly Ser Phe Phe Leu Tyr Ser
   420 425 430
   Lys Leu Thr Val Asp Lys Ser Arg Trp Gln Gln Gly Asn Val Phe Ser
   435 440 445
   Cys Ser Val Met His Glu Ala Leu His Asn His Tyr Thr Gln Lys Ser
   450 455 460
   Leu Ser Leu Ser Pro Gly Lys Gly Ser Gly Gly Gly Gly Ser Gly Gly
   465 470 475 480
   Gly Gly Ser Gly Gly Gly Gly Ser Pro Lys Ser Ser Asp Lys Thr His
   485 490 495
   Thr Cys Pro Pro Cys Pro Ala Pro Glu Leu Leu Gly Gly Pro Ser Val
   500 505 510
   Phe Leu Phe Pro Pro Lys Pro Lys Asp Thr Leu Met Ile Ser Arg Thr
   515 520 525
   Pro Glu Val Thr Cys Val Val Val Asp Val Ser His Glu Asp Pro Glu
   530 535 540
   Val Lys Phe Asn Trp Tyr Val Asp Gly Val Glu Val His Asn Ala Lys
   545 550 555 560
   Thr Lys Pro Arg Glu Glu Gln Tyr Asn Ser Thr Tyr Arg Val Val Ser
   565 570 575
   Val Leu Thr Val Leu His Gln Asp Trp Leu Asn Gly Lys Glu Tyr Lys
   580 585 590
   Cys Lys Val Ser Asn Lys Ala Leu Pro Ala Pro Ile Glu Lys Thr Ile
   595 600 605
   Ser Lys Ala Lys Gly Gln Pro Arg Glu Pro Gln Val Tyr Thr Leu Pro
   610 615 620
   Pro Ser Arg Asp Glu Leu Thr Lys Asn Gln Val Ser Leu Thr Cys Leu
   625 630 635 640
   Val Lys Gly Phe Tyr Pro Ser Asp Ile Ala Val Glu Trp Glu Ser Asn
   645 650 655
   Gly Gln Pro Glu Asn Asn Tyr Lys Thr Thr Pro Pro Val Leu Asp Ser
   660 665 670
   Asp Gly Ser Phe Phe Leu Tyr Ser Lys Leu Thr Val Asp Lys Ser Arg
   675 680 685
   Trp Gln Gln Gly Asn Val Phe Ser Cys Ser Val Met His Glu Ala Leu
   690 695 700
   His Asn His Tyr Thr Gln Lys Ser Leu Ser Leu Ser Pro Gly Lys
   705                 710                 715
<210> 13
   <211> 2814
   <212> DNA
   <213> Artificial
<220>
   <223> An artificially synthesized nucleotide sequence
<400> 13
   atgggatgga gctgtatcat cttctttttg gtagcaacag ctacaggtgt ccactcccag 60
   gtgcaactgc ggcagcctgg ggctgagctg gtgaagcctg gggcctcagt gaagatgtcc 120
   tgcaaggctt ctggctacac atttaccagt tacaatatgc actgggtaaa gcagacacct 180
   ggacagggcc tggaatggat tggagctatt tatccaggaa atggtgatac ttcctacaat 240
   cagaagttca aaggcaaggc cacattgact gcagacaaat cctccagcac agcctacatg 300
   cagctcagca gtctgacatc tgaggactct gcggtctatt actgtgcaag atcgcactac 360
   ggtagtaact acgtagacta ctttgactac tggggccaag gcaccactct cacagtctcc 420
   tctaagctta ccaagggccc atcggtcttc cccctggcac cctcctccaa gagcacctct 480
   gggggcacag cggccctggg ctgcctggtc aaggactact tccccgaacc ggtgacggtg 540
   tcgtggaact caggcgccct gaccagcggc gtgcacacct tcccggctgt cctacagtcc 600
   tcaggactct actccctcag cagcgtggtg accgtgccct ccagcagctt gggcacccag 660
   acctacatct gcaacgtgaa tcacaagccc agcaacacca aggtggacaa gaaggttgag 720
   cccaaatctt gtgacaaaac tcacacatgc ccaccgtgcc cagcacctga actcctgggg 780
   ggaccgtcag tcttcctctt ccccccaaaa cccaaggaca ccctcatgat ctcccggacc 840
   cctgaggtca catgcgtggt ggtggacgtg agccacgaag accctgaggt caagttcaac 900
   tggtacgtgg acggcgtgga ggtgcataat gccaagacaa agccgcggga ggagcagtac 960
   aacagcacgt accgtgtggt cagcgtcctc accgtcctgc accaggactg gctgaatggc 1020
   aaggagtaca agtgcaaggt ctccaacaaa gccctcccag cccccatcga gaaaaccatc 1080
   tccaaagcca aagggcagcc ccgagaacca caggtgtaca ccctgccccc atcccgggat 1140
   gagctgacca agaaccaggt cagcctgacc tgcctggtca aaggcttcta tcccagcgac 1200
   atcgccgtgg agtgggagag caatgggcag ccggagaaca actacaagac cacgcctccc 1260
   gtgctggact ccgacggctc cttcttcctc tacagcaagc tcaccgtgga caagagcagg 1320
   tggcagcagg ggaacgtctt ctcatgctcc gtgatgcatg aggctctgca caaccactac 1380
   acgcagaaga gcctctccct gtctccgggt aaaggatccc ccaaatctag tgacaaaact 1440
   cacacatgcc caccgtgccc agcacctgaa ctcctggggg gaccgtcagt cttcctcttc 1500
   cccccaaaac ccaaggacac cctcatgatc tcccggaccc ctgaggtcac atgcgtggtg   1560
   gtggacgtga gccacgaaga ccctgaggtc aagttcaact ggtacgtgga cggcgtggag   1620
   gtgcataatg ccaagacaaa gccgcgggag gagcagtaca acagcacgta ccgtgtggtc   1680
   agcgtcctca ccgtcctgca ccaggactgg ctgaatggca aggagtacaa gtgcaaggtc   1740
   tccaacaaag ccctcccagc ccccatcgag aaaaccatct ccaaagccaa agggcagccc   1800
   cgagaaccac aggtgtacac cctgccccca tcccgggatg agctgaccaa gaaccaggtc   1860
   agcctgacct gcctggtcaa aggcttctat cccagcgaca tcgccgtgga gtgggagagc   1920
   aatgggcagc cggagaacaa ctacaagacc acgcctcccg tgctggactc cgacggctcc   1980
   ttcttcctct acagcaagct caccgtggac aagagcaggt ggcagcaggg gaacgtcttc   2040
   tcatgctccg tgatgcatga ggctctgcac aaccactaca cgcagaagag cctctccctg   2100
   tctccgggta aatctagacc caaatctagt gacaaaactc acacatgccc accgtgccca   2160
   gcacctgaac tcctgggggg accgtcagtc ttcctcttcc ccccaaaacc caaggacacc   2220
   ctcatgatct cccggacccc tgaggtcaca tgcgtggtgg tggacgtgag ccacgaagac   2280
   cctgaggtca agttcaactg gtacgtggac ggcgtggagg tgcataatgc caagacaaag   2340
   ccgcgggagg agcagtacaa cagcacgtac cgtgtggtca gcgtcctcac cgtcctgcac   2400
   caggactggc tgaatggcaa ggagtacaag tgcaaggtct ccaacaaagc cctcccagcc   2460
   cccatcgaga aaaccatctc caaagccaaa gggcagcccc gagaaccaca ggtgtacacc   2520
   ctgcccccat cccgggatga gctgaccaag aaccaggtca gcctgacctg cctggtcaaa   2580
   ggcttctatc ccagcgacat cgccgtggag tgggagagca atgggcagcc ggagaacaac   2640
   tacaagacca cgcctcccgt gctggactcc gacggctcct tcttcctcta cagcaagctc   2700
   accgtggaca agagcaggtg gcagcagggg aacgtcttct catgctccgt gatgcatgag   2760
   gctctgcaca accactacac gcagaagagc ctctccctgt ctccgggtaa atga         2814
<210> 14
   <211> 937
   <212> PRT
   <213> Artificial
<220>
   <223> An artificially synthesized peptide sequence
<400> 14
<210> 15
   <211> 2844
   <212> DNA
   <213> Artificial
<220>
   <223> An artificially synthesized nucleotide sequence
<400> 15
   atgggatgga gctgtatcat cttctttttg gtagcaacag ctacaggtgt ccactcccag     60
   gtgcaactgc ggcagcctgg ggctgagctg gtgaagcctg gggcctcagt gaagatgtcc    120
   tgcaaggctt ctggctacac atttaccagt tacaatatgc actgggtaaa gcagacacct    180
   ggacagggcc tggaatggat tggagctatt tatccaggaa atggtgatac ttcctacaat    240
   cagaagttca aaggcaaggc cacattgact gcagacaaat cctccagcac agcctacatg    300
   cagctcagca gtctgacatc tgaggactct gcggtctatt actgtgcaag atcgcactac    360
   ggtagtaact acgtagacta ctttgactac tggggccaag gcaccactct cacagtctcc    420
   tctaagctta ccaagggccc atcggtcttc cccctggcac cctcctccaa gagcacctct    480
   gggggcacag cggccctggg ctgcctggtc aaggactact tccccgaacc ggtgacggtg    540
   tcgtggaact caggcgccct gaccagcggc gtgcacacct tcccggctgt cctacagtcc    600
   tcaggactct actccctcag cagcgtggtg accgtgccct ccagcagctt gggcacccag    660
   acctacatct gcaacgtgaa tcacaagccc agcaacacca aggtggacaa gaaggttgag    720
   cccaaatctt gtgacaaaac tcacacatgc ccaccgtgcc cagcacctga actcctgggg    780
   ggaccgtcag tcttcctctt ccccccaaaa cccaaggaca ccctcatgat ctcccggacc    840
   cctgaggtca catgcgtggt ggtggacgtg agccacgaag accctgaggt caagttcaac    900
   tggtacgtgg acggcgtgga ggtgcataat gccaagacaa agccgcggga ggagcagtac    960
   aacagcacgt accgtgtggt cagcgtcctc accgtcctgc accaggactg gctgaatggc   1020
   aaggagtaca agtgcaaggt ctccaacaaa gccctcccag cccccatcga gaaaaccatc   1080
   tccaaagcca aagggcagcc ccgagaacca caggtgtaca ccctgccccc atcccgggat   1140
   gagctgacca agaaccaggt cagcctgacc tgcctggtca aaggcttcta tcccagcgac   1200
   atcgccgtgg agtgggagag caatgggcag ccggagaaca actacaagac cacgcctccc   1260
   gtgctggact ccgacggctc cttcttcctc tacagcaagc tcaccgtgga caagagcagg   1320
   tggcagcagg ggaacgtctt ctcatgctcc gtgatgcatg aggctctgca caaccactac   1380
   acgcagaaga gcctctccct gtctccgggt aaaggatccg gtggcggtgg ctcgcccaaa   1440
   tctagtgaca aaactcacac atgcccaccg tgcccagcac ctgaactcct ggggggaccg   1500
   tcagtcttcc tcttcccccc aaaacccaag gacaccctca tgatctcccg gacccctgag   1560
   gtcacatgcg tggtggtgga cgtgagccac gaagaccctg aggtcaagtt caactggtac   1620
   gtggacggcg tggaggtgca taatgccaag acaaagccgc gggaggagca gtacaacagc   1680
   acgtaccgtg tggtcagcgt cctcaccgtc ctgcaccagg actggctgaa tggcaaggag   1740
   tacaagtgca aggtctccaa caaagccctc ccagccccca tcgagaaaac catctccaaa   1800
   gccaaagggc agccccgaga accacaggtg tacaccctgc ccccatcccg ggatgagctg   1860
   accaagaacc aggtcagcct gacctgcctg gtcaaaggct tctatcccag cgacatcgcc   1920
   gtggagtggg agagcaatgg gcagccggag aacaactaca agaccacgcc tcccgtgctg   1980
   gactccgacg gctccttctt cctctacagc aagctcaccg tggacaagag caggtggcag   2040
   caggggaacg tcttctcatg ctccgtgatg catgaggctc tgcacaacca ctacacgcag   2100
   aagagcctct ccctgtctcc gggtaaatct agaggtggcg gtggctcgcc caaatctagt   2160
   gacaaaactc acacatgccc accgtgccca gcacctgaac tcctgggggg accgtcagtc   2220
   ttcctcttcc ccccaaaacc caaggacacc ctcatgatct cccggacccc tgaggtcaca   2280
   tgcgtggtgg tggacgtgag ccacgaagac cctgaggtca agttcaactg gtacgtggac   2340
   ggcgtggagg tgcataatgc caagacaaag ccgcgggagg agcagtacaa cagcacgtac   2400
   cgtgtggtca gcgtcctcac cgtcctgcac caggactggc tgaatggcaa ggagtacaag   2460
   tgcaaggtct ccaacaaagc cctcccagcc cccatcgaga aaaccatctc caaagccaaa   2520
   gggcagcccc gagaaccaca ggtgtacacc ctgcccccat cccgggatga gctgaccaag   2580
   aaccaggtca gcctgacctg cctggtcaaa ggcttctatc ccagcgacat cgccgtggag   2640
   tgggagagca atgggcagcc ggagaacaac tacaagacca cgcctcccgt gctggactcc   2700
   gacggctcct tcttcctcta cagcaagctc accgtggaca agagcaggtg gcagcagggg   2760
   aacgtcttct catgctccgt gatgcatgag gctctgcaca accactacac gcagaagagc   2820
   ctctccctgt ctccgggtaa atga                                          2844
<210> 16
   <211> 947
   <212> PRT
   <213> Artificial
<220>
   <223> An artificially synthesized peptide sequence
<400> 16
   Met Gly Trp Ser Cys Ile Ile Phe Phe Leu Val Ala Thr Ala Thr Gly
   1               5                   10                  15
   Val His Ser Gln Val Gln Leu Arg Gln Pro Gly Ala Glu Leu Val Lys
               20                  25                  30
   Pro Gly Ala Ser Val Lys Met Ser Cys Lys Ala Ser Gly Tyr Thr Phe
           35                  40                  45
   Thr Ser Tyr Asn Met His Trp Val Lys Gln Thr Pro Gly Gln Gly Leu
       50                  55                  60
   Glu Trp Ile Gly Ala Ile Tyr Pro Gly Asn Gly Asp Thr Ser Tyr Asn
   65                  70                  75                  80
   Gln Lys Phe Lys Gly Lys Ala Thr Leu Thr Ala Asp Lys Ser Ser Ser
                   85                  90                  95
   Thr Ala Tyr Met Gln Leu Ser Ser Leu Thr Ser Glu Asp Ser Ala Val
               100                 105                 110
   Tyr Tyr Cys Ala Arg Ser His Tyr Gly Ser Asn Tyr Val Asp Tyr Phe
           115                 120                 125
   Asp Tyr Trp Gly Gln Gly Thr Thr Leu Thr Val Ser Ser Lys Leu Thr
   130 135 140
   Lys Gly Pro Ser Val Phe Pro Leu Ala Pro Ser Ser Lys Ser Thr Ser
   145 150 155 160
   Gly Gly Thr Ala Ala Leu Gly Cys Leu Val Lys Asp Tyr Phe Pro Glu
   165 170 175
   Pro Val Thr Val Ser Trp Asn Ser Gly Ala Leu Thr Ser Gly Val His
   180 185 190
   Thr Phe Pro Ala Val Leu Gln Ser Ser Gly Leu Tyr Ser Leu Ser Ser
   195 200 205
   Val Val Thr Val Pro Ser Ser Ser Leu Gly Thr Gln Thr Tyr Ile Cys
   210 215 220
   Asn Val Asn His Lys Pro Ser Asn Thr Lys Val Asp Lys Lys Val Glu
   225 230 235 240
   Pro Lys Ser Cys Asp Lys Thr His Thr Cys Pro Pro Cys Pro Ala Pro
   245 250 255
   Glu Leu Leu Gly Gly Pro Ser Val Phe Leu Phe Pro Pro Lys Pro Lys
   260 265 270
   Asp Thr Leu Met Ile Ser Arg Thr Pro Glu Val Thr Cys Val Val Val
   275 280 285
   Asp Val Ser His Glu Asp Pro Glu Val Lys Phe Asn Trp Tyr Val Asp
   290 295 300
   Gly Val Glu Val His Asn Ala Lys Thr Lys Pro Arg Glu Glu Gln Tyr
   305 310 315 320
   Asn Ser Thr Tyr Arg Val Val Ser Val Leu Thr Val Leu His Gln Asp
   325 330 335
   Trp Leu Asn Gly Lys Glu Tyr Lys Cys Lys Val Ser Asn Lys Ala Leu
   340 345 350
   Pro Ala Pro Ile Glu Lys Thr Ile Ser Lys Ala Lys Gly Gln Pro Arg
   355 360 365
   Glu Pro Gln Val Tyr Thr Leu Pro Pro Ser Arg Asp Glu Leu Thr Lys
   370 375 380
   Asn Gln Val Ser Leu Thr Cys Leu Val Lys Gly Phe Tyr Pro Ser Asp
   385                 390                 395                 400
   Ile Ala Val Glu Trp Glu Ser Asn Gly Gln Pro Glu Asn Asn Tyr Lys
                   405                 410                 415
   Thr Thr Pro Pro Val Leu Asp Ser Asp Gly Ser Phe Phe Leu Tyr Ser
               420                 425                 430
   Lys Leu Thr Val Asp Lys Ser Arg Trp Gln Gln Gly Asn Val Phe Ser
           435                 440                 445
   Cys Ser Val Met His Glu Ala Leu His Asn His Tyr Thr Gln Lys Ser
       450                 455                 460
   Leu Ser Leu Ser Pro Gly Lys Gly Ser Gly Gly Gly Gly Ser Pro Lys
   465                 470                 475                 480
   Ser Ser Asp Lys Thr His Thr Cys Pro Pro Cys Pro Ala Pro Glu Leu
                   485                 490                 495
   Leu Gly Gly Pro Ser Val Phe Leu Phe Pro Pro Lys Pro Lys Asp Thr
               500                 505                 510
   Leu Met Ile Ser Arg Thr Pro Glu Val Thr Cys Val Val Val Asp Val
           515                 520                 525
   Ser His Glu Asp Pro Glu Val Lys Phe Asn Trp Tyr Val Asp Gly Val
       530                 535                 540
   Glu Val His Asn Ala Lys Thr Lys Pro Arg Glu Glu Gln Tyr Asn Ser
   545                 550                 555                 560
   Thr Tyr Arg Val Val Ser Val Leu Thr Val Leu His Gln Asp Trp Leu
                   565                 570                 575
   Asn Gly Lys Glu Tyr Lys Cys Lys Val Ser Asn Lys Ala Leu Pro Ala
               580                 585                 590
   Pro Ile Glu Lys Thr Ile Ser Lys Ala Lys Gly Gln Pro Arg Glu Pro
           595                 600                 605
   Gln Val Tyr Thr Leu Pro Pro Ser Arg Asp Glu Leu Thr Lys Asn Gln
       610                 615                 620
   Val Ser Leu Thr Cys Leu Val Lys Gly Phe Tyr Pro Ser Asp Ile Ala
   625                 630                 635                 640
   Val Glu Trp Glu Ser Asn Gly Gln Pro Glu Asn Asn Tyr Lys Thr Thr
   645 650 655
   Pro Pro Val Leu Asp Ser Asp Gly Ser Phe Phe Leu Tyr Ser Lys Leu
   660 665 670
   Thr Val Asp Lys Ser Arg Trp Gln Gln Gly Asn Val Phe Ser Cys Ser
   675 680 685
   Val Met His Glu Ala Leu His Asn His Tyr Thr Gln Lys Ser Leu Ser
   690 695 700
   Leu Ser Pro Gly Lys Ser Arg Gly Gly Gly Gly Ser Pro Lys Ser Ser
   705 710 715 720
   Asp Lys Thr His Thr Cys Pro Pro Cys Pro Ala Pro Glu Leu Leu Gly
   725 730 735
   Gly Pro Ser Val Phe Leu Phe Pro Pro Lys Pro Lys Asp Thr Leu Met
   740 745 750
   Ile Ser Arg Thr Pro Glu Val Thr Cys Val Val Val Asp Val Ser His
   755 760 765
   Glu Asp Pro Glu Val Lys Phe Asn Trp Tyr Val Asp Gly Val Glu Val
   770 775 780
   His Asn Ala Lys Thr Lys Pro Arg Glu Glu Gln Tyr Asn Ser Thr Tyr
   785 790 795 800
   Arg Val Val Ser Val Leu Thr Val Leu His Gln Asp Trp Leu Asn Gly
   805 810 815
   Lys Glu Tyr Lys Cys Lys Val Ser Asn Lys Ala Leu Pro Ala Pro Ile
   820 825 830
   Glu Lys Thr Ile Ser Lys Ala Lys Gly Gln Pro Arg Glu Pro Gln Val
   835 840 845
   Tyr Thr Leu Pro Pro Ser Arg Asp Glu Leu Thr Lys Asn Gln Val Ser
   850 855 860
   Leu Thr Cys Leu Val Lys Gly Phe Tyr Pro Ser Asp Ile Ala Val Glu
   865 870 875 880
   Trp Glu Ser Asn Gly Gln Pro Glu Asn Asn Tyr Lys Thr Thr Pro Pro
   885 890 895
   Val Leu Asp Ser Asp Gly Ser Phe Phe Leu Tyr Ser Lys Leu Thr Val
   900 905 910
   Asp Lys Ser Arg Trp Gln Gln Gly Asn Val Phe Ser Cys Ser Val Met
   915 920 925
   His Glu Ala Leu His Asn His Tyr Thr Gln Lys Ser Leu Ser Leu Ser
   930 935 940
   Pro Gly Lys
   945
<210> 17
   <211> 2874
   <212> DNA
   <213> Artificial
<220>
   <223> An artificially synthesized nucleotide sequence
<400> 17
   atgggatgga gctgtatcat cttctttttg gtagcaacag ctacaggtgt ccactcccag 60
   gtgcaactgc ggcagcctgg ggctgagctg gtgaagcctg gggcctcagt gaagatgtcc 120
   tgcaaggctt ctggctacac atttaccagt tacaatatgc actgggtaaa gcagacacct 180
   ggacagggcc tggaatggat tggagctatt tatccaggaa atggtgatac ttcctacaat 240
   cagaagttca aaggcaaggc cacattgact gcagacaaat cctccagcac agcctacatg 300
   cagctcagca gtctgacatc tgaggactct gcggtctatt actgtgcaag atcgcactac 360
   ggtagtaact acgtagacta ctttgactac tggggccaag gcaccactct cacagtctcc 420
   tctaagctta ccaagggccc atcggtcttc cccctggcac cctcctccaa gagcacctct 480
   gggggcacag cggccctggg ctgcctggtc aaggactact tccccgaacc ggtgacggtg 540
   tcgtggaact caggcgccct gaccagcggc gtgcacacct tcccggctgt cctacagtcc 600
   tcaggactct actccctcag cagcgtggtg accgtgccct ccagcagctt gggcacccag 660
   acctacatct gcaacgtgaa tcacaagccc agcaacacca aggtggacaa gaaggttgag 720
   cccaaatctt gtgacaaaac tcacacatgc ccaccgtgcc cagcacctga actcctgggg 780
   ggaccgtcag tcttcctctt ccccccaaaa cccaaggaca ccctcatgat ctcccggacc 840
   cctgaggtca catgcgtggt ggtggacgtg agccacgaag accctgaggt caagttcaac 900
   tggtacgtgg acggcgtgga ggtgcataat gccaagacaa agccgcggga ggagcagtac 960
   aacagcacgt accgtgtggt cagcgtcctc accgtcctgc accaggactg gctgaatggc 1020
   aaggagtaca agtgcaaggt ctccaacaaa gccctcccag cccccatcga gaaaaccatc 1080
   tccaaagcca aagggcagcc ccgagaacca caggtgtaca ccctgccccc atcccgggat 1140
   gagctgacca agaaccaggt cagcctgacc tgcctggtca aaggcttcta tcccagcgac 1200
   atcgccgtgg agtgggagag caatgggcag ccggagaaca actacaagac cacgcctccc 1260
   gtgctggact ccgacggctc cttcttcctc tacagcaagc tcaccgtgga caagagcagg 1320
   tggcagcagg ggaacgtctt ctcatgctcc gtgatgcatg aggctctgca caaccactac 1380
   acgcagaaga gcctctccct gtctccgggt aaaggatccg gtggcggtgg ctcgggcggt 1440
   ggtgggtcgc ccaaatctag tgacaaaact cacacatgcc caccgtgccc agcacctgaa 1500
   ctcctggggg gaccgtcagt cttcctcttc cccccaaaac ccaaggacac cctcatgatc 1560
   tcccggaccc ctgaggtcac atgcgtggtg gtggacgtga gccacgaaga ccctgaggtc 1620
   aagttcaact ggtacgtgga cggcgtggag gtgcataatg ccaagacaaa gccgcgggag 1680
   gagcagtaca acagcacgta ccgtgtggtc agcgtcctca ccgtcctgca ccaggactgg 1740
   ctgaatggca aggagtacaa gtgcaaggtc tccaacaaag ccctcccagc ccccatcgag 1800
   aaaaccatct ccaaagccaa agggcagccc cgagaaccac aggtgtacac cctgccccca 1860
   tcccgggatg agctgaccaa gaaccaggtc agcctgacct gcctggtcaa aggcttctat 1920
   cccagcgaca tcgccgtgga gtgggagagc aatgggcagc cggagaacaa ctacaagacc 1980
   acgcctcccg tgctggactc cgacggctcc ttcttcctct acagcaagct caccgtggac 2040
   aagagcaggt ggcagcaggg gaacgtcttc tcatgctccg tgatgcatga ggctctgcac 2100
   aaccactaca cgcagaagag cctctccctg tctccgggta aatctagagg tggcggtggc 2160
   tcgggcggtg gtgggtcgcc caaatctagt gacaaaactc acacatgccc accgtgccca 2220
   gcacctgaac tcctgggggg accgtcagtc ttcctcttcc ccccaaaacc caaggacacc 2280
   ctcatgatct cccggacccc tgaggtcaca tgcgtggtgg tggacgtgag ccacgaagac 2340
   cctgaggtca agttcaactg gtacgtggac ggcgtggagg tgcataatgc caagacaaag 2400
   ccgcgggagg agcagtacaa cagcacgtac cgtgtggtca gcgtcctcac cgtcctgcac 2460
   caggactggc tgaatggcaa ggagtacaag tgcaaggtct ccaacaaagc cctcccagcc 2520
   cccatcgaga aaaccatctc caaagccaaa gggcagcccc gagaaccaca ggtgtacacc 2580
   ctgcccccat cccgggatga gctgaccaag aaccaggtca gcctgacctg cctggtcaaa 2640
   ggcttctatc ccagcgacat cgccgtggag tgggagagca atgggcagcc ggagaacaac 2700
   tacaagacca cgcctcccgt gctggactcc gacggctcct tcttcctcta cagcaagctc 2760
   accgtggaca agagcaggtg gcagcagggg aacgtcttct catgctccgt gatgcatgag 2820
   gctctgcaca accactacac gcagaagagc ctctccctgt ctccgggtaa atga 2874
<210> 18
   <211> 957
   <212> PRT
   <213> Artificial
<220>
   <223> An artificially synthesized peptide sequence
<400> 18
   Met Gly Trp Ser Cys Ile Ile Phe Phe Leu Val Ala Thr Ala Thr Gly
   1               5                   10                  15
   Val His Ser Gln Val Gln Leu Arg Gln Pro Gly Ala Glu Leu Val Lys
               20                  25                  30
   Pro Gly Ala Ser Val Lys Met Ser Cys Lys Ala Ser Gly Tyr Thr Phe
           35                  40                  45
   Thr Ser Tyr Asn Met His Trp Val Lys Gln Thr Pro Gly Gln Gly Leu
       50                  55                  60
   Glu Trp Ile Gly Ala Ile Tyr Pro Gly Asn Gly Asp Thr Ser Tyr Asn
   65                  70                  75                  80
   Gln Lys Phe Lys Gly Lys Ala Thr Leu Thr Ala Asp Lys Ser Ser Ser
                   85                  90                  95
   Thr Ala Tyr Met Gln Leu Ser Ser Leu Thr Ser Glu Asp Ser Ala Val
               100                 105                 110
   Tyr Tyr Cys Ala Arg Ser His Tyr Gly Ser Asn Tyr Val Asp Tyr Phe
           115                 120                 125
   Asp Tyr Trp Gly Gln Gly Thr Thr Leu Thr Val Ser Ser Lys Leu Thr
       130                 135                 140
   Lys Gly Pro Ser Val Phe Pro Leu Ala Pro Ser Ser Lys Ser Thr Ser
   145                 150                 155                 160
   Gly Gly Thr Ala Ala Leu Gly Cys Leu Val Lys Asp Tyr Phe Pro Glu
                   165                 170                 175
   Pro Val Thr Val Ser Trp Asn Ser Gly Ala Leu Thr Ser Gly Val His
               180                 185                 190
   Thr Phe Pro Ala Val Leu Gln Ser Ser Gly Leu Tyr Ser Leu Ser Ser
           195                 200                 205
   Val Val Thr Val Pro Ser Ser Ser Leu Gly Thr Gln Thr Tyr Ile Cys
       210                 215                 220
   Asn Val Asn His Lys Pro Ser Asn Thr Lys Val Asp Lys Lys Val Glu
   225 230 235 240
   Pro Lys Ser Cys Asp Lys Thr His Thr Cys Pro Pro Cys Pro Ala Pro
   245 250 255
   Glu Leu Leu Gly Gly Pro Ser Val Phe Leu Phe Pro Pro Lys Pro Lys
   260 265 270
   Asp Thr Leu Met Ile Ser Arg Thr Pro Glu Val Thr Cys Val Val Val
   275 280 285
   Asp Val Ser His Glu Asp Pro Glu Val Lys Phe Asn Trp Tyr Val Asp
   290 295 300
   Gly Val Glu Val His Asn Ala Lys Thr Lys Pro Arg Glu Glu Gln Tyr
   305 310 315 320
   Asn Ser Thr Tyr Arg Val Val Ser Val Leu Thr Val Leu His Gln Asp
   325 330 335
   Trp Leu Asn Gly Lys Glu Tyr Lys Cys Lys Val Ser Asn Lys Ala Leu
   340 345 350
   Pro Ala Pro Ile Glu Lys Thr Ile Ser Lys Ala Lys Gly Gln Pro Arg
   355 360 365
   Glu Pro Gln Val Tyr Thr Leu Pro Pro Ser Arg Asp Glu Leu Thr Lys
   370 375 380
   Asn Gln Val Ser Leu Thr Cys Leu Val Lys Gly Phe Tyr Pro Ser Asp
   385 390 395 400
   Ile Ala Val Glu Trp Glu Ser Asn Gly Gln Pro Glu Asn Asn Tyr Lys
   405 410 415
   Thr Thr Pro Pro Val Leu Asp Ser Asp Gly Ser Phe Phe Leu Tyr Ser
   420 425 430
   Lys Leu Thr Val Asp Lys Ser Arg Trp Gln Gln Gly Asn Val Phe Ser
   435 440 445
   Cys Ser Val Met His Glu Ala Leu His Asn His Tyr Thr Gln Lys Ser
   450 455 460
   Leu Ser Leu Ser Pro Gly Lys Gly Ser Gly Gly Gly Gly Ser Gly Gly
   465 470 475 480
   Gly Gly Ser Pro Lys Ser Ser Asp Lys Thr His Thr Cys Pro Pro Cys
   485 490 495
   Pro Ala Pro Glu Leu Leu Gly Gly Pro Ser Val Phe Leu Phe Pro Pro
   500 505 510
   Lys Pro Lys Asp Thr Leu Met Ile Ser Arg Thr Pro Glu Val Thr Cys
   515 520 525
   Val Val Val Asp Val Ser His Glu Asp Pro Glu Val Lys Phe Asn Trp
   530 535 540
   Tyr Val Asp Gly Val Glu Val His Asn Ala Lys Thr Lys Pro Arg Glu
   545 550 555 560
   Glu Gln Tyr Asn Ser Thr Tyr Arg Val Val Ser Val Leu Thr Val Leu
   565 570 575
   His Gln Asp Trp Leu Asn Gly Lys Glu Tyr Lys Cys Lys Val Ser Asn
   580 585 590
   Lys Ala Leu Pro Ala Pro Ile Glu Lys Thr Ile Ser Lys Ala Lys Gly
   595 600 605
   Gln Pro Arg Glu Pro Gln Val Tyr Thr Leu Pro Pro Ser Arg Asp Glu
   610 615 620
   Leu Thr Lys Asn Gln Val Ser Leu Thr Cys Leu Val Lys Gly Phe Tyr
   625 630 635 640
   Pro Ser Asp Ile Ala Val Glu Trp Glu Ser Asn Gly Gln Pro Glu Asn
   645 650 655
   Asn Tyr Lys Thr Thr Pro Pro Val Leu Asp Ser Asp Gly Ser Phe Phe
   660 665 670
   Leu Tyr Ser Lys Leu Thr Val Asp Lys Ser Arg Trp Gln Gln Gly Asn
   675 680 685
   Val Phe Ser Cys Ser Val Met His Glu Ala Leu His Asn His Tyr Thr
   690 695 700
   Gln Lys Ser Leu Ser Leu Ser Pro Gly Lys Ser Arg Gly Gly Gly Gly
   705 710 715 720
   Ser Gly Gly Gly Gly Ser Pro Lys Ser Ser Asp Lys Thr His Thr Cys
                           725                 730                 735
   Pro Pro Cys Pro Ala Pro Glu Leu Leu Gly Gly Pro Ser Val Phe Leu
                   740                 745                 750
   Phe Pro Pro Lys Pro Lys Asp Thr Leu Met Ile Ser Arg Thr Pro Glu
               755                 760                 765
   Val Thr Cys Val Val Val Asp Val Ser His Glu Asp Pro Glu Val Lys
           770                 775                 780
   Phe Asn Trp Tyr Val Asp Gly Val Glu Val His Asn Ala Lys Thr Lys
   785                 790                 795                 800
   Pro Arg Glu Glu Gln Tyr Asn Ser Thr Tyr Arg Val Val Ser Val Leu
                   805                 810                 815
   Thr Val Leu His Gln Asp Trp Leu Asn Gly Lys Glu Tyr Lys Cys Lys
               820                 825                 830
   Val Ser Asn Lys Ala Leu Pro Ala Pro Ile Glu Lys Thr Ile Ser Lys
           835                 840                 845
   Ala Lys Gly Gln Pro Arg Glu Pro Gln Val Tyr Thr Leu Pro Pro Ser
       850                 855                 860
   Arg Asp Glu Leu Thr Lys Asn Gln Val Ser Leu Thr Cys Leu Val Lys
   865                 870                 875                 880
   Gly Phe Tyr Pro Ser Asp Ile Ala Val Glu Trp Glu Ser Asn Gly Gln
                   885                 890                 895
   Pro Glu Asn Asn Tyr Lys Thr Thr Pro Pro Val Leu Asp Ser Asp Gly
               900                 905                 910
   Ser Phe Phe Leu Tyr Ser Lys Leu Thr Val Asp Lys Ser Arg Trp Gln
           915                 920                 925
   Gln Gly Asn Val Phe Ser Cys Ser Val Met His Glu Ala Leu His Asn
       930                 935                 940
   His Tyr Thr Gln Lys Ser Leu Ser Leu Ser Pro Gly Lys
   945                 950                 955
<210> 19
   <211> 2904
   <212> DNA
   <213> Artificial
<220>
   <223> An artificially synthesized nucleotide sequence
<400> 19
   atgggatgga gctgtatcat cttctttttg gtagcaacag ctacaggtgt ccactcccag 60
   gtgcaactgc ggcagcctgg ggctgagctg gtgaagcctg gggcctcagt gaagatgtcc 120
   tgcaaggctt ctggctacac atttaccagt tacaatatgc actgggtaaa gcagacacct 180
   ggacagggcc tggaatggat tggagctatt tatccaggaa atggtgatac ttcctacaat 240
   cagaagttca aaggcaaggc cacattgact gcagacaaat cctccagcac agcctacatg 300
   cagctcagca gtctgacatc tgaggactct gcggtctatt actgtgcaag atcgcactac 360
   ggtagtaact acgtagacta ctttgactac tggggccaag gcaccactct cacagtctcc 420
   tctaagctta ccaagggccc atcggtcttc cccctggcac cctcctccaa gagcacctct 480
   gggggcacag cggccctggg ctgcctggtc aaggactact tccccgaacc ggtgacggtg 540
   tcgtggaact caggcgccct gaccagcggc gtgcacacct tcccggctgt cctacagtcc 600
   tcaggactct actccctcag cagcgtggtg accgtgccct ccagcagctt gggcacccag 660
   acctacatct gcaacgtgaa tcacaagccc agcaacacca aggtggacaa gaaggttgag 720
   cccaaatctt gtgacaaaac tcacacatgc ccaccgtgcc cagcacctga actcctgggg 780
   ggaccgtcag tcttcctctt ccccccaaaa cccaaggaca ccctcatgat ctcccggacc 840
   cctgaggtca catgcgtggt ggtggacgtg agccacgaag accctgaggt caagttcaac 900
   tggtacgtgg acggcgtgga ggtgcataat gccaagacaa agccgcggga ggagcagtac 960
   aacagcacgt accgtgtggt cagcgtcctc accgtcctgc accaggactg gctgaatggc 1020
   aaggagtaca agtgcaaggt ctccaacaaa gccctcccag cccccatcga gaaaaccatc 1080
   tccaaagcca aagggcagcc ccgagaacca caggtgtaca ccctgccccc atcccgggat 1140
   gagctgacca agaaccaggt cagcctgacc tgcctggtca aaggcttcta tcccagcgac 1200
   atcgccgtgg agtgggagag caatgggcag ccggagaaca actacaagac cacgcctccc 1260
   gtgctggact ccgacggctc cttcttcctc tacagcaagc tcaccgtgga caagagcagg 1320
   tggcagcagg ggaacgtctt ctcatgctcc gtgatgcatg aggctctgca caaccactac 1380
   acgcagaaga gcctctccct gtctccgggt aaaggatccg gtggcggtgg ctcgggcggt 1440
   ggtgggtcgg gtggcggcgg atctcccaaa tctagtgaca aaactcacac atgcccaccg 1500
   tgcccagcac ctgaactcct ggggggaccg tcagtcttcc tcttcccccc aaaacccaag 1560
   gacaccctca tgatctcccg gacccctgag gtcacatgcg tggtggtgga cgtgagccac 1620
   gaagaccctg aggtcaagtt caactggtac gtggacggcg tggaggtgca taatgccaag 1680
   acaaagccgc gggaggagca gtacaacagc acgtaccgtg tggtcagcgt cctcaccgtc 1740
   ctgcaccagg actggctgaa tggcaaggag tacaagtgca aggtctccaa caaagccctc   1800
   ccagccccca tcgagaaaac catctccaaa gccaaagggc agccccgaga accacaggtg   1860
   tacaccctgc ccccatcccg ggatgagctg accaagaacc aggtcagcct gacctgcctg   1920
   gtcaaaggct tctatcccag cgacatcgcc gtggagtggg agagcaatgg gcagccggag   1980
   aacaactaca agaccacgcc tcccgtgctg gactccgacg gctccttctt cctctacagc   2040
   aagctcaccg tggacaagag caggtggcag caggggaacg tcttctcatg ctccgtgatg   2100
   catgaggctc tgcacaacca ctacacgcag aagagcctct ccctgtctcc gggtaaatct   2160
   agaggtggcg gtggctcggg cggtggtggg tcgggtggcg gcggatctcc caaatctagt   2220
   gacaaaactc acacatgccc accgtgccca gcacctgaac tcctgggggg accgtcagtc   2280
   ttcctcttcc ccccaaaacc caaggacacc ctcatgatct cccggacccc tgaggtcaca   2340
   tgcgtggtgg tggacgtgag ccacgaagac cctgaggtca agttcaactg gtacgtggac   2400
   ggcgtggagg tgcataatgc caagacaaag ccgcgggagg agcagtacaa cagcacgtac   2460
   cgtgtggtca gcgtcctcac cgtcctgcac caggactggc tgaatggcaa ggagtacaag   2520
   tgcaaggtct ccaacaaagc cctcccagcc cccatcgaga aaaccatctc caaagccaaa   2580
   gggcagcccc gagaaccaca ggtgtacacc ctgcccccat cccgggatga gctgaccaag   2640
   aaccaggtca gcctgacctg cctggtcaaa ggcttctatc ccagcgacat cgccgtggag   2700
   tgggagagca atgggcagcc ggagaacaac tacaagacca cgcctcccgt gctggactcc   2760
   gacggctcct tcttcctcta cagcaagctc accgtggaca agagcaggtg gcagcagggg   2820
   aacgtcttct catgctccgt gatgcatgag gctctgcaca accactacac gcagaagagc   2880
   ctctccctgt ctccgggtaa atga                                          2904
<210> 20
   <211> 967
   <212> PRT
   <213> Artificial
<220>
   <223> An artificially synthesized peptide sequence
<400> 20
<210> 21
   <211> 35
   <212> DNA
   <213> Artificial
<220>
   <223> An artificially synthesized primer sequence
<400> 21
   cgagctcgag cggccgcttt tttttttttt ttttt 35
<210> 22
   <211> 35
   <212> DNA
   <213> Artificial
<220>
   <223> An artificially synthesized primer sequence
<400> 22
   tcgtctaggc tagcattgtt ctctcccagt ctcca 35
<210> 23
   <211> 35
   <212> DNA
   <213> Artificial
<220>
   <223> An artificially synthesized primer sequence
<400> 23
   gcttgagact cgagcagctt ggtcccagca ccgaa 35
<210> 24
   <211> 111
   <212> DNA
   <213> Artificial
<220>
   <223> An artificially synthesized primer sequence
<400> 24
   gagtttgcta gcgccgccat ggattttcaa gtgcagattt tcagcttcct gctaatcagt 60
   gcttcagtca taatgtccag aggacaaatt gttctctccc agtctccagc a 111
<210> 25
   <211> 35
   <212> DNA
   <213> Artificial
<220>
   <223> An artificially synthesized primer sequence
<400> 25
   gcttgagact cgagcagctt ggtcccagca ccgaa 35
<210> 26
   <211> 34
   <212> DNA
   <213> Artificial
<220>
   <223> An artificially synthesized primer sequence
<400> 26
   acctctaact cgagactgtg gctgcaccat ctgt 34
<210> 27
   <211> 30
   <212> DNA
   <213> Artificial
<220>
   <223> An artificially synthesized primer sequence
<400> 27
   acttgaattc ctaacactct cccctgttga 30
<210> 28
   <211> 35
   <212> DNA
   <213> Artificial
<220>
   <223> An artificially synthesized oligonucleotide sequence
<400> 28
   gtcgacgcta gcaaggatcc ttgaattcct taagg 35
<210> 29
   <211> 35
   <212> DNA
   <213> Artificial
<220>
   <223> An artificially synthesized oligonucleotide sequence
<400> 29
   gtcgacctta aggaattcaa ggatccttgc tagcg 35
<210> 30
   <211> 35
   <212> DNA
   <213> Artificial
<220>
   <223> An artificially synthesized primer sequence
<400> 30
   cacgcgtcga cgccgccatg gcccaggtgc aactg 35
<210> 31
   <211> 35
   <212> DNA
   <213> Artificial
<220>
   <223> An artificially synthesized primer sequence
<400> 31
   gcggccaagc ttagaggaga ctgtgagagt ggtgc 35
<210> 32
   <211> 98
   <212> DNA
   <213> Artificial
<220>
   <223> An artificially synthesized primer sequence
<400> 32
   cacgcgtcga cgccgccatg ggatggagct gtatcatctt ctttttggta gcaacagcta 60
   caggtgtcca ctcccaggtg caactgcggc agcctggg 98
<210> 33
   <211> 35
   <212> DNA
   <213> Artificial
<220>
   <223> An artificially synthesized primer sequence
<400> 33
   gcggccaagc ttagaggaga ctgtgagagt ggtgc 35
<210> 34
   <211> 35
   <212> DNA
   <213> Artificial
<220>
   <223> An artificially synthesized primer sequence
<400> 34
   tctcctcaaa gcttaccaag ggcccatcgg tcttc 35
<210> 35
   <211> 35
   <212> DNA
   <213> Artificial
<220>
   <223> An artificially synthesized primer sequence
<400> 35
   cttgccggcg cggccgctca tttacccgga gacag 35
<210> 36
   <211> 30
   <212> DNA
   <213> Artificial
<220>
   <223> An artificially synthesized primer sequence
<400> 36
   gtcgggatcc cccaaatcta gtgacaaaac 30
<210> 37
   <211> 45
   <212> DNA
   <213> Artificial
<220>
   <223> An artificially synthesized primer sequence
<400> 37
   gtcgggatcc ggtggcggtg gctcgcccaa atctagtgac aaaac 45
<210> 38
   <211> 60
   <212> DNA
   <213> Artificial
<220>
   <223> An artificially synthesized primer sequence
<400> 38
   gtcgggatcc ggtggcggtg gctcgggcgg tggtgggtcg cccaaatcta gtgacaaaac 60
<210> 39
   <211> 75
   <212> DNA
   <213> Artificial
<220>
   <223> An artificially synthesized primer sequence
<400> 39
   gtcgggatcc ggtggcggtg gctcgggcgg tggtgggtcg ggtggcggcg gatctcccaa 60
   atctagtgac aaaac 75
<210> 40
   <211> 31
   <212> DNA
   <213> Artificial
<220>
   <223> An artificially synthesized primer sequence
<400> 40
   gtcgggatcc tttacccgga gacagggaga g 31
<210> 41
   <211> 32
   <212> DNA
   <213> Artificial
<220>
   <223> An artificially synthesized primer sequence
<400> 41
   tcctagtcta gacccaaatc tagtgacaaa ac 32
<210> 42
   <211> 47
   <212> DNA
   <213> Artificial
<220>
   <223> An artificially synthesized primer sequence
<400> 42
   tcctagtcta gaggtggcgg tggctcgccc aaatctagtg acaaaac 47
<210> 43
   <211> 62
   <212> DNA
   <213> Artificial
<220>
   <223> An artificially synthesized primer sequence
<400> 43
   tcctagtcta gaggtggcgg tggctcgggc ggtggtgggt cgcccaaatc tagtgacaaa 60
   ac 62
<210> 44
   <211> 77
   <212> DNA
   <213> Artificial
<220>
   <223> An artificially synthesized primer sequence
<400> 44
   tcctagtcta gaggtggcgg tggctcgggc ggtggtgggt cgggtggcgg cggatctccc 60
   aaatctagtg acaaaac 77
<210> 45
   <211> 33
   <212> DNA
   <213> Artificial
<220>
   <223> An artificially synthesized primer sequence
<400> 45
   tcctagtcta gatttacccg gagacaggga gag 33
<210> 46
   <211> 35
   <212> DNA
   <213> Artificial
<220>
   <223> An artificially synthesized oligonucleotide sequence
<400> 46
   gtcgacgcta gcaaggatcc ttgaattcct taagg 35
<210> 47
   <211> 35
   <212> DNA
   <213> Artificial
<220>
   <223> An artificially synthesized oligonucleotide sequence
<400> 47
   gtcgacctta aggaattcaa ggatccttgc tagcg 35
<210> 48
   <211> 5
   <212> PRT
   <213> Artificial
<220>
   <223> An artificially synthesized peptide sequence
<400> 48
   Gly Gly Gly Gly Ser
   1               5
<210> 49
   <211> 10
   <212> PRT
   <213> Artificial
<220>
   <223> An artificially synthesized peptide sequence
<400> 49
   Gly Gly Gly Gly Ser Gly Gly Gly Gly Ser
   1               5                   10
<210> 50
   <211> 15
   <212> PRT
   <213> Artificial
<220>
   <223> An artificially synthesized peptide sequence
<400> 50
   Gly Gly Gly Gly Ser Gly Gly Gly Gly Ser Gly Gly Gly Gly Ser
   1               5                   10                  15
<210> 51
   <211> 55
   <212> DNA
   <213> Artificial
<220>
   <223> An artificially synthesized primer sequence
<400> 51
   atctatctag aggccatcac catcaccatc acatgtcccc tatactaggt tattg 55
<210> 52
   <211> 35
   <212> DNA
   <213> Artificial
<220>
   <223> An artificially synthesized primer sequence
<400> 52
   attaatcagc ggccgctcac ggggatccaa cagat 35
<210> 53
   <211> 35
   <212> DNA
   <213> Artificial
<220>
   <223> An artificially synthesized primer sequence
<400> 53
   ccccaagctt gccgccatgt ggttcttgac aactc 35
<210> 54
   <211> 35
   <212> DNA
   <213> Artificial
<220>
   <223> An artificially synthesized primer sequence
<400> 54
   aacaaaagct tgccgccatg gagacccaaa tgtct 35
<210> 55
   <211> 35
   <212> DNA
   <213> Artificial
<220>
   <223> An artificially synthesized primer sequence
<400> 55
   ccccaagctt gccgccatgg gaatcctgtc attct 35
<210> 56
   <211> 31
   <212> DNA
   <213> Artificial
<220>
   <223> An artificially synthesized primer sequence
<400> 56
   atatgaattc gccgccatgt ggcagctgct c 31
<210> 57
   <211> 28
   <212> DNA
   <213> Artificial
<220>
   <223> An artificially synthesized primer sequence
<400> 57
   gcgaatctag aatgaaacca gacaggag 28
<210> 58
   <211> 29
   <212> DNA
   <213> Artificial
<220>
   <223> An artificially synthesized primer sequence
<400> 58
   acgattctag acattggtga agagctgcc 29
<210> 59
   <211> 29
   <212> DNA
   <213> Artificial
<220>
   <223> An artificially synthesized primer sequence
<400> 59
   acgattctag acatcggtga agagctggg 29
<210> 60
   <211> 30
   <212> DNA
   <213> Artificial
<220>
   <223> An artificially synthesized primer sequence
<400> 60
   cggcatctag attggtaccc aggtggaaag 30
<210> 61
   <211> 29
   <212> DNA
   <213> Artificial
<220>
   <223> An artificially synthesized primer sequence
<400> 61
   tctgcagggg gctttttggg agtaaaaat 29
<210> 62
   <211> 29
   <212> DNA
   <213> Artificial
<220>
   <223> An artificially synthesized primer sequence
<400> 62
   atttttactc ccaaaaagcc ccctgcaga 29
<210> 63
   <211> 7
   <212> PRT
   <213> Artificial
<220>
   <223> An artificially synthesized peptide sequence
<220>
   <221> MISC_FEATURE
   <222> (2) .. (6)
   <223> arbitrary repeat
<400> 63
   Ala Glu Ala Ala Ala Lys Ala
   1               5